# EUROPEAN PATENT APPLICATION

(11) **EP 4 851 172 A1**
(43) Date of publication of application: **09.12.2026**
(21) Application number: 25748021.0
(22) Date of filing: 27.01.2025
(51) Int. Cl.: C07F 9/6558, A61K 31/675, A61P 35/00

(54) **NEW LIGAND-TARGETED COMPOUND, CHELATE, COMPOSITION AND USE THEREOF**

(30) Priority: 02.02.2024 WO PCT/CN2024/075535
(71) Applicant: Peking University, Beijing 100871 (CN)
(72) Inventor: LIU, Zhibo, Beijing 100871 (CN); WEN, Zihao, Beijing 100871 (CN); CUI, Xiyang, Beijing 100871 (CN)
(74) Representative: Cabinet Becker et Associés
(86) International application number: PCT/CN2025/075553
(87) International publication number: WO 2025/162459

(57) **Abstract**

A compound of formula (I), or a pharmaceutically acceptable salt, stereoisomer, or solvate thereof, wherein W is oxygen or sulfur; X₁ is -L₁-R₁; Yi is -L₁-R₂; Z₁ is -L₂-R₃-L₁-R₄; L₁, at each occurrence, is independently a bond or a linking group; L₂ is O, S, or NH; R₃ is an optionally substituted arylene, heteroarylene, cycloalkylene or heterocycloalkylene group; one of R₁, R₂ and R₄ is a targeting group, and the remaining two are independently a targeting group, a drug molecule group, an isotope chelator or labeling precursor group, a fluorescent or molecular labeling group, or a capping group. The present disclosure further provides a chelate, a pharmaceutical composition, and uses thereof as a diagnostic or therapeutic agent for diagnosing and treating a disease.

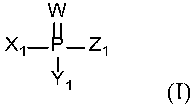

## Description

### Technical Field

The present disclosure relates to the fields of medicine and diagnosis, and in particular to a novel ligand-targeted compound, chelate, composition, and uses thereof.

### Background Technology

A targeted drug delivery strategy is a method for precisely and controllably releasing a drug at a specific site to achieve a therapeutic effect. A targeted drug delivery system mainly consists of the following components: a targeting ligand, a linker (controllable-release structure), and a payload. Depending on the different targeting ligand and payload, different targeted drug delivery systems may be classified as antibody-drug conjugates (ADCs), peptide-drug conjugates (PDCs), small-molecule drug conjugates (SMDCs), aptamer-drug conjugates (ApDCs), radionuclide-drug conjugates (RDCs), and the like. Cleavable structures are critical for achieving controllable drug release. They are mainly divided into two categories: chemically cleavable linkers and enzyme-cleavable linkers. Among chemically cleavable linkers, a hydrazone structure is a classic acid-sensitive linker. Hydrazone linkers are generally stable in the bloodstream; however, after a molecule is internalized by a target cancer cell, the acidic environment in lysosomes (pH 4.8) and endosomes (pH 5.5-6.2) hydrolyzes the hydrazone structure to release a cytotoxic drug. Among marketed ADCs, Mylotarg and Besponsa use a hydrazone as the controllable-release structure. A disulfide linker is another classic chemically cleavable linker and is sensitive to reduced glutathione (GSH). The concentration of GSH in blood is significantly lower than the intracellular concentration in cancer cells. Accordingly, this type of linker can remain stable in the circulatory system while controllably releasing an active payload in cancer cells having elevated GSH levels. Among marketed ADC drugs, Elahere uses a disulfide bond as the controllable-release structure. Among enzyme-cleavable linkers, various peptide-based linkers are sensitive to lysosomal proteases and have been used in many ADCs. Lysosomal proteases, such as cathepsin B, are generally overexpressed in cancer cells, thereby allowing a drug to be accurately released in the vicinity of a tumor. Among 15 marketed ADC drugs, 9 use enzyme-cleavable linkers. However, existing controllable-release strategies have insufficient specificity, resulting in pronounced off-target toxicity during the long in vivo circulation of ADCs.

Therefore, there remains a need to develop a ligand-targeted compound that addresses one or more of the foregoing deficiencies.

### Summary of the Invention

The present disclosure provides a ligand-targeted compound having a phosphoryl group of a specific structural type as its core, so as to improve the specificity of drug delivery and/or reduce off-target toxicity.

In one aspect, the present disclosure provides a compound of formula (I), or a pharmaceutically acceptable salt, isomer, or solvate thereof, 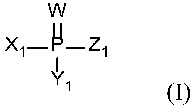 wherein
W is oxygen or sulfur, preferably oxygen;
X₁ is -L₁-R₁;
Y₁ is -L₁-R₂;
Z₁ is -L₂-R₃-L₁-R₄;
L₁, at each occurrence, is independently a bond or a linking group;
L₂ is O, S, or NH, preferably oxygen;
R₃ is an optionally substituted arylene, heteroarylene, cycloalkylene or heterocycloalkylene group; the substituent is selected from F, Br, Cl, methoxy, trifluoromethyl, and cyano;
one of R₁, R₂ and R₄ is a targeting group, and the remaining two are independently a targeting group, a drug molecule group, an isotope chelator or labeling precursor group, a fluorescent or molecular labeling group, or a capping group.

In some embodiments, one of R₁, R₂, and R₄ is an isotope chelator or labeling precursor group.

In another aspect, the present disclosure provides a chelate or nuclide label comprising the compound of formula (I), or a pharmaceutically acceptable salt, stereoisomer, or solvate thereof, and a radionuclide.

In a further aspect, the present disclosure provides a pharmaceutical composition comprising or consisting of:
(a) the compound of formula (I) as described above, or a pharmaceutically acceptable salt, stereoisomer, or solvate thereof; or the chelate or nuclide label described above;
(b) optionally, and a pharmaceutically acceptable excipient.

In yet another aspect, the present disclosure further provides the diagnostic or therapeutic use of the compound of formula (I) as described above, or a pharmaceutically acceptable salt, stereoisomer, or solvate thereof; or the diagnostic or therapeutic use of the chelate or nuclide label as described above; or the diagnostic or therapeutic use of the pharmaceutical composition as described above; or a kit of the same.

In yet another aspect, the present disclosure further provides a compound of formula (II), or a pharmaceutically acceptable salt, stereoisomer, or solvate thereof, which can be used as an intermediate for preparing a compound of formula (I).

### Description of the Drawings

To more clearly illustrate the technical solutions of the embodiments of the present disclosure, the drawings used in the embodiments are briefly described below. It will be apparent that the drawings described below relate only to some embodiments of the present disclosure and do not limit the present invention.
FIG. 1 shows that LDP-FAP-MMAE releases the drug molecule MMAE only in the presence of FAP protein, and that the release can be inhibited by the addition of FAPI-04.
FIG. 2 shows that LDP-FAP-MMAE can release the drug molecule MMAE in FAP-positive cells, with a significant difference from FAP-negative cells.
FIG. 3 shows photographs of the distribution of ⁶⁸Ga-LDP-FAP-MMA in HT1080-FAP tumor-bearing mice.
FIG. 4 shows MMAE release from LDP-FAP-MMAE in different tissues of HT1080-FAP tumor-bearing mice.
FIG. 5 shows tumor volumes in HT1080-FAP tumor-bearing mice after treatment with LDP-FAP-MMAE.
FIG. 6 shows SDS-PAGE and autoradiography results obtained after co-incubation of ¹⁷⁷Lu-labeled LDP-FAPI-DOTA with FAP protein.
FIG. 7 shows PET-CT imaging results obtained after administration of ⁶⁸Ga-FAPI-04 and ⁶⁸Ga-LDP-FAPI-DOTA to mice.
FIG. 8 shows the percentages of MMAE molecules released from LDP-FAP-H-FITC, LDP-B-FAP-H-MMAE, LDP-B-FAP-F-MMAE, LDP-B-FAP-F-NIR-DOTA, LDP-B-FAP-H-MMAE-DOTA, and LDP-B-FAP-F-MMAE-DOTA in the presence of FAP protein.
FIG. 9 shows fluorescence confocal microscopy images obtained after LDP-FAP-H-FITC and LDP-B-FAP-F-NIR-DOTA were respectively co-incubated with HT1080 cells and HT080-FAP-expressing cells.
FIG. 10 shows the dosing regimen, changes in tumor volume, changes in body weight, and tumor-change curves for each mouse after administration of LDP-B-FAP-F-MMAE-DOTA in a PDX tumor model.
FIG. 11 shows in vivo near-infrared fluorescence imaging of LDP-FAP-PEG0-S0456, LDP-FAP-PEG2-S0456, LDP-FAP-PEG5-S0456, and LDP-FAP-F-PEG5-S0456 in HT1080-FAP tumor-bearing mice.
FIG. 12 shows autoradiography results from an SDS-PAGE experiment after co-incubation of LDP-FAP-S0456-DOTA with FAP protein.
FIG. 13 shows PET-CT imaging of ⁶⁸Ga-radiolabeled LDP-FAP-S0456-DOTA in HT1080-FAP tumor-bearing mice.
FIG. 14 shows near-infrared fluorescence imaging of LDP-FAP-S0456-DOTA in HT1080-FAP tumor-bearing mice.
FIG. 15 shows that FAP-Cy5-Quencher and FAP-Cy5-Quencher-DOTA release the quencher group after addition of FAP protein, resulting in a significant increase in fluorescence signal.
FIG. 16 shows fluorescence confocal microscopy images obtained at 6-24 hours after FAP-Cy5-Quencher and FAP-Cy5-Quencher-DOTA were respectively co-incubated with HT1080 cells and HT080-FAP-expressing cells.
FIG. 17 shows near-infrared fluorescence imaging of FAP-Cy5-Quencher and FAP-Cy5-Quencher-DOTA in HT1080-FAP tumor-bearing mice.
FIG. 18 shows the percentages of drug molecules released from LDP-Dox, LDP-Exatecan, and LDP-Dxd in the presence of FAP protein.
FIG. 19 shows that CA-P-1 and CA-P-2 release fluorescent molecules in the presence of carbonic anhydrase 1 protein, resulting in an increase in fluorescence signal.

### Detailed Description

To make the objectives, technical solutions, and advantages of the embodiments of the present disclosure clearer, the technical solutions of the embodiments of the present disclosure are described clearly and completely below with reference to the drawings of the embodiments of the present disclosure. It is apparent that the described embodiments are only some, rather than all, of the embodiments of the present disclosure. All other embodiments obtained by a person of ordinary skill in the art based on the described embodiments of the present disclosure without inventive labor fall within the scope of protection of the present invention.

The present invention may be embodied in other specific forms without departing from the essential attributes of the present invention. It should be understood that, provided that no conflict arises, the technical features of any and all embodiments of the present invention may be combined with those of any other embodiment or embodiments to obtain further embodiments. The present invention includes such further embodiments obtained by combination.

All publications and patents mentioned in the present disclosure are hereby incorporated into the present disclosure by reference in their entirety. If any use or term used in any publication or patent incorporated by reference conflicts with a use or term used in the present disclosure, the use and term in the present disclosure shall prevail.

The section headings used herein are for organizational purposes only and should not be construed as limiting the described subject matter.

Unless otherwise specified, all technical and scientific terms used herein have the meanings commonly understood in the art to which the claimed subject matter belongs. If multiple definitions exist for a term, the definition herein shall prevail.

Unless otherwise stated, when any type of range is disclosed or claimed, it is intended to individually disclose or claim every possible numerical value that the range may reasonably encompass, including any subrange encompassed therein. For example, a substituent number of 1 to 5 indicates integers within the range, wherein 1-5 should be understood to include 1, 2, 3, 4 and 5, and also subranges of 1-4 and 1-3.

The specification of the present disclosure should be interpreted consistently with the rules and principles of chemical bonding. In some cases, a hydrogen atom may be removed in order to accommodate a substituent at a given position.

As used in the present disclosure, words such as "comprise", "contain" or "include" mean that the element preceding such word encompasses the elements listed after such word and equivalents thereof, without excluding unrecited elements. As used herein, the terms "contain" or "comprise/include" may be open-ended, semi-close-ended or close-ended. In other words, the terms also include "consisting essentially of" or "consisting of".

The term "pharmaceutically acceptable" as used in the present application means that a compound or composition is chemically and/or toxicologically compatible with the other ingredients constituting a formulation and/or with the human or mammal for whom it is used to prevent or treat a disease or disorder.

The terms "subject" or "patient" in the present application include humans and mammals.

In the context of the present application, unless specifically indicated to the contrary, the term "treatment" may also include prevention.

The term "solvate" in the present application refers to a complex formed by combining a compound of formula (I), or a pharmaceutically acceptable salt thereof, with a solvent. It should be understood that any solvate of a compound of formula (I) used in diagnosing or treating a disease or disorder described in the present application, although it may provide different properties (including pharmacokinetic properties), once absorbed into a subject, will give the compound of formula (I), such that use of the compound of formula (I) respectively encompasses use of any solvate of the compound of formula (I).

The term "hydrate" refers to the case in which the solvent in the above term "solvate" is water.

It should further be understood that the compound of formula (I), or a pharmaceutically acceptable salt thereof, may be isolated in solvated form, and therefore any such solvate is included within the scope of the present invention. For example, the compound of formula (I), or a pharmaceutically acceptable salt thereof, may exist in unsolvated form and in solvated forms formed with pharmaceutically acceptable solvents, such as water, ethanol, and the like.

The term "pharmaceutically acceptable salt" refers to a relatively non-toxic addition salt of a compound of the present disclosure. For example, see S. M. Berge et al., "Pharmaceutical Salts", J. Pharm. Sci. 1977, 66, 1-19.

Suitable pharmaceutically acceptable salts of the compounds of the present disclosure may be, for example, acid addition salts of compounds of the present disclosure having sufficient basicity and carrying a nitrogen atom in a chain or ring, for example acid addition salts formed with inorganic or organic acids.

In addition, another suitable pharmaceutically acceptable salt of a compound of the present invention having sufficient acidity is an alkali metal salt, an alkaline earth metal salt, or a salt formed with an organic base that provides a physiologically acceptable cation.

Those skilled in the art will also recognize that acid addition salts of the claimed compounds may be prepared by reacting the compounds with an appropriate inorganic or organic acid by any one of various known methods. Alternatively, alkali metal salts and alkaline earth metal salts of acidic compounds of the present disclosure are prepared by reacting them with an appropriate base by various known methods.

The present invention includes all possible salts of the compounds of the present disclosure, which may be a single salt or any mixture of the salts in any ratio.

It should be understood that the term "compound of the present disclosure" as used in the present application may, depending on the context, include: the compound represented by formula (I), a pharmaceutically acceptable salt thereof, a solvate thereof, a solvate of a pharmaceutically acceptable salt thereof, and mixtures thereof.

The compounds of the present disclosure may contain one or more asymmetric centers, depending on the positions and nature of various desired substituents. Asymmetric carbon atoms may be present in the (R) or (S) configuration, resulting in a racemic mixture in the case of a single asymmetric center and a mixture of diastereomers in the case of multiple asymmetric centers. In certain cases, asymmetry may also exist due to restricted rotation around a particular bond, for example, a central bond joining two substituted aromatic rings of a particular compound.

Preferred compounds are those that produce more desirable biological activity. Isolated, purified or partially purified isomers and stereoisomers of the compounds of the present disclosure, or racemic mixtures or mixtures of diastereomers, are all included within the scope of the present invention. Purification and separation of such substances may be achieved by standard techniques known in the art.

The term "optional" as used herein to describe a condition means that the condition may or may not occur. For example, the term "optionally substituted" means unsubstituted or having at least one non-hydrogen substituent that does not destroy the desired properties possessed by the unsubstituted analog. For example, with respect to a pharmaceutical composition, the expression "optionally, and a pharmaceutically acceptable excipient" as used herein indicates that the pharmaceutically acceptable excipient may be present in the pharmaceutical composition or may be absent from the pharmaceutical composition.

In the present disclosure, unless otherwise specifically stated, the number of "substitutions" may be one or more; when there are multiple substitutions, there may be 2, 3 or 4 substitutions. Moreover, when the number of "substitutions" is multiple, the "substitutions" may be the same or different.

In the present disclosure, unless otherwise specifically stated, the position of "substitution" may be any position.

As used herein, the term "alkyl" refers to a linear or branched alkane chain containing 1-14 carbon atoms. For example, representative examples of C₁-C₁₄ alkyl include, but are not limited to, methyl (C₁), ethyl (C₂), n-propyl (C₃), isopropyl (C₃), n-butyl (C₄), tert-butyl (C₄), sec-butyl (C₄), and isobutyl (C₄). Representative examples of C₅-C₁₄ alkyl include, but are not limited to, n-pentyl (C₅), 3-pentanyl (C₅), neopentyl (C₅), 3-methyl-2-butanyl (C₅), tert-pentyl (C₅), n-hexyl (C₆), and the like. The term "lower alkyl" refers to a linear or branched alkyl having 1 to 4 carbon atoms. "Substituted alkyl" means an alkyl substituted at any available point of attachment by one or more substituents, preferably 1 to 4 substituents. The term "haloalkyl" refers to an alkyl having one or more halo substituents, including but not limited to groups such as -CH₂Br, -CH₂I, -CH₂Cl, -CH₂F, -CHF₂ and -CF₃.

As used herein, the term "optionally substituted alkyl" means that the alkyl may be an unsubstituted alkyl or a substituted alkyl. Substituted alkyl includes, but is not limited to, C₁₋₅ alkyl substituted with one or more substituents selected from C₁₋₄ alkyl, C₁₋₄ alkoxy, hydroxy, amino, thiol, halogen, nitro and -CN.

As used herein, the term "alkylene" refers to a divalent hydrocarbon group as described above for "alkyl" but having two points of attachment. For example, methylene is a -CH₂- group, and ethylene is a -CH₂-CH₂- group.

As used herein, the terms "alkoxy" and "alkylthio" refer to the alkyl described above attached via an oxygen bond (-O-) or a sulfur bond (-S-), respectively. The terms "substituted alkoxy" and "substituted alkylthio" refer to substituted alkyl attached via an oxygen bond or sulfur bond, respectively. "Lower alkoxy" is a group OR, wherein R is lower alkyl (an alkyl containing 1 to 4 carbon atoms).

As used herein, the term "halogen" refers to fluorine, chlorine, iodine or bromine.

As used herein, the term "cycloalkyl" refers to a saturated cycloalkyl group derived by removing one hydrogen atom from a single carbon atom of the parent cycloalkane. Typical C₃₋₁₄ cycloalkyl includes, but is not limited to, groups of cyclopropane, cyclobutane, cyclopentane, cyclohexane, cycloheptane, cyclooctane, and the like. Cycloalkyl may be described by the number of carbon atoms in the ring. For example, cycloalkyl having 5-7 ring atoms may be referred to as (C₅-C₇)cycloalkyl. In certain embodiments, cycloalkyl may be (C₅-C₁₄)cycloalkyl, (C₅-C₈)cycloalkyl, (C₅-C₇)cycloalkyl, or (C₅-C₆)cycloalkyl, and these may be referred to using alternative language as C₅-C₁₄ cycloalkyl, C₅-C₈ cycloalkyl, C₅-C₇ cycloalkyl, C₅-C₆ cycloalkyl or C₅-C₇ cycloalkyl.

As used herein, the term "cycloalkylene" refers to the generic term for the remaining divalent group after two hydrogen atoms are removed from any positions of the carbocyclic ring of a cycloalkyl. Typical cycloalkylene includes, but is not limited to, cyclopentylene, cyclohexylene, cycloheptylene, cyclooctylene, and the like.

As used herein, the term "heterocycloalkyl", interchangeably "heterocyclyl", refers to a saturated, partially unsaturated or fully unsaturated monocyclic, bicyclic or tricyclic heterocyclic group containing at least one, preferably 1 to 3, heteroatoms as ring members, wherein each such heteroatom may be independently selected from nitrogen, oxygen and sulfur, wherein each ring is preferably a 5- to 8-membered ring, preferably each ring is a 5- to 6-membered ring. Typical heterocycloalkyl includes, but is not limited to, tetrahydrofuranyl, tetrahydropyrrolyl, tetrahydrothienyl, tetrahydropyranyl, piperidyl, tetrahydrothiopyranyl, dioxanyl, piperazinyl, pyrazinyl, morpholinyl, dioxanyl, and the like.

As used herein, the term "heterocycloalkylene" refers to the generic term for the remaining divalent group after two hydrogen atoms are removed from any positions on the ring of a heterocycloalkyl. Typical heterocycloalkylene includes, but is not limited to, tetrahydrofuranylene, tetrahydropyrrolylene, tetrahydrothienylene, tetrahydropyranylene, piperidylene, tetrahydrothiopyranylene, dioxanylene, piperazinylene, 1,4-piperazinylene, pyrazinylene, morpholinylene, dioxanylene, and the like.

As used herein, the term "arylene" refers to the generic term for the remaining divalent group after two hydrogen atoms are removed from carbons at any positions on the aromatic nucleus of an aromatic hydrocarbon molecule, such as phenylene, naphthylene, 1,2-phenylene, 1,3-phenylene, 1,4-phenylene, 1,2-naphthylene, 1,5-naphthylene, 1,6-naphthylene or 2,6-naphthylene, and the like.

As used herein, the term "optionally substituted arylene" includes unsubstituted arylene and substituted arylene, for example arylene substituted with one or more substituents selected from C₁₋₄ alkyl, C₁₋₄ haloalkyl, C₁₋₄ alkoxy, hydroxy, amino, thiol, halogen, nitro and -CN.

As used herein, the term "heteroaryl" refers to any substituted monovalent aryl group containing about 5 to about 14 skeletal ring-forming atoms, wherein one to three ring-forming atoms are heteroatoms independently selected from, but not limited to, oxygen, nitrogen and sulfur, provided that the ring of the group does not contain two adjacent O or S atoms. In embodiments in which two or more heteroatoms are present in the ring, the two or more heteroatoms may be the same as each other, or some or all of the two or more heteroatoms may be different from each other. The term heteroaryl includes monovalent fused or non-fused heteroaryl having at least one heteroatom and being optionally substituted. In addition, the term heteroaryl further includes fused and non-fused heteroaryl containing 5 to about 14 skeletal ring-forming atoms, and fused and non-fused heteroaryl having 5 to about 10 skeletal ring-forming atoms. Heteroaryl may be attached through a carbon atom or a heteroatom. Thus, by way of example, imidazole may be attached to a parent molecule through any of its carbon atoms (imidazol-2-yl, imidazol-4-yl or imidazol-5-yl) or through its nitrogen atom (imidazol-1-yl or imidazol-3-yl). Similarly, a heteroaryl group may be further substituted through any or all carbon atoms and/or any or all heteroatoms thereof. A fused heteroaryl may comprise a fused ring in which 2-4 aromatic heterocycles are fused, and other independent rings may be alicyclic, heterocyclic, aromatic, aromatic heterocyclic, or any combination thereof. Non-limiting examples of monocyclic heteroaryl include pyridyl; fused ring heteroaryl includes benzimidazolyl, quinolinyl and acridinyl; and non-fused biheteroaryl includes bipyridinyl. Other examples of heteroaryl include, but are not limited to: furanyl, thienyl, oxazolyl, acridinyl, phenazinyl, benzimidazolyl, benzofuranyl, benzoxazolyl, benzothiazolyl, benzothiadiazolyl, benzothiophenyl, benzoxadiazolyl, benzotriazolyl, imidazolyl, indolyl, isoxazolyl, isoquinolinyl, indolizinyl, isothiazolyl, isoindolyl, oxadiazolyl, indazolyl, pyridyl, pyridazyl, pyrimidyl, pyrazinyl, pyrrolyl, pyrazolyl, purinyl, phthalazinyl, pteridinyl, quinolinyl, quinazolinyl, quinoxalinyl, triazolyl, tetrazolyl, thiazolyl, triazinyl, thiadiazolyl, and the like, and oxides thereof, for example pyridyl-N-oxide.

As used herein, the term "heteroarylene" refers to the generic term for the remaining divalent group after two hydrogen atoms are removed from carbons at any positions on the heteroaromatic nucleus of a heteroaryl molecule, such as furanylene, thienylene, pyrrolylene, imidazolylene, pyrazolylene, triazolylene, pyridinylene, pyrazinylene, pyrimidinylene or pyridazinylene, and the like. As used herein, the term "optionally substituted heteroarylene" includes unsubstituted heteroarylene and substituted heteroarylene, for example heteroarylene substituted with one or more substituents selected from C₁₋₄ alkyl, C₁₋₄ haloalkyl, C₁₋₄ alkoxy, hydroxy, amino, thiol, halogen, nitro and -CN.

In the compounds provided herein, any atom that is not specifically defined as a particular isotope represents any stable isotope of that atom. Isotopic substitution, for example deuterium substitution, may be partial or complete. Partial deuterium substitution means that at least one hydrogen is replaced by deuterium. As non-limiting general examples, isotopes of hydrogen such as deuterium (²H) and tritium (³H) may be used anywhere in the structure to achieve the desired result. Alternatively, isotopes of carbon, for example ¹³C and ¹⁴C, may also be used.

In certain embodiments, the isotope is at least about 90, 95 or 99% or higher isotopically enriched at any involved position. In one non-limiting embodiment, deuterium is at least about 90, 95 or 99% enriched at the desired position.

In some non-limiting embodiments, substitution of hydrogen by deuterium occurs in a group selected from R¹, R², R³, R⁴, R^{a}, R^{b}, R^{c}, or any other substituent group defined herein. In other non-limiting embodiments, at least one of R¹, R², R³, R⁴, R^{a}, R^{b}, R^{c} is enriched with deuterium. For example, when any group is, or comprises, for example, a substituent, methyl, ethyl or methoxy, the alkyl residue may be deuterated (for example, CDH₂, CD₂H, CD₃, CH₂CD₃, CD₂CD₃, CHDCH₂D, CH₂CD₃, CHDCHD₂, OCDH₂, OCD₂H, or OCD₃, and the like). In certain other embodiments, when two substituents are combined to form a ring, an unsubstituted carbon may be deuterated.

It should be understood that the singular forms (such as "a" or "an") used in the present disclosure may include plural referents, unless otherwise specified.

Unless otherwise indicated, the present disclosure employs standard nomenclature and standard laboratory procedures and techniques of analytical chemistry, organic synthetic chemistry, and coordination chemistry. Unless otherwise stated, the present disclosure employs conventional methods of mass spectrometry and elemental analysis, and the steps and conditions may refer to conventional procedures and conditions in the art.

The reagents and starting materials used in the present disclosure are commercially available or may be prepared by conventional chemical synthesis methods.

The term "targeting group" as used herein refers to a ligand group that specifically binds to a target protein. For example, a targeting group is a ligand group having high affinity for a tumor- or tissue-specific biomarker.

As used herein, the term "drug molecule group" refers to a group derived from a drug molecule. The drug molecule is, for example, a drug molecule having one or more functions of cytotoxicity, immunomodulation, protein degradation, and antibacterial activity. The drug molecule is, for example, a cytotoxic drug, an immunomodulator, an antibiotic, a protein degrader or a molecular glue. In one embodiment, the drug molecule is a DNA crosslinking agent, a microtubule inhibitor, a DNA alkylating agent, a topoisomerase inhibitor, or a combination thereof. For example, the drug molecule is selected from Catharanthus alkaloids, laulimalide, taxanes, colchicine, tubulysin, Cryptophycin, Hemiasterlin, Cemadotin, Rhizoxin, Discodermolide, taccalonolide A, B, AF or AJ, taccalonolide Al-epoxide, CA-4, epothilone A and B, paclitaxel, docetaxel, doxorubicin, camptothecin, iSGD-1882, centanamycin, PNU-159682, uncialamycin, indolinobenzodiazepine dimers, beta-amanitin, Amatoxin, thailanstatins or derivatives or analogs thereof, or combinations thereof. The immunomodulator may be selected, for example, from: cytokines, chemokines, stem cell growth factors, lymphotoxins, hematopoietic factors, colony stimulating factors (CSF), interferons, erythropoietin, thrombopoietin, tumor necrosis factor (TNF), interleukins (IL), granulocyte colony stimulating factor (G-CSF), granulocyte macrophage colony stimulating factor (GM-CSF), stem cell growth factor known as "S1 factor", activators of costimulatory molecules (for example, OX40 agonists), immune checkpoint molecules (for example, inhibitors of PD-1, PD-L1, LAG-3, TIM-3, CEACAM or CTLA-4, VISTA, BTLA, TIGIT, LAIR1, CD160, 2B4 and/or TGFR beta).

The term "isotope chelator or labeling precursor group" as used herein refers to a group derived from an isotope chelator or labeling precursor molecule. The isotope chelator or labeling precursor may be an isotope chelator or labeling precursor capable of chelating a metallic nuclide or being labeled with a medical radionuclide, for example, HYNIC, DTPA, DOTA, NOTA, and derivatives thereof.

The term "fluorescent or molecular labeling group" as used herein refers to a group derived from a fluorescent or molecular label. The fluorescent or molecular label may be, for example, a fluorescent dye, a quantum dot, biotin, or a nuclide (for example, ¹⁸F, ⁵¹Cr, ⁶⁷Ga, ⁶⁸Ga, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, ⁸⁶Y, ⁸⁸Y, ⁹⁰Y, ¹⁴⁹Pm, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg, ²¹¹At, ¹⁵¹Eu, ¹⁵³Eu, ¹⁶⁹Eu, ²⁰¹Tl, ²⁰³Pb, ²¹²Pb, ⁶⁴Cu, ⁶⁷Cu, ¹⁸⁸Re, ¹⁸⁶Re, ¹⁹⁸Au, ²²⁵Ac, ^{227Th} and ¹⁹⁹Ag).

The term "capping group" as used herein refers to a low-molecular-weight monovalent group that does not readily undergo chemical transformation under typical synthetic reaction conditions. Examples of capping groups include methyl, ethyl, n-propyl, isopropyl, methoxy, ethoxy, n-propoxy, isopropoxy, and the like.

In one aspect, the present disclosure provides a compound of formula (I), or a pharmaceutically acceptable salt, stereoisomer or solvate thereof, 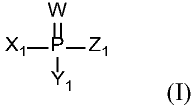 wherein
W is oxygen or sulfur, preferably oxygen;
X₁ is -L₁-R₁;
Y₁ is -L₁-R₂;
Z₁ is -L₂-R₃-L₁-R₄;
L₁, at each occurrence, is independently a bond or a linking group;
L₂ is O, S, or NH, preferably oxygen;
R₃ is an optionally substituted arylene, heteroarylene, cycloalkylene or heterocycloalkylene group, for example, phenylene; the substituent is selected from F, Br, Cl, methoxy, trifluoromethyl, and cyano;
one of R₁, R₂ and R₄ is a targeting group, and the remaining two are independently a targeting group, a drug molecule group, an isotope chelator or labeling precursor group, a fluorescent or molecular labeling group, or a capping group.

In one embodiment, R₃ is optionally substituted C₅₋₁₄ arylene, C₅₋₁₄ heteroarylene, C₅₋₁₄ cycloalkylene, or C₅₋₁₄ heterocycloalkylene.

In one embodiment, R₃ is selected from
optionally substituted C₅₋₁₄ arylene;
optionally substituted C₅₋₁₄ heteroarylene containing 1 to 3 heteroatoms independently selected from N, O, or S;
optionally substituted divalent saturated or partially unsaturated fused, bridged or spiro C₅₋₁₄ cycloalkylene containing a single ring or multiple rings;
optionally substituted divalent saturated or partially unsaturated fused, bridged or spiro C₅₋₁₄ cycloalkylene containing a single ring or multiple rings; or
optionally substituted divalent saturated or partially unsaturated fused, bridged or spiro C₅₋₁₄ heterocycloalkylene containing a single ring or multiple rings, which contains 1 to 3 heteroatoms independently selected from N, O, or S;
the substituent is selected from F, Br, Cl, methoxy, trifluoromethyl, and cyano.

In other embodiments, R₃ is selected from optionally substituted 1,2-phenylene, 1,3-phenylene, 1,4-phenylene, 1,2-naphthylene, 1,5-naphthylene, 1,6-naphthylene, 2,6-naphthylene, furanylene, thienylene, pyrrolylene, imidazolylene, pyrazolylene, triazolylene, pyridylene, pyrazinylene, pyrimidinylene, pyridazinylene, cyclopentylene, cyclohexylene, tetrahydrofuranylene, tetrahydropyrrolylene, tetrahydrothienylene, tetrahydropyranylene, piperidinylene, tetrahydrothiopyranylene, dioxanylene, piperazinylene, 1,4-piperazinylene, pyrazinylene, morpholinylene, and dioxanylene; the substituent is selected from F, Br, Cl, methoxy, trifluoromethyl, and cyano.

In other embodiments, R₃ is selected from 3-X-1,4-phenylene, 2-X-1,4-phenylene, 2-X-1,5-phenylene, 3-X-1,5-phenylene, 3-X-1-phenylene, 2-X-1-phenylene, or 4-X-1-phenylene (structures shown below), X is selected from F, Br, Cl, methoxy, trifluoromethyl, and cyano.

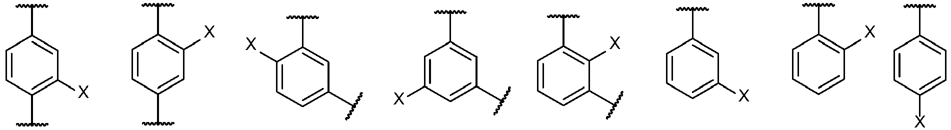

L₁ being a bond means that the groups on the both sides of L₁ are directly connected. For example, when X₁ is described as -L₁-R₁, L1 being a bond means that X₁ is -R₁.

A linking group in the present disclosure refers to a divalent group connecting the groups on its two sides. A linking group for L₁ may be independently selected at each occurrence. For example, the linking groups appearing in X₁, Y₁, and Z₁ may be the same, partially the same, or different. Examples of linking groups include, but are not limited to, -C(O)-, -O-, -S-, -SS-, - NH-, -N(CH₃)-, -CH₂-, -CH(CH₃)-, -CH₂CH₂-, -CH₂O-, 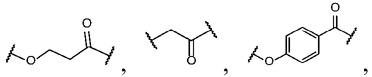 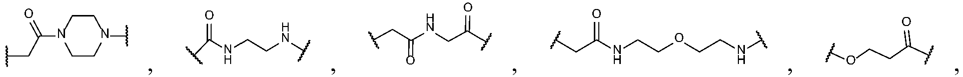 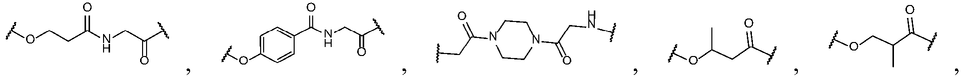 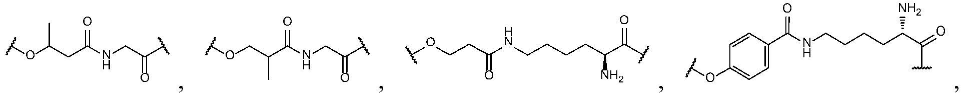 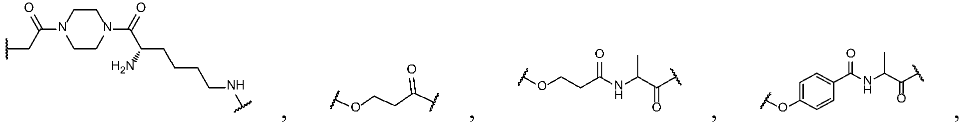 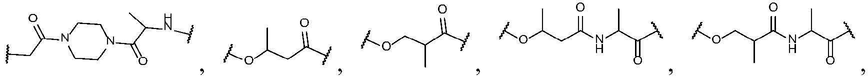 or forms thereof substituted with one or more substituents selected from C₁₋₄ alkyl, C₁₋₄ alkoxy, hydroxy, amino, mercapto, halogen, nitro, and -CN.

In one embodiment, L₁, at each occurrence, is independently a bond, or -Lₐ-(L_{b})ₘ-L_{c}-(L_{d})ₙ-Lₑ-L_{f}-, wherein Lₐ, L_{b}, L_{c}, L_{d}, Lₑ, L_{f}, at each occurrence, are each independently selected from a bond, -O-, -S-, -SS-, -S(=O)₂-, -NH-, -N(CH₃)-, -C(R^{a})(R^{b})-, -C(=O)-, -C(=O)O-, -OC(=O)-, - C(=O)NH-, -NHC(=O)-, -C(=O)-N(CH₃)-, -N(CH₃)-C(=O)-, -CH₂CH₂-, -CH₂CH₂CH₂-, - CH₂CH₂CH₂CH₂-, -CH₂NH-, -NHCH₂-, -CH₂O-, -OCH₂-, -CH=N-O-, -C(CH₃)=N-NH-, - C(CH₃)=N-NH-C(=O)-, -NH-CH₂-C(=CH-COOH)-, a divalent peptide group containing 1 to 4 amino acids, cyclohexylene, phenylene, heterocycloalkylene (for example, 1,4-piperazinylene), or 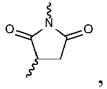 R^{a} and R^{d}, at each occurrence, are each independently selected from H, methyl, ethyl, propyl, isopropyl, phenyl, -OH, -SH, -NH₂, -NH(CH₃), or -NH(CH₃)₂, the subscripts m and n are each independently an integer of 0-4.

In some embodiments, after the targeting group of a compound of formula (I) of the present disclosure, or a pharmaceutically acceptable salt, stereoisomer, or solvate thereof, recognizes and binds to a target protein, a neighboring nucleophilic amino acid residue (lysine, cysteine, tyrosine, or the like) in the protein pocket undergoes an S_{N}2 reaction therewith, and the -R₃-L₁-R₄ group departs.

In one embodiment, one of R₁, R₂, and R₄ is a targeting group for a target protein, and the remaining two are independently a drug molecule group, an isotope chelator or labeling precursor group, a fluorescent or molecular labeling group, or a capping group. For example, R₄ is a targeting group for a target protein, R₁ is a drug molecule group, an isotope chelator or labeling precursor group, or a fluorescent or molecular labeling group, and R₂ is a capping group; or R₄ is a targeting group for a target protein, R₂ is a drug molecule group, an isotope chelator or labeling precursor group, or a fluorescent or molecular labeling group, and R₁ is a capping group. In another embodiment, two of R₁, R₂, and R₄ are independently targeting groups for a target protein, and the remaining one is a drug molecule group, an isotope chelator or labeling precursor group, a fluorescent or molecular labeling group, or a capping group. For example, R₁ and R₂ are independently targeting groups for a target protein, and R₄ is a drug molecule group, an isotope chelator or labeling precursor group, a fluorescent or molecular labeling group, or a capping group; or R₁ and R₄ are independently targeting groups for a target protein, and R₂ is a drug molecule group, an isotope chelator or labeling precursor group, a fluorescent or molecular labeling group, or a capping group; or R₂ and R₄ are independently targeting groups for a target protein, and R₁ is a drug molecule group, an isotope chelator or labeling precursor group, a fluorescent or molecular labeling group, or a capping group.

In one embodiment, R₄ is a targeting group or a drug molecule group, R₁ and R₂ are each independently a targeting group, an isotope chelator or labeling precursor group, a fluorescent or molecular labeling group, or a capping group.

In one embodiment, R₄ is a targeting group, R₁ and R₂ are each independently a drug molecule group, an isotope chelator or labeling precursor group, a fluorescent or molecular labeling group, or a capping group.

In one embodiment, R₄ is a targeting group, one of R₁ and R₂ is a drug molecule group, an isotope chelator or labeling precursor group, or a fluorescent or molecular labeling group, and the other is a capping group.

In one embodiment, R₄ is a targeting group for a target protein, one of R₁ and R₂ is an isotope chelator or labeling precursor group, and the other is a capping group.

When R₄ is a targeting group for a target protein, after R₄ recognizes and binds to the target protein, a neighboring nucleophilic amino acid residue (lysine, cysteine, tyrosine, or the like) in the protein pocket undergoes an S_{N}2 reaction with an electrophilic linker having a cleavable reaction site. The targeting ligand departs while a functional molecule is covalently attached to the target protein, thereby achieving "traceless" labeling of the target protein.

In one embodiment, R₄ is a targeting group for a target protein, one of R₁ and R₂ is a drug molecule group, an isotope chelator or labeling precursor group, or a fluorescent or molecular labeling group, and the other is a capping group. By virtue of highly specific binding of R₄ to the protein, the compound of formula (I), or a pharmaceutically acceptable salt, stereoisomer, or solvate thereof, rapidly and specifically releases a drug molecule, isotope chelator or labeling precursor, or fluorescent or molecular label, thereby enabling diagnosis and treatment of a disease such as a tumor.

In one embodiment, the compound of formula (I), or a pharmaceutically acceptable salt, stereoisomer or solvate thereof, satisfying one or more of the following conditions:
(a) L₁, at each occurrence, is independently a bond, or -Lₐ-(L_{b})ₘ-L_{c}-(L_{d})ₙ-Lₑ-L_{f}-, wherein Lₐ, L_{b}, L_{c}, L_{d}, Lₑ, L_{f}, at each occurrence, are each independently selected from a bond, -O-, -S-, -SS-, -S(=O)₂-, -NH-, -N(CH₃)-, -C(R^{a})(R^{b})-, -C(=O)-, -C(=O)O-, -OC(=O)-, -C(=O)NH-, - NHC(=O)-, -C(=O)-N(CH₃)-, -N(CH₃)-C(=O)-, -CH₂CH₂-, -CH₂CH₂CH₂-, -CH₂CH₂CH₂CH₂-, -CH₂NH-, -NHCH₂-, -CH₂O-, -OCH₂-, -CH=N-O-, -C(CH₃)=N-NH-, -C(CH₃)=N-NH-C(=O)-, -NH-CH₂-C(=CH-COOH)-, a divalent peptide group containing 1 to 4 amino acids, cyclohexylene, phenylene, heterocycloalkylene (for example, 1,4-piperazinylene), or 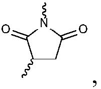 R^{a} and R^{d}, at each occurrence, are each independently selected from H, methyl, ethyl, propyl, isopropyl, phenyl, -OH, -SH, -NH₂, -NH(CH₃), or -NH(CH₃)₂, the subscripts m and n are each independently an integer of 0-4;
(b) the capping group is selected from -L_{g}-(Lₕ)ₙ-Lᵢ-R^{c}, wherein L_{g}, Lₕ, and Lᵢ, at each occurrence, are each independently selected from a bond, -O-, -S-, -C(R^{a})(R^{b})-, -NH-, -N(CH₃)-, -C(=O)-, -CH₂O-, or -OCH₂-, the subscript n is an integer of 0-4, R^{a} and R^{b}, at each occurrence, are independently selected from H, methyl, ethyl, propyl, isopropyl, phenyl, -OH, -SH, -NH₂, - NH(CH₃), or -NH(CH₃)₂, R^{c} is selected from methyl, ethyl, hydroxyl, or thiol;
(c) the drug molecule group is derived from a cytotoxic drug, an immunomodulator, an antibiotic, a protein degrader or a molecular glue;
(d) the isotope chelator or labeling precursor group is selected from 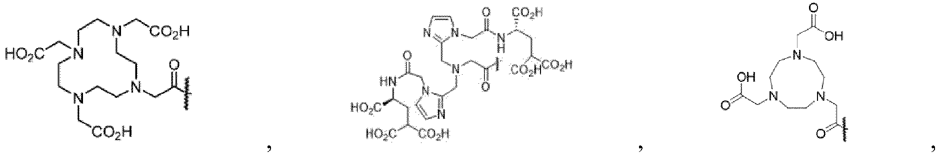 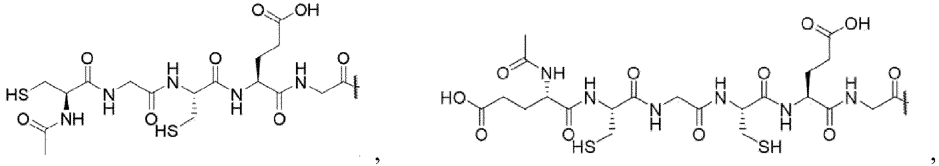 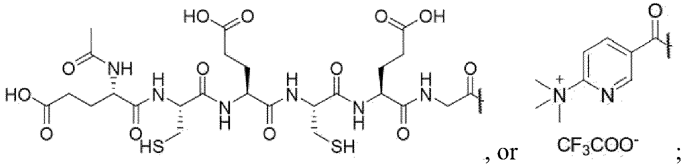
(e) the fluorescent or molecular labeling group is selected from a fluorescent or molecule labeling group, an isotope fluorescent or molecule labeling group, an affinity purification labeling group, or a click-chemistry labeling group;
(f) the targeting group is selected from ligand groups having high affinity for FAP, FOLR1, integrin, folate hydrolase, carbonic anhydrase, or Nectin4

In one embodiment, L₁ is selected from -NH-, -CH₂-, -N(CH₃)-, -C(=O)-, 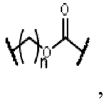 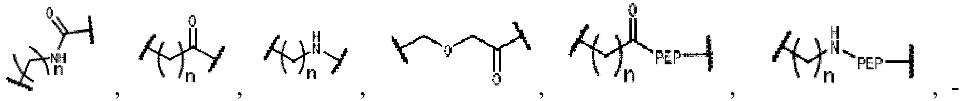 C(=O)-PEP-, -NH-PEP-, 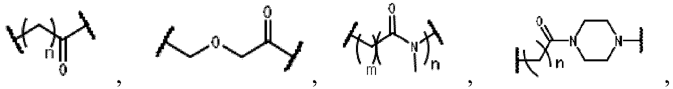 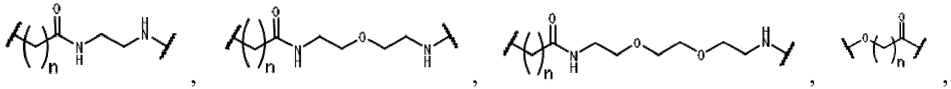 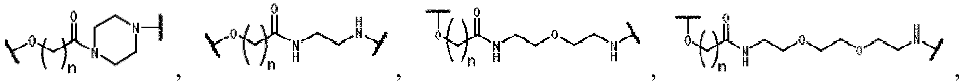 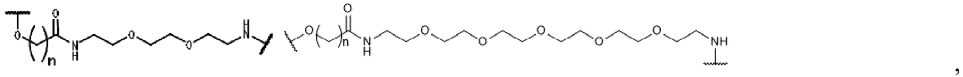 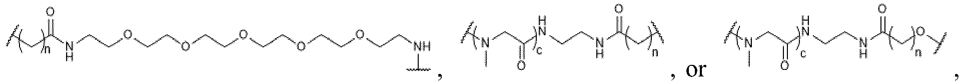 wherein the subscripts m and n are each independently an integer of 0-4, the subscript c is an integer of 1-10, and PEP represents a divalent peptide group containing 1 to 4 amino acids.

In one embodiment, the capping group is selected from methyl, methoxy, 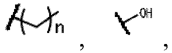 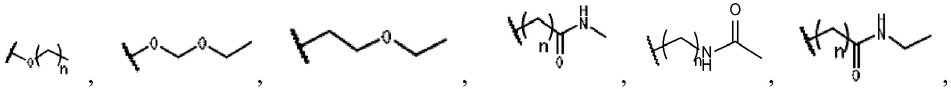 or 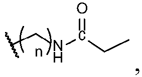 the subscript n is an integer of 1-4.

In one embodiment, L₁ in Z₁ is selected from -NH-, -CH₂-, -N(CH₃)-, -C(=O)-, 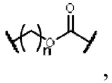 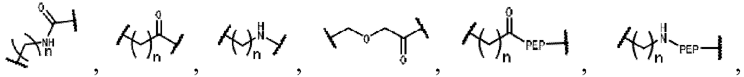 -C(=O)-PEP-, -NH-PEP-, wherein the subscript n is an integer of 1-4 and PEP represents a divalent peptide group containing 1 to 4 amino acids.

In one embodiment, L₁ in X₁ or Y₁ is selected from 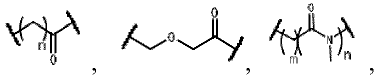 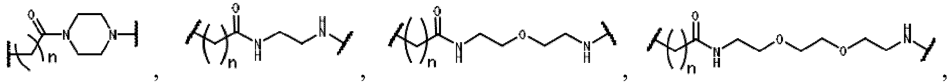 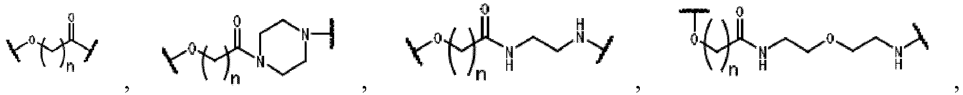 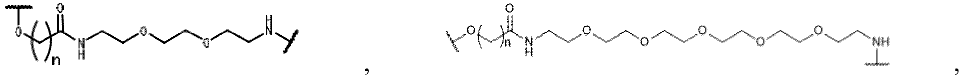 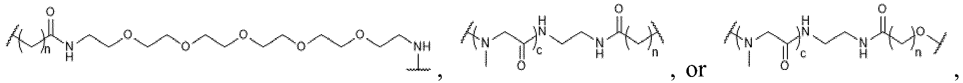 wherein the subscripts m and n are each independently an integer of 0-4 and the subscript c is an integer of 1-10.

In one embodiment, the drug molecule group is selected from:

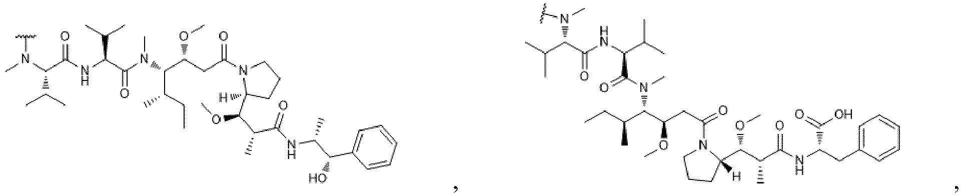

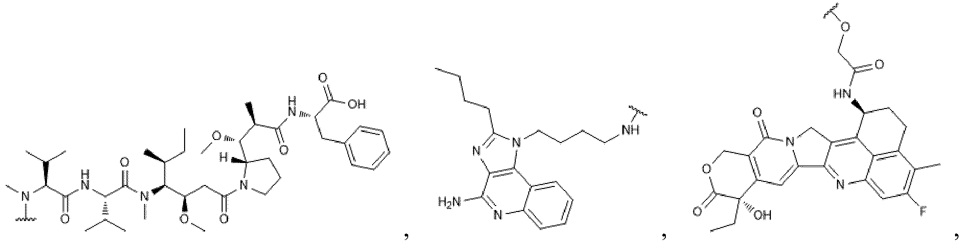

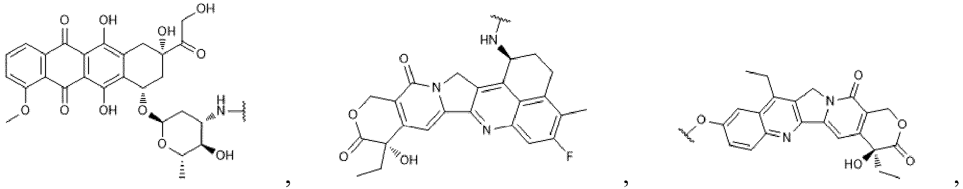

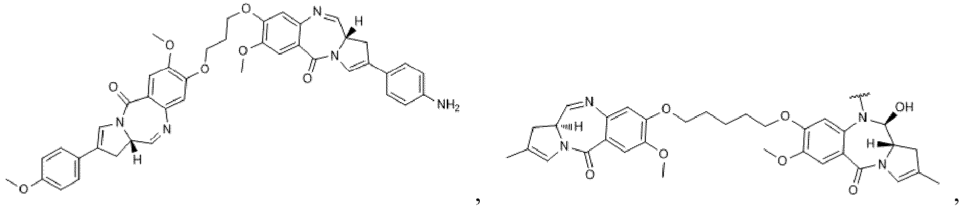

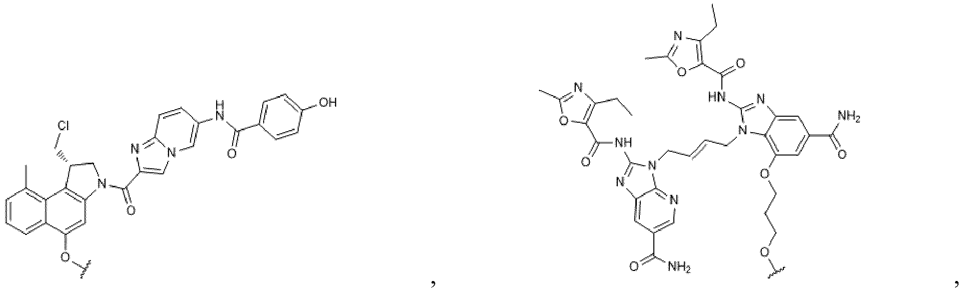

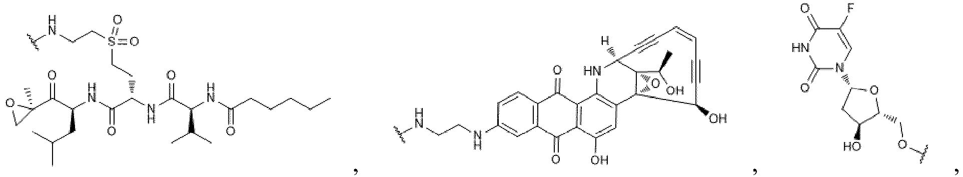

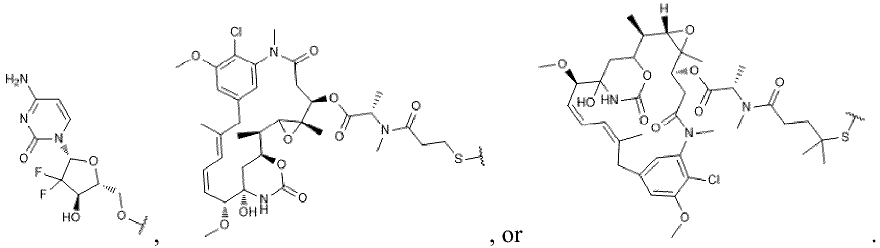

In one embodiment, the isotope chelator or labeling precursor group or the fluorescent or molecular labeling group are selected from:

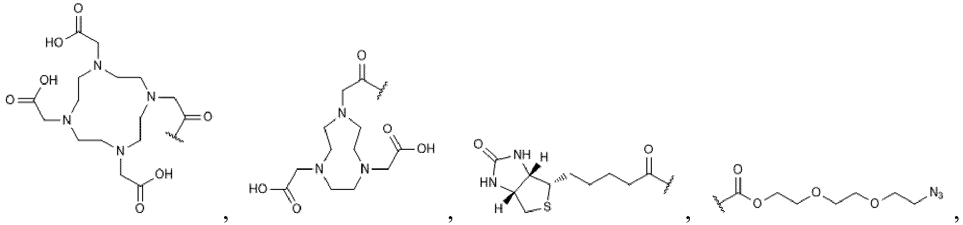

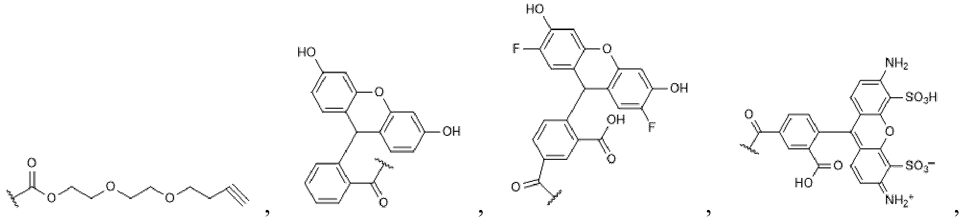

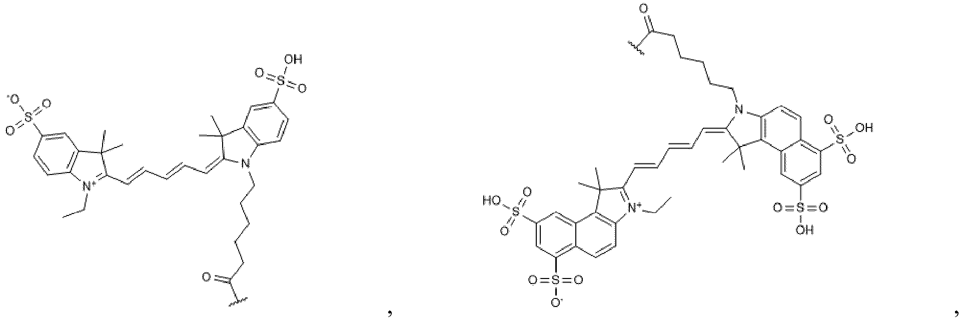

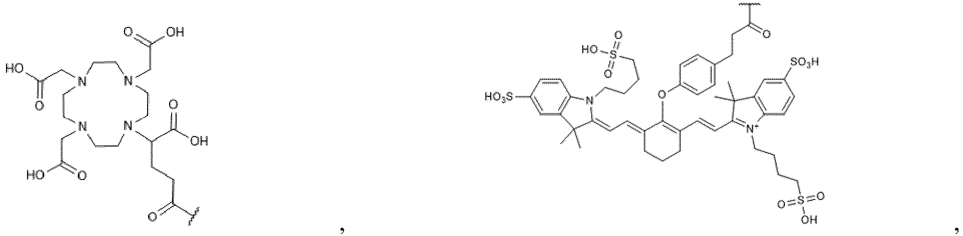

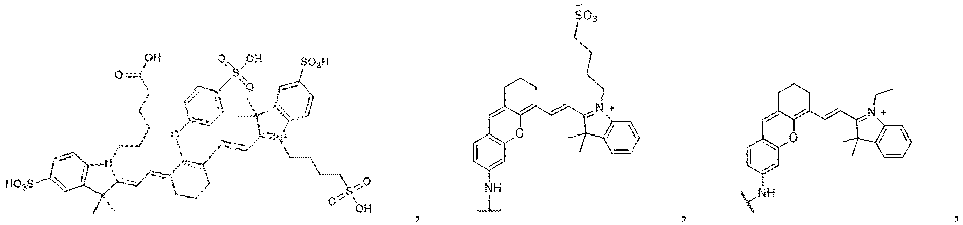

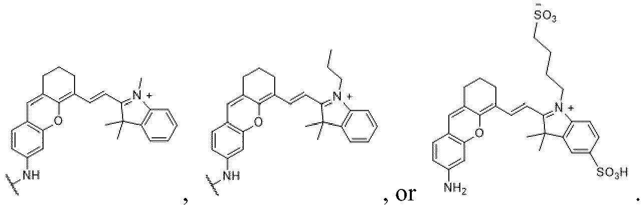

In one embodiment, the targeting group for a target protein is selected from: 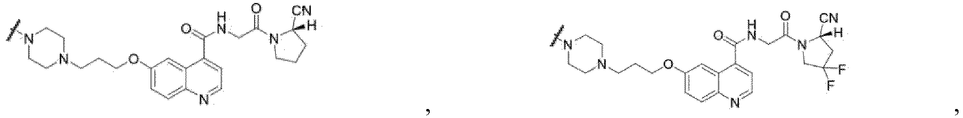 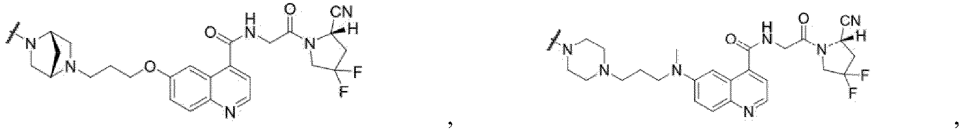 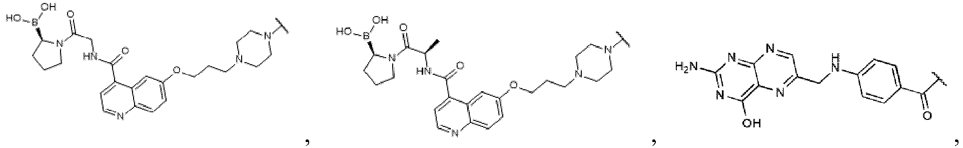 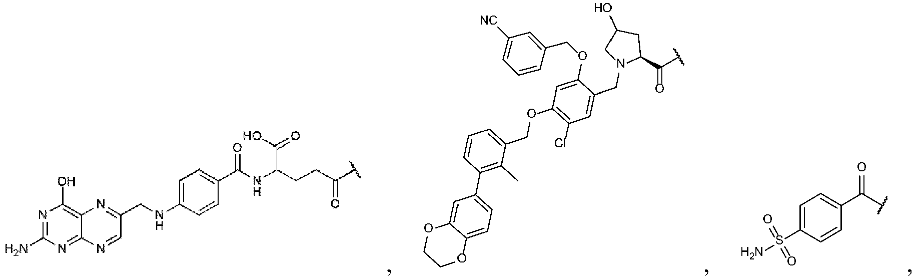 or

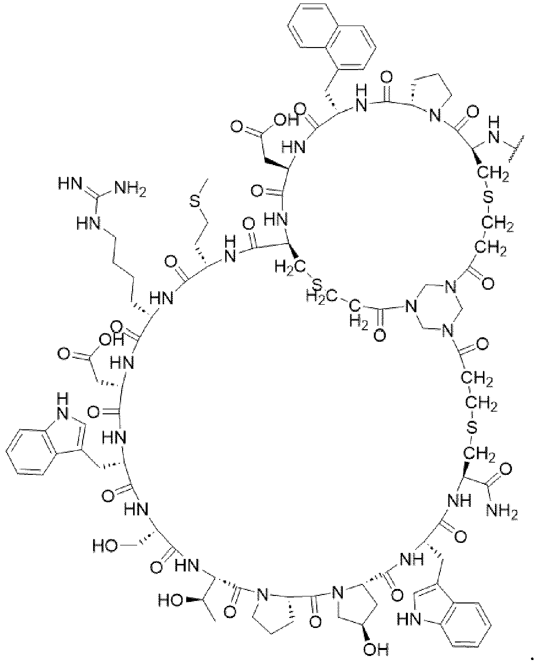

In a preferred embodiment, the targeting group for a target protein is

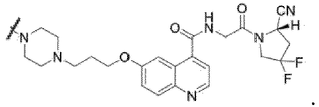

In some embodiments, one of R₁, R₂, and R₄ is an isotope chelator or labeling precursor group; preferably, R₁ or R₂ is an isotope chelator or labeling precursor group.

For example, the compound of formula (I) is

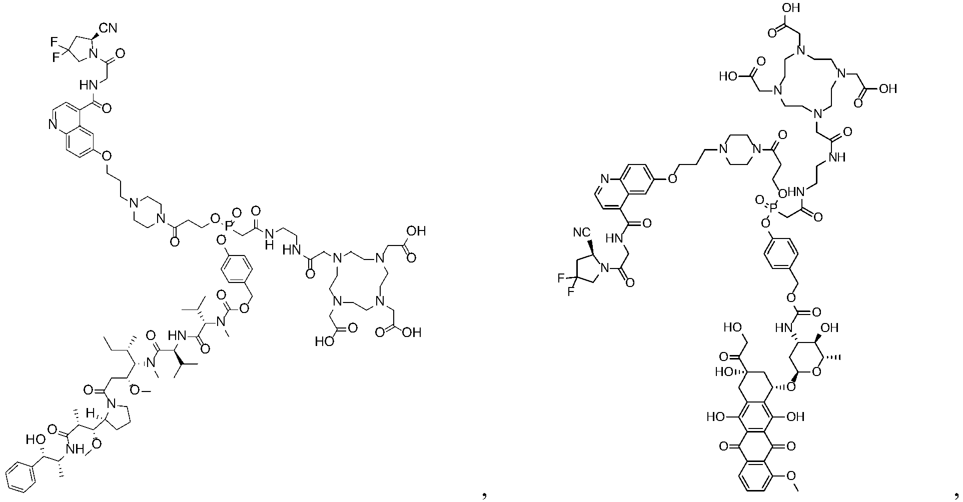

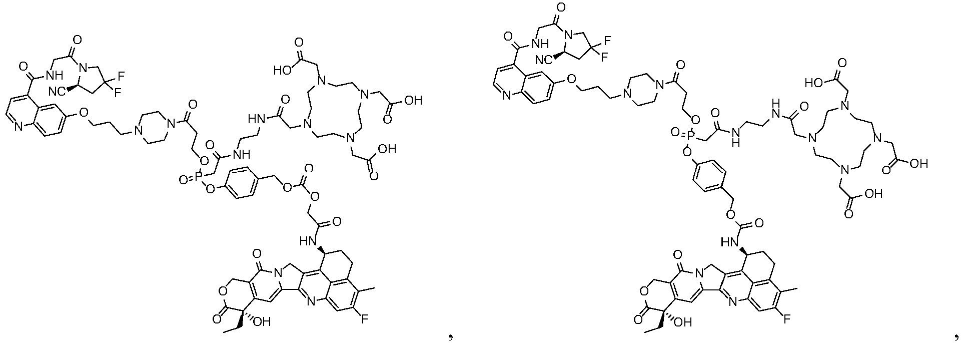

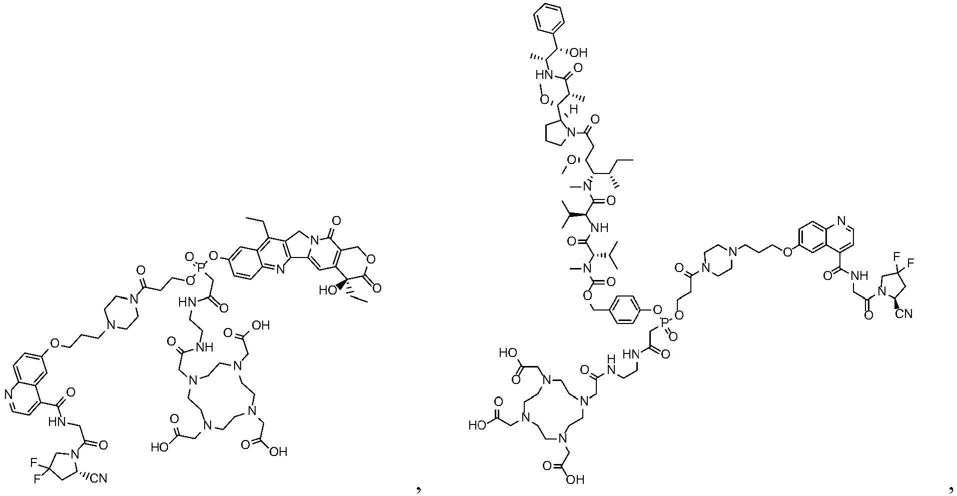

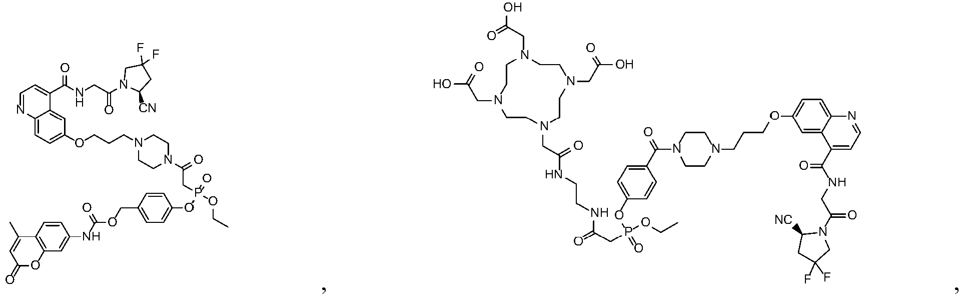

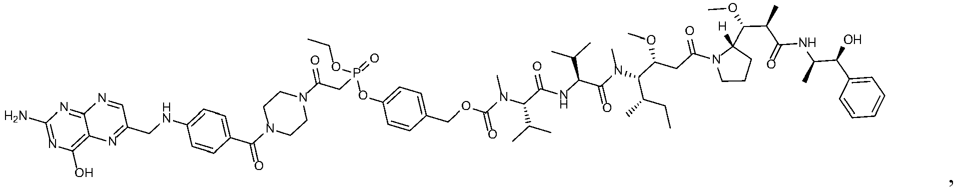

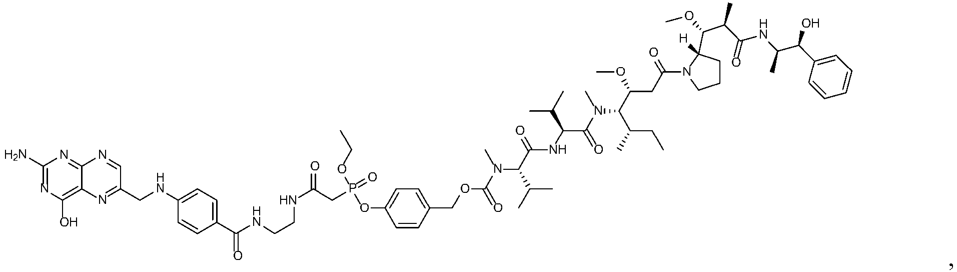

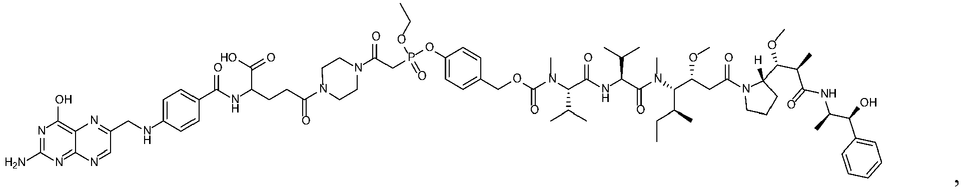

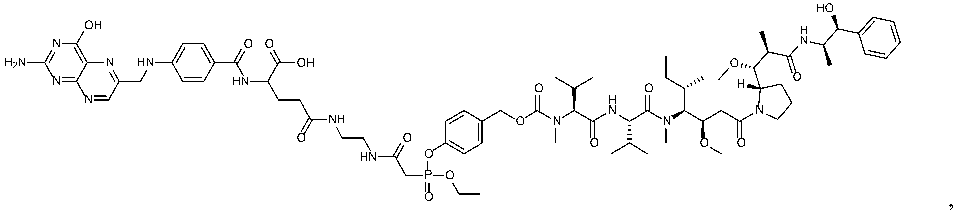

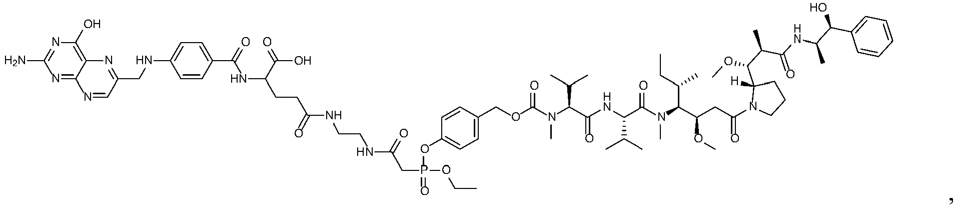

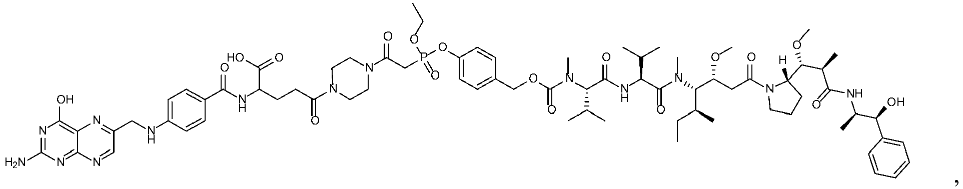

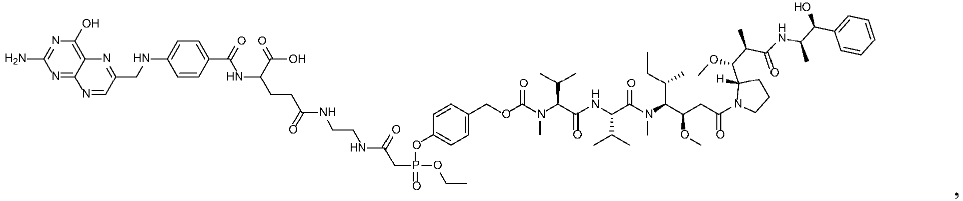

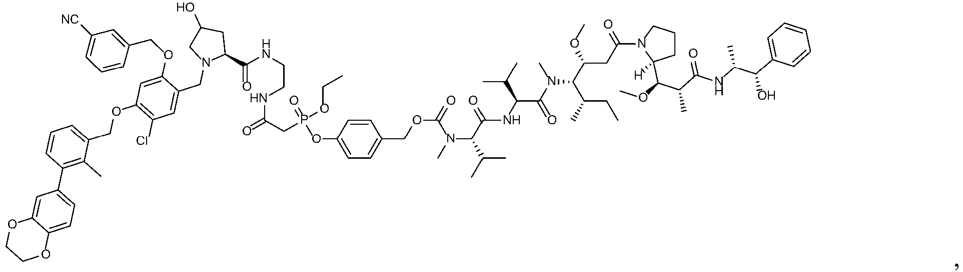

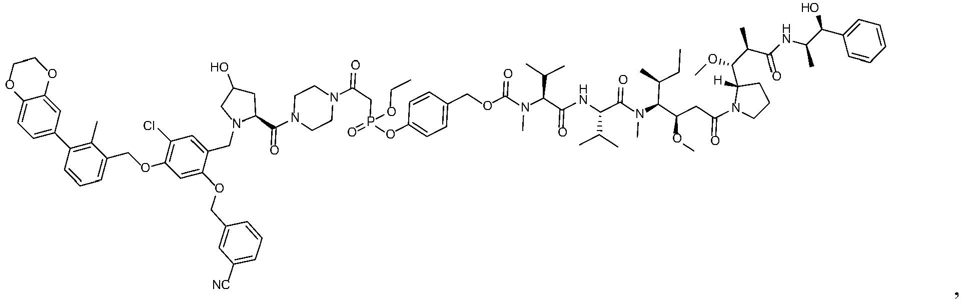

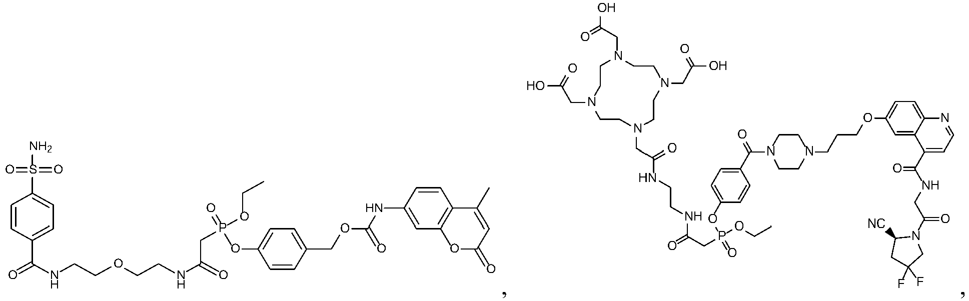

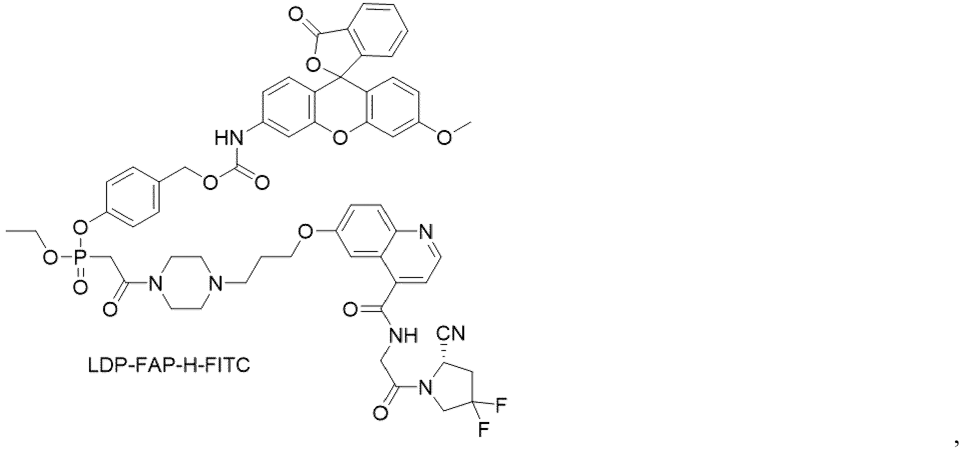

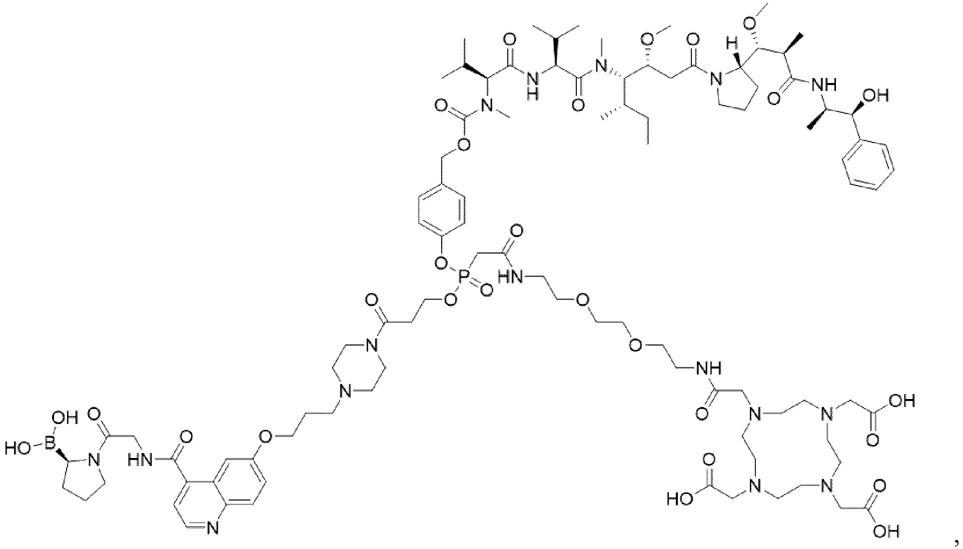

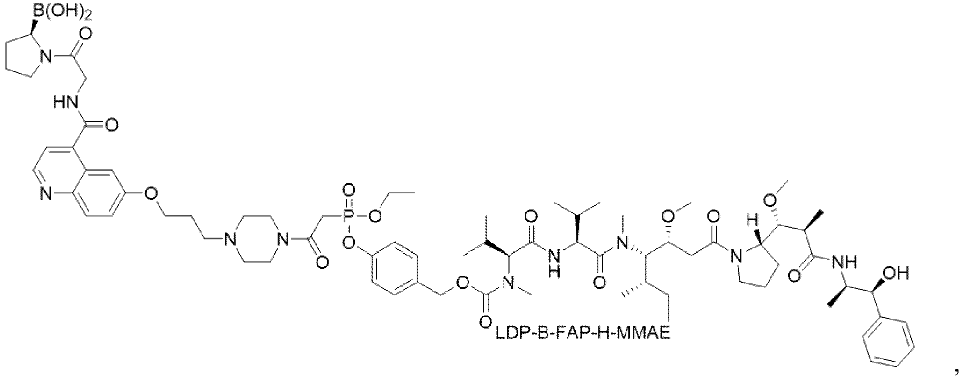

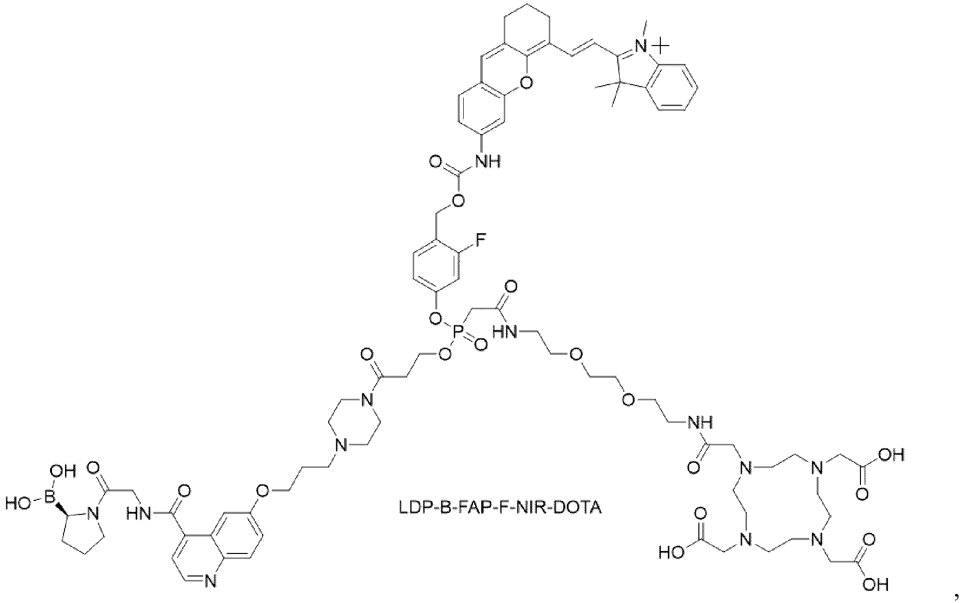

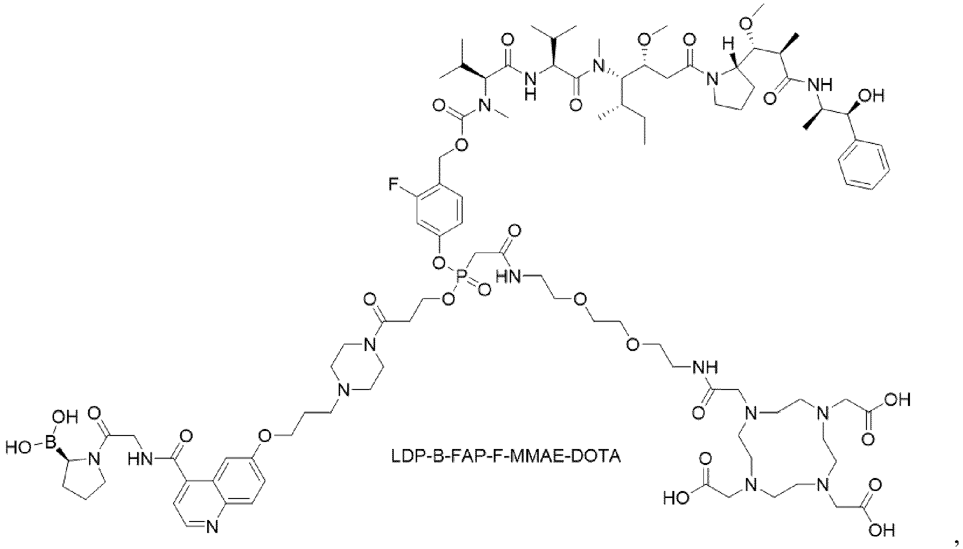

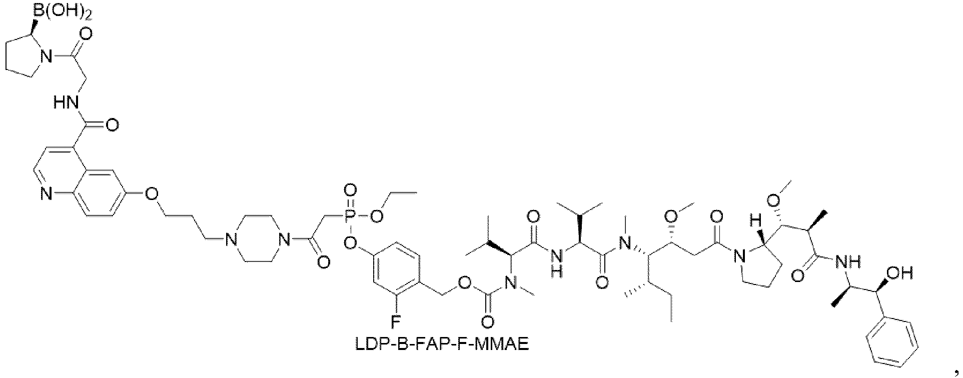

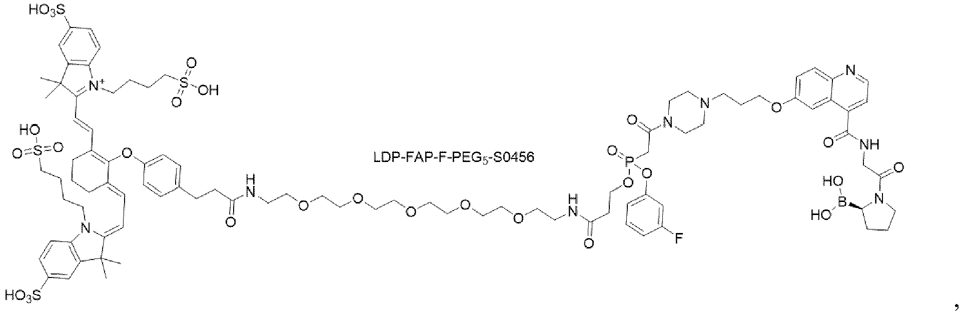

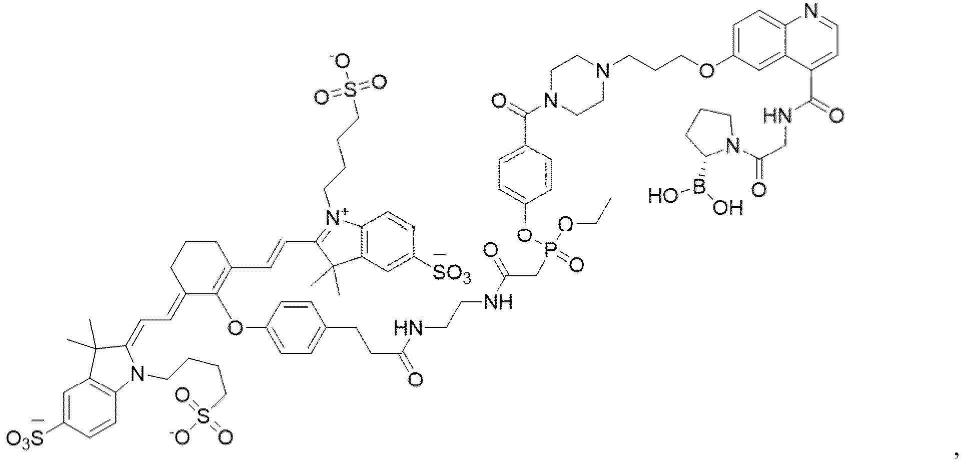

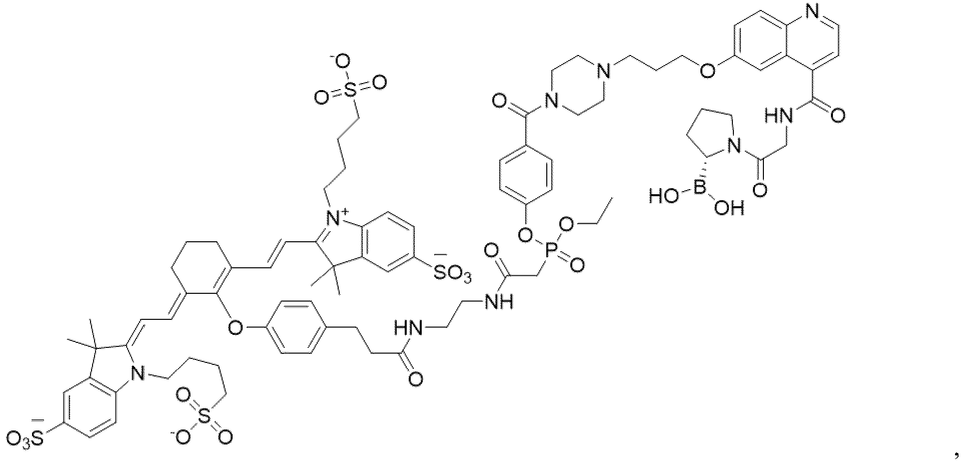

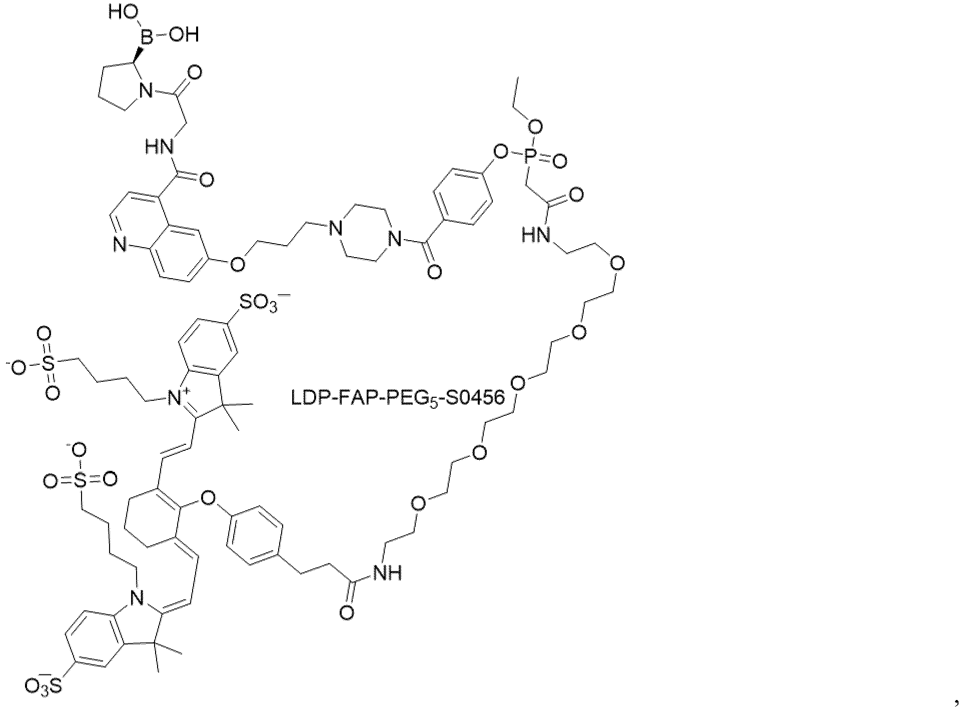

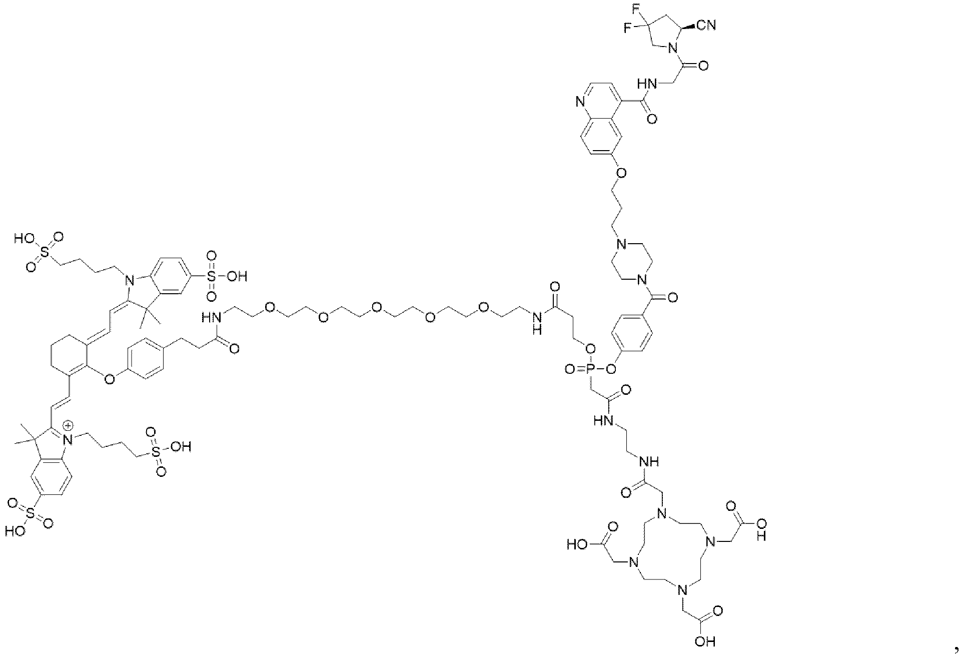

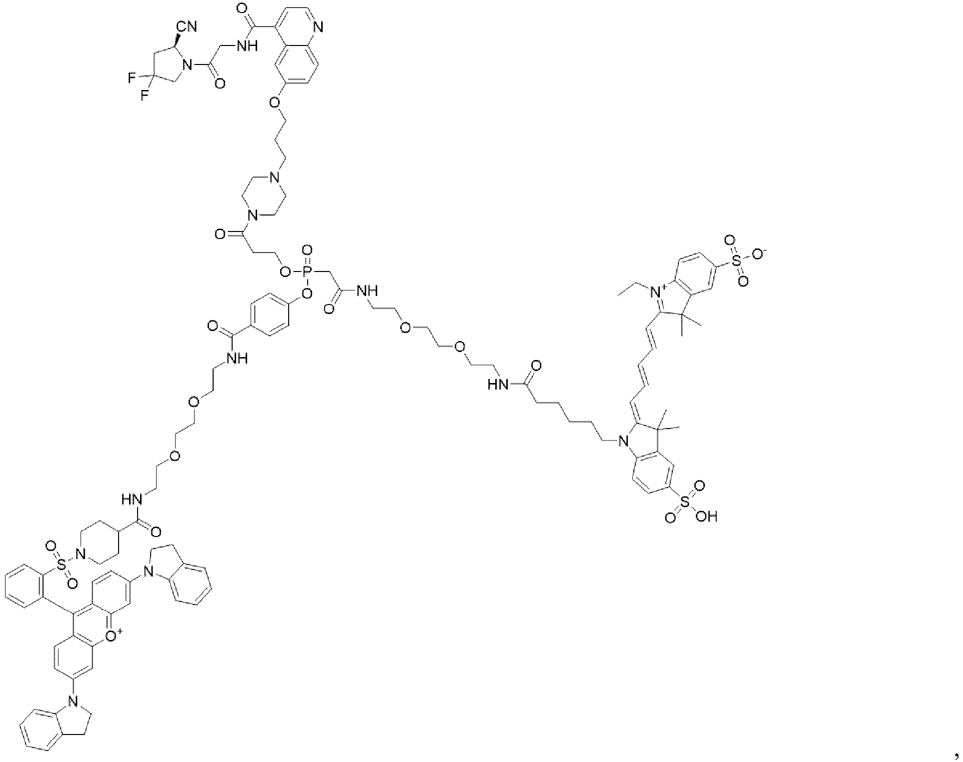

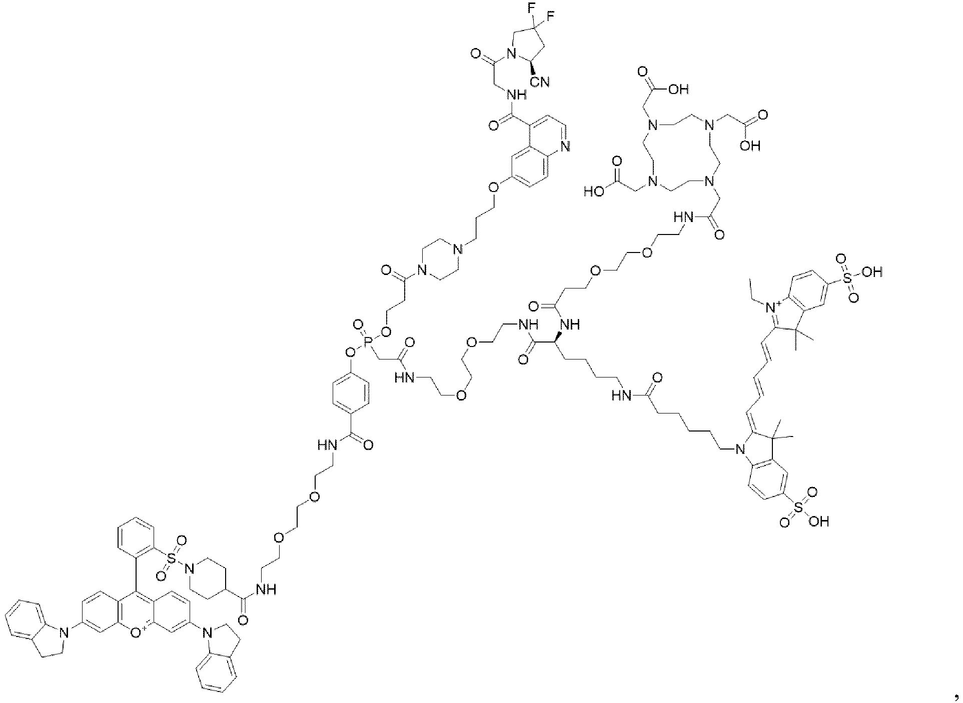

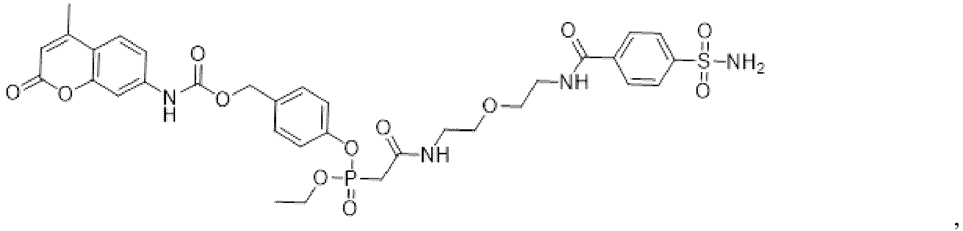

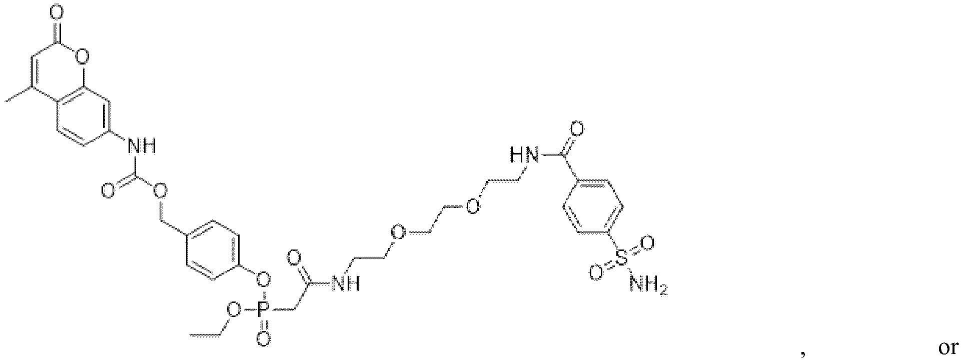

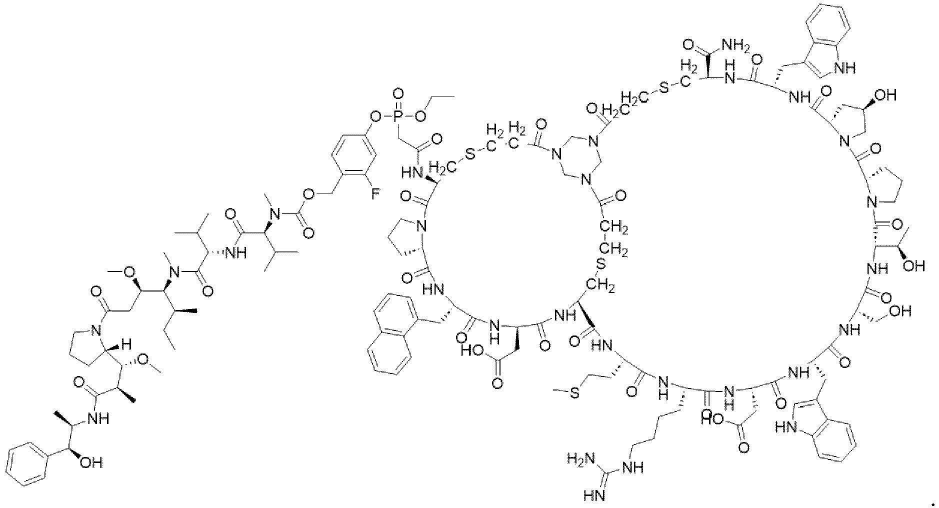

The small-molecule drug conjugate LDP-FAP-MMAE targeting FAP protein can release the drug molecule MMAE after specifically binding to FAP protein.

A compound of formula (I) may be prepared by the reaction shown below or a similar reaction.

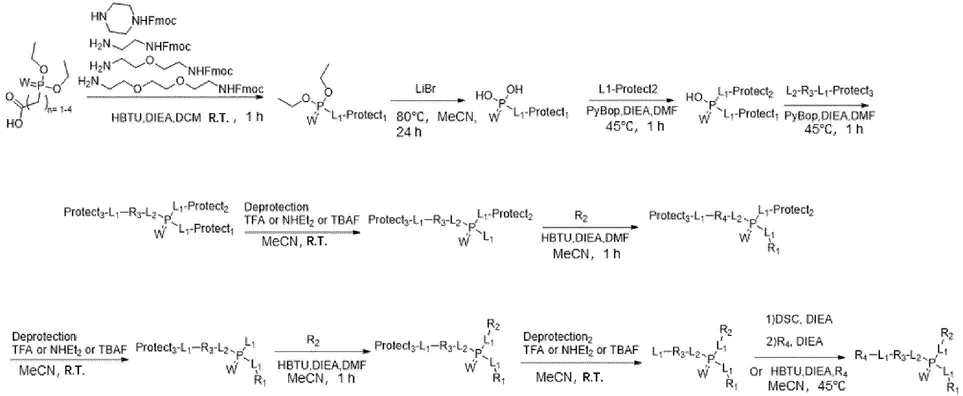

The present disclosure further provides a chelate or nuclide label, comprising:
(i) the compound of formula (I) as described above, or a pharmaceutically acceptable salt, stereoisomer or solvate thereof; and
(ii) a radionuclide.

In some embodiments, in the chelate or nuclide label, the isotope chelator or labeling precursor unit directly chelates the radionuclide (for example, ⁶⁸Ga is chelated by an isotope chelator or labeling precursor unit derived from DOTA), or the radionuclide is introduced indirectly through chelation with another metal (for example, Al³⁺ or Sc³⁺ is chelated by an isotope chelator unit derived from DOTA, and radionuclide ¹⁸F is introduced into the chelate in a form coordinated with the metal ion).

In some embodiments, the radionuclide is selected from: ¹⁸F , ⁵¹Cr, ⁶⁷Ga, ⁶⁸Ga, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, ⁸⁶Y, ⁸⁸Y, ⁹⁰Y, ¹⁴⁹Pm, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg, ²¹¹At, ¹⁵¹Eu, ¹⁵³Eu, ¹⁶⁹Eu, ²⁰¹Tl, ²⁰³Pb, ²¹²Pb, ⁶⁴Cu, ⁶⁷Cu, ¹⁸⁸Re, ¹⁸⁶Re, ¹⁹⁸Au, ²²⁵Ac, ^{227Th}, and ¹⁹⁹Ag. For example, the radionuclide is ⁶⁸Ga.

The present disclosure further provides a pharmaceutical composition, comprising or consisting of:
(1) the compound of formula (I) as described above, or a pharmaceutically acceptable salt, stereoisomer or solvate thereof; or the chelate or nuclide label as described above,
(2) optionally, and a pharmaceutically acceptable excipient.

In one embodiment, the pharmaceutical composition comprises or consists of (1) the compound of formula (I) as described above, or a pharmaceutically acceptable salt, stereoisomer, or solvate thereof; or the chelate or nuclide label as described above. In another embodiment, the pharmaceutical composition comprises or consists of (1) the compound of formula (I) as described above, or a pharmaceutically acceptable salt, stereoisomer, or solvate thereof; or the chelate or nuclide label as described above; and (2) a pharmaceutically acceptable excipient.

The composition of the present disclosure must, or may as needed, further comprise a pharmaceutically acceptable excipient for formulating the chelate or nuclide label for the intended route of administration.

In another aspect, the present invention provides a kit comprising or consisting of:
(i) the compound of formula (I) according to any one of embodiment, or a pharmaceutically acceptable salt, stereoisomer or solvate thereof; or the chelate or nuclide label according to any one of embodiment; or the pharmaceutical composition according to any one of embodiment, and
(ii) instructions for diagnosing or treating a disease.

In some embodiments, the present invention provides a kit comprising or consisting of the compound of formula (I) as described above, or a pharmaceutically acceptable salt, stereoisomer, or solvate thereof; the chelate or nuclide label as described above; or the pharmaceutical composition as described above; and instructions for diagnosing or treating a disease. In a preferred embodiment, the disease is a disease characterized by overexpression of fibroblast activation protein.

In another aspect, the present disclosure relates to the compound of formula (I) as described above, or a pharmaceutically acceptable salt, stereoisomer, or solvate thereof, or the chelate or nuclide label or composition, for use in diagnosing or treating a disease in a mammal or human.

In another aspect, the present disclosure relates to use of the compound of formula (I) as described above, or a pharmaceutically acceptable salt, stereoisomer, or solvate thereof, or the chelate or nuclide label or composition, in the manufacture of a medicament for targeted therapy or diagnosis.

In some embodiments, the disease to be treated by the targeted therapy is a malignant proliferative disease, an immune disease, or an infectious disease.

In some embodiments, the malignant proliferative disease is selected from breast cancer, pancreatic cancer, small intestinal cancer, colon cancer, rectal cancer, lung cancer, head and neck cancer, ovarian cancer, hepatocellular carcinoma, esophageal cancer, hypopharyngeal cancer, nasopharyngeal cancer, laryngeal cancer, myeloma cells, bladder cancer, cholangiocarcinoma, clear cell renal carcinoma, neuroendocrine tumor, oncogenic osteomalacia, sarcoma, cancer of unknown primary, thymic carcinoma, glioma, neuroglioma, astrocytoma, cervical cancer, prostate cancer, thyroid cancer, gastric cancer, bone cancer, lung cancer, lymphoma, endometrial cancer, gallbladder cancer, oral cancer, and testicular cancer.

In some embodiments, the immune disease is selected from: rheumatoid arthritis (RA), ankylosing spondylitis (AS), juvenile idiopathic arthritis (JIA), non-radiographic axial spondyloarthritis (nr-AxSpA), psoriasis, psoriatic arthritis (PsA), Crohn's disease (CD), ulcerative colitis (UC), systemic lupus erythematosus (SLE), lupus nephritis (LN), multiple sclerosis (MS), bronchial asthma, and the like.

In another aspect, the present disclosure relates to a method for targeted therapy or diagnosis, comprising administering to a subject in need thereof the compound of formula (I) as described above, or a pharmaceutically acceptable salt, stereoisomer, or solvate thereof, or the chelate or nuclide label or the composition.

In another aspect, the present disclosure relates to a method for inhibiting overexpression of fibroblast activation protein in a subject in need thereof, the method comprising administering to the subject in need thereof the compound of formula (I) as described above, or a pharmaceutically acceptable salt, stereoisomer or solvate thereof, or the chelate or nuclide label or the composition.

In another aspect, the present disclosure relates to a method for diagnosing or treating a disease characterized by overexpression of fibroblast activation protein, comprising administering to a subject in need thereof the compound of formula (I) as described above, or a pharmaceutically acceptable salt, stereoisomer or solvate thereof, or the chelate or nuclide label or the composition.

In another aspect, the present disclosure relates to the use of the compound of formula (I) as described above, or a pharmaceutically acceptable salt, stereoisomer or solvate thereof, or the chelate or nuclide label or the composition in the manufacture of a medicament for inhibiting overexpression of fibroblast activation protein in a subject in need thereof.

In another aspect, the present disclosure relates to the use of the compound of formula (I) as described above, or a pharmaceutically acceptable salt, stereoisomer or solvate thereof, or the chelate or nuclide label or the composition in the manufacture of a medicament for diagnosing or treating a disease characterized by overexpression of fibroblast activation protein.

In some embodiments, the compound of formula (I) as described above, or a pharmaceutically acceptable salt, stereoisomer, or solvate thereof, or the chelate or nuclide label targets fibroblast activation protein (FAP) and can be used to diagnose or treat a disease characterized by overexpression of fibroblast activation protein. For example, the disease characterized by overexpression of fibroblast activation protein (FAP) is selected from cancer, chronic inflammation, atherosclerosis, fibrosis, tissue remodeling and scarring disease. Preferably, the cancer is selected from breast cancer, pancreatic cancer, small intestine cancer, colon cancer, rectal cancer, lung cancer, head and neck cancer, ovarian cancer, hepatocellular carcinoma, esophageal cancer, hypopharyngeal cancer, nasopharyngeal cancer, laryngeal cancer, myeloma cells, bladder cancer, cholangiocarcinoma, clear cell renal cell carcinoma, neuroendocrine tumor, oncogenic osteomalacia, sarcoma, cancer of unknown primary (CUP), thymic carcinoma, glioma, neuroglioma, astrocytoma, cervical cancer and prostate cancer.

In yet another aspect, the present disclosure relates to a compound of formula (II), or a pharmaceutically acceptable salt, stereoisomer, or solvate thereof, which can be used as an intermediate for preparing a compound of formula (I).

In some embodiments, the compound of formula (II), or a pharmaceutically acceptable salt, stereoisomer, or solvate thereof, has the following structure: 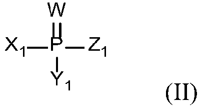 wherein
W is oxygen or sulfur, preferably oxygen;
X₁ is -L₁-R₁;
Y₁ is -L₁-R₂;
Z₁ is -L₂-R₃-L₁-R₄;
L₁, at each occurrence, is independently a bond, a linking group or absent;
L₂ is O, S, or NH, preferably oxygen;
R₃ is an optionally substituted arylene, heteroarylene, cycloalkylene or heterocycloalkylene, the substituent is selected from F, Br, Cl, methoxy, trifluoromethyl, and cyano;
R₁, R₂, R₄ are capping groups;
the capping group is selected from -L_{g}-(Lₕ)ₙ-Lᵢ-R^{c}, wherein L_{g}, Lₕ, Lᵢ, at each occurrence, are each independently selected from a bond, -O-, -S-, -C(R^{a})(R^{b})-, -NH-, -N(CH₃)-, -C(=O)-, - CH₂O-, or -OCH₂-, the subscript n is an integer of 0-4, R^{a} and R^{b}, at each occurrence, are independently selected from H, methyl, ethyl, propyl, isopropyl, phenyl, -OH, -SH, -NH₂, - NH(CH₃), or -NH(CH₃)₂, R^{c} is selected from methyl, ethyl, hydroxyl, or thiol.

In some embodiments, the definitions of the respective groups or moieties in the structure of the compound of formula (II) are the same as those of the corresponding groups or moieties in the compound of formula (I).

In some embodiments, R₃ in the structure of the compound of formula (II) is selected from:
optionally substituted C₅₋₁₄ arylene;
optionally substituted C₅₋₁₄ heteroarylene containing 1 to 3 heteroatoms independently selected from N, O, or S;
optionally substituted divalent saturated or partially unsaturated fused, bridged or spiro C₅₋₁₄ cycloalkylene containing a single ring or multiple rings; or
optionally substituted divalent saturated or partially unsaturated fused, bridged or spiro C₅₋₁₄ heterocycloalkylene containing a single ring or multiple rings, which contains 1 to 3 heteroatoms independently selected from N, O, or S;
the substituent is selected from F, Br, Cl, methoxy, trifluoromethyl, and cyano.

In some embodiments, R₃ in the structure of the compound of formula (II) is specifically selected from optionally substituted 1,2-phenylene, 1,3-phenylene, 1,4-phenylene, 1,2-naphthylene, 1,5-naphthylene, 1,6-naphthylene, 2,6-naphthylene, furanylene, thienylene, pyrrolylene, imidazolylene, pyrazolylene, triazolylene, pyridylene, pyrazinylene, pyrimidinylene, pyridazinylene, cyclopentylene, cyclohexylene, tetrahydrofuranylene, tetrahydropyrrolylene, tetrahydrothienylene, tetrahydropyranylene, piperidinylene, tetrahydrothiopyranylene, dioxanylene, piperazinylene, 1,4-piperazinylene, pyrazinylene, morpholinylene, and dioxanylene; the substituent is selected from F, Br, Cl, methoxy, trifluoromethyl, and cyano.

In some embodiments, R₃ is preferably 3-X-1,4-phenylene, 2-X-1,4-phenylene, 2-X-1,5-phenylene, 3-X-1,5-phenylene, 3-X-1-phenylene, 2-X-1-phenylene, 4-X-1-phenylene; X is selected from F, Br, Cl, methoxy, trifluoromethyl, and cyano.

In yet another aspect, the present disclosure relates to use of a compound of formula (II) as described above as an intermediate in preparing the compound of formula (I).

In yet another aspect, the present disclosure relates to a method for preparing the compound of formula (I) as described above, comprising using a compound of formula (II) as an intermediate. In yet another aspect, the present disclosure relates to use of a compound of formula (II) in the manufacture of a medicament for targeted therapy or diagnosis.

### Example

The starting materials used in the Examples are commercially available and/or may be prepared by various methods well known to those skilled in the art of organic synthesis. A person skilled in the art of organic synthesis will appropriately select reaction conditions (including solvent, reaction atmosphere, reaction temperature, duration of the experiment, and post treatment) in the synthetic methods described below. A person skilled in the art of organic synthesis will understand that the functional groups present in the various portions of a molecule should be compatible with the proposed reagents and reactions.

### Example 1: LDP-FAP-MMAE

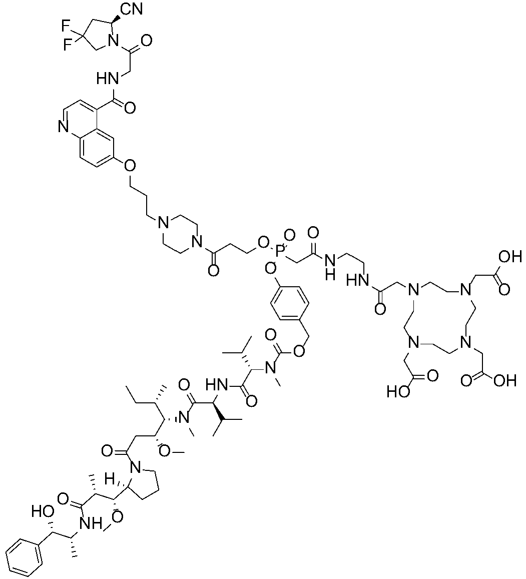

### Preparation of LDP-FAP-MMAE

LDP-FAP-MMAE may be prepared according to the foregoing Reaction Scheme (1), with the specific reaction shown below.

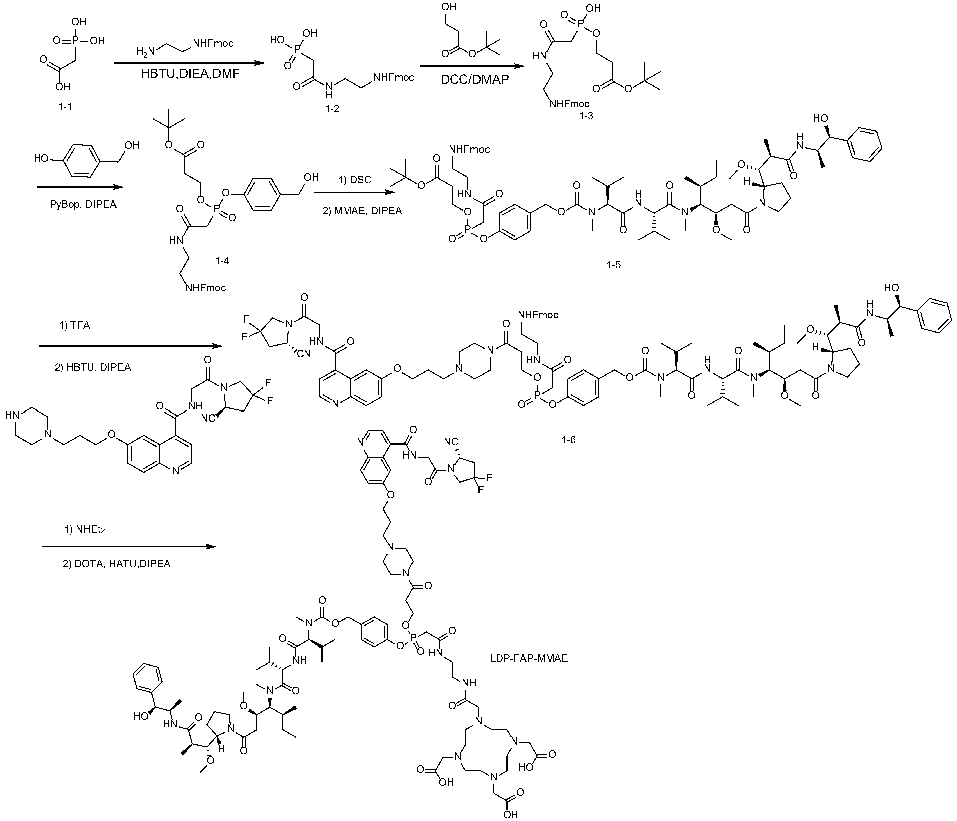

1-1 (1 equivalent) was dissolved in DMF, and N-Fmoc-ethylenediamine (1.2 equivalents), HBTU (1.2 equivalents), and DIPEA (3 equivalents) were added. The reaction was conducted at room temperature for 30 min. The solvent was removed by evaporation, and an aqueous NaOH solution was added for dissolution. After washing three times with ethyl acetate, the aqueous phase was adjusted to acidic with dilute hydrochloric acid, whereupon a precipitate formed. The precipitate was filtered to give 1-2. The product was characterized by UPLC-MS, and the measured molecular weight was 404.11.

1-2 (1 equivalent) was dissolved in DMF, and tert-butyl 3-hydroxypropionate (1 equivalent), DCC (2.2 equivalents), and DMAP (0.05 equivalent) were added. After reaction for 1 h, the mixture was filtered and the filtrate was collected. The solvent was removed by evaporation, and an aqueous NaOH solution was added for dissolution. After washing three times with ethyl acetate, the aqueous phase was adjusted to acidic with dilute hydrochloric acid, whereupon a precipitate formed. The precipitate was filtered to give 2-3. The product was characterized by UPLC-MS, and the measured molecular weight was 532.20.

1-3 (1 equivalent) was dissolved in DMF, and p-hydroxybenzyl alcohol (1.2 equivalents), PyBop (1.2 equivalents), and DIPEA (3 equivalents) were added. The reaction was conducted at room temperature for 30 min, followed by separation on a silica gel column using MeOH:DCM = 1:20 to give 1-4. The product was characterized by UPLC-MS, and the measured molecular weight was 638.24.

1-4 (1 equivalent) was dissolved in DMF, and DSC (1.1 equivalents) and DIPEA (3 equivalents) were added. After reaction at room temperature for 5 h, MMAE (1 equivalent) was added. After reaction at room temperature for 30 min, 1-5 was separated by reverse-phase HPLC. The product was characterized by UPLC-MS, and the measured molecular weight was 1381.72.

1-5 (1 equivalent) was dissolved in MeCN, TFA was added, and the reaction was conducted for 30 min, followed by evaporation to dryness. TsOH was added to FAP-int (1 equivalent) to remove the protecting group. The resulting material was dissolved in MeCN and mixed with deprotected 1-5, and HBTU (1.2 equivalents) and DIPEA (3 equivalents) were added. After reaction for 30 min, the mixture was separated by reverse-phase HPLC to give 1-6. The product was characterized by UPLC-MS, and the measured molecular weight was 1793.87.

FAP-int: ¹H NMR (400 MHz, DMSO-d6) δ ppm 1.41 (s, 9H), 2.27-2.33 (m, 2H), 3.27-3.29 (m, 5H), 3.43-3.55 (m, 2H), 3.93-4.21 (m, 10H), 5.19 (dd, J = 9.26, 2.75 Hz, 1H), 7.59 (dd, J = 9.26, 2.63 Hz, 1H), 7.69 (d, J = 4.63 Hz, 1H), 7.87-8.00 (m, 1H), 8.14 (d, J = 9.26 Hz, 1H), 8.95 (d, J = 4.63 Hz, 1H), 9.24 ( t, J = 5.82 Hz, 1H), 11.00 (brs, 1H).

Diethylamine was added to 1-6 (1 equivalent) to remove the protecting group, followed by evaporation to dryness. DOTA (10 equivalents), HATU (2 equivalents), and DIPEA (3 equivalents) were added, and the reaction was conducted for 1 h. The mixture was separated by reverse-phase HPLC to give LDP-FAP-MMAE. The product was characterized by UPLC-MS and high-resolution mass spectrometry, and the measured molecular weight was [C₉₄H₁₃₉F₂KN₁₇O₂₄P]²⁺ = 998.97, t_{R} = 2.04 min. Throughout the present disclosure, the analytical column used for UPLC-MS was an ACQUITY UPLC BEH C18, 1.7 micrometers, 2.1 x 50 mm column, and the method was: 0-0.2 min, 90% H₂O (0.1% FA) + 10% MeCN; 0.2-3 min, linear change to 100% MeCN; 3-4 min, 100% MeCN; 4-4.5 min, linear change to 90% H₂O (0.1% FA) + 10% MeCN; and 4.5-5 min, 90% H₂O (0.1% FA) + 10% MeCN.

### FAP protein-mediated specific release of MMAE from LDP-FAP-MMAE

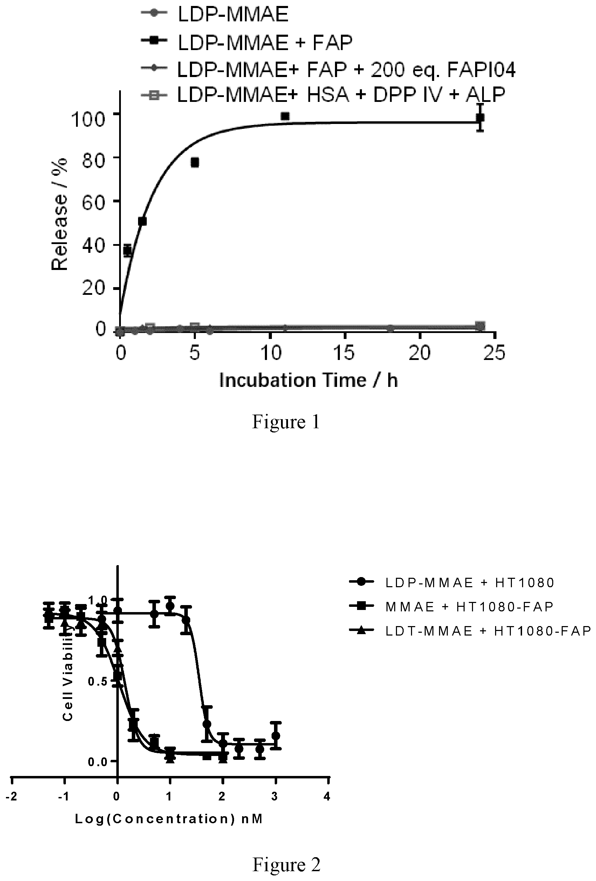

FAP protein (5 µM) and LDP-FAP-MMAE (250 nM) were co-incubated in PBS at pH 7.4 and 37°C, and MMAE release was measured by UPLC-MS (SIR M/Z = 719) at 0 min, 30 min, 90 min, 5 h, 11 h, and 24 h. LDP-FAP-MMAE (500 nM) was incubated in PBS at pH 7.4 and 37°C, and MMAE release was measured by UPLC-MS (SIR M/Z = 719) at 0 h, 1 h, 2 h, 4 h, 6 h, 18 h, and 24 h. FAP protein (5 µM), LDP-FAP-MMAE (250 nM), and FAPI-04 (100 µM) were co-incubated in PBS at pH 7.4 and 37°C, and MMAE release was measured by UPLC-MS (SIR M/Z = 719) at 0 h, 1 h, 2 h, 5 h, 11 h, and 24 h. FAP protein (5 µM), LDP-FAP-MMAE (250 nM), human serum albumin HSA (45 g/L), DPP IV (5 µM; a homologous protein of FAP), and alkaline phosphatase ALP (5 µM) were co-incubated in PBS at pH 7.4 and 37°C, and MMAE release was measured by UPLC-MS (SIR M/Z = 719) at 0 h, 2 h, 5 h, and 24 h. The experiment showed that LDP-FAP-MMAE rapidly released MMAE only when FAP protein was added, and the release was rapid and highly specific. The test results are shown in FIG. 1.

### LDP-FAP-MMAE releases MMAE in FAP-positive cells to kill the cells

LDP-FAP-MMAE was prepared in concentration gradients of 0, 0.5, 1, 5, 10, 20, 50, 100, 200, 500, and 1000 nM and of 0, 0.05, 0.1, 0.2, 0.5, 1, 2, 5, 10, and 50 nM, and was respectively co-incubated with HT1080-FAP cells and HT1080 cells in 96-well plates for 48 h (5000 cells per well). MMAE was prepared in a concentration gradient of 0, 0.05, 0.1, 0.2, 0.5, 1, 2, 5, 10, 50, and 100 nM and co-incubated with HT1080-FAP cells. After 48 h, the medium was replaced with a medium containing 10% CCK-8. After incubation for 1 h, absorbance at 450 nm was measured with a microplate reader, and cell viability was calculated according to the CCK-8 formula: cell proliferation rate (%) = (OD experimental group - OD blank group)/(OD control group - OD blank group) x 100%. The experiment showed that the EC50 of LDP-FAP-MMAE for HT1080-FAP cells was 1.4 nM, the EC50 of MMAE for HT1080-FAP cells was 1.1 nM, and the EC50 of LDP-FAP-MMAE for HT1080 cells was 35.6 nM. The test results are shown in FIG. 2.

### Specific release of MMAE from LDP-FAP-MMAE in vivo

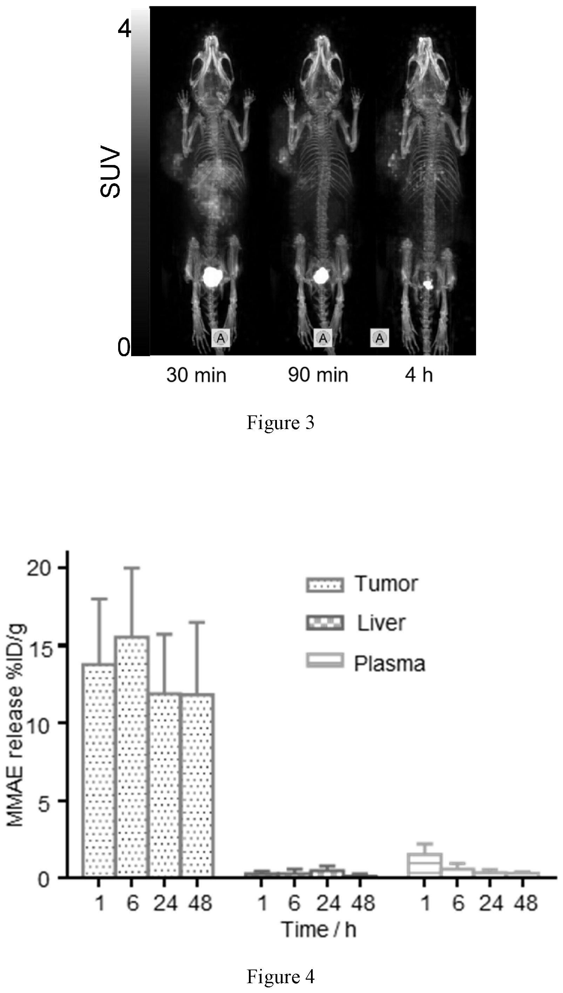

A germanium-gallium generator was eluted with 5 mL of 0.6 M high-purity hydrochloric acid to obtain a Ga-68 hydrochloric acid solution. 1 mL of the eluted Ga-68 solution was taken, and 100 microliters of 3 M sodium hydroxide and 130 microliters of 3 M sodium acetate were added to adjust the acidity to a final pH of 4.0. Fifty micrograms of TEFAPI-06 precursor was added, and the reaction mixture was heated to 90°C and maintained for 10 min. The reaction solution was passed through a C18 cartridge to remove free ions, and the C18 column was then eluted with an ethanol solution to obtain labeled ⁶⁸Ga-LDP-FAP-MMAE. 37 MBq of the labeled product was diluted with 200 microliters of normal saline and drawn into an insulin syringe for use; the ethanol content of the drug was no more than 5%. Healthy mice were anesthetized in advance and placed on the acquisition bed of a PET/CT scanner, and an indwelling needle was placed in the tail vein. The syringe was connected to the indwelling needle, and PET data acquisition was started at time zero when injection began. Acquisition times were 30 min, 90 min, and 4 h. The test results are shown in FIG. 3.

After the tumors of HT1080-FAP tumor-bearing mice reached 200-300 mm³, each mouse was administered LDP-FAP-MMAE (0.2 mg/kg). The mice (n = 5 per group) were dissected at 1 h, 6 h, 24 h, and 48 h. Plasma, liver, and tumor samples were homogenized and extracted with methanol, and the MMAE content in the tissues was measured by UPLC-MS. The experimental results showed that LDP-FAP-MMAE released a large amount of MMAE only in the tumor. The liver exhibited high uptake of LDP-FAP-MMAE but did not release MMAE, demonstrating specific release. The test results are shown in FIG. 4.

### Treatment of HT1080-FAP tumor-bearing mice with LDP-FAP-MMAE

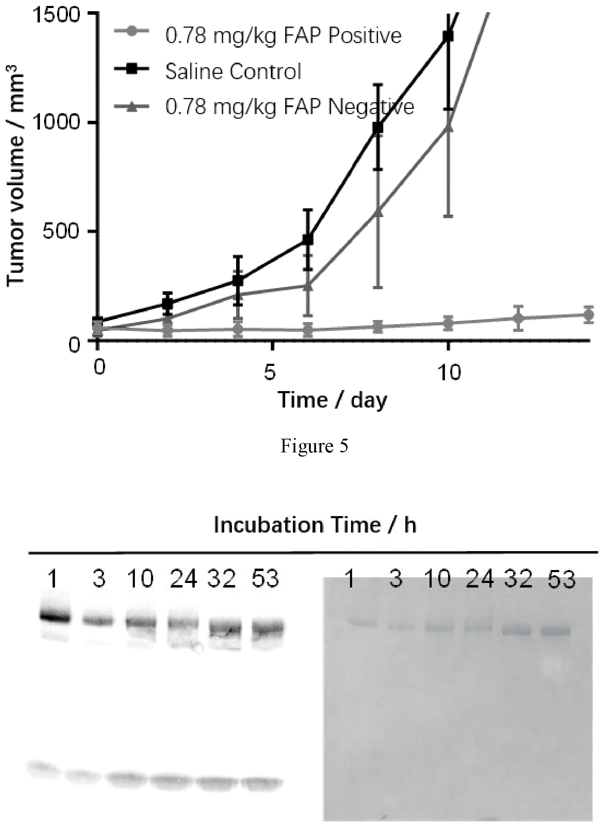

HT1080-FAP tumor-bearing mice and HT1080 tumor-bearing mice were treated when the tumor size was 50-100 mm³ (n = 5; single dose of 0.78 mg/kg), with administration on day 0 and day 4. After treatment, it can be observed that the growth of the FAP-positive tumors was markedly inhibited. The test results are shown in FIG. 5.

### Example 2: LDP-FAPI-DOTA

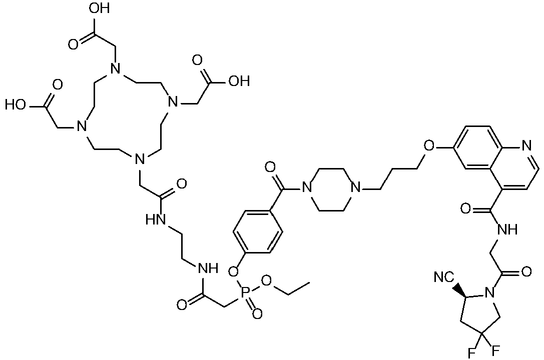

### LDP-FAPI-DOTA may be prepared according to the foregoing Reaction Scheme (1).

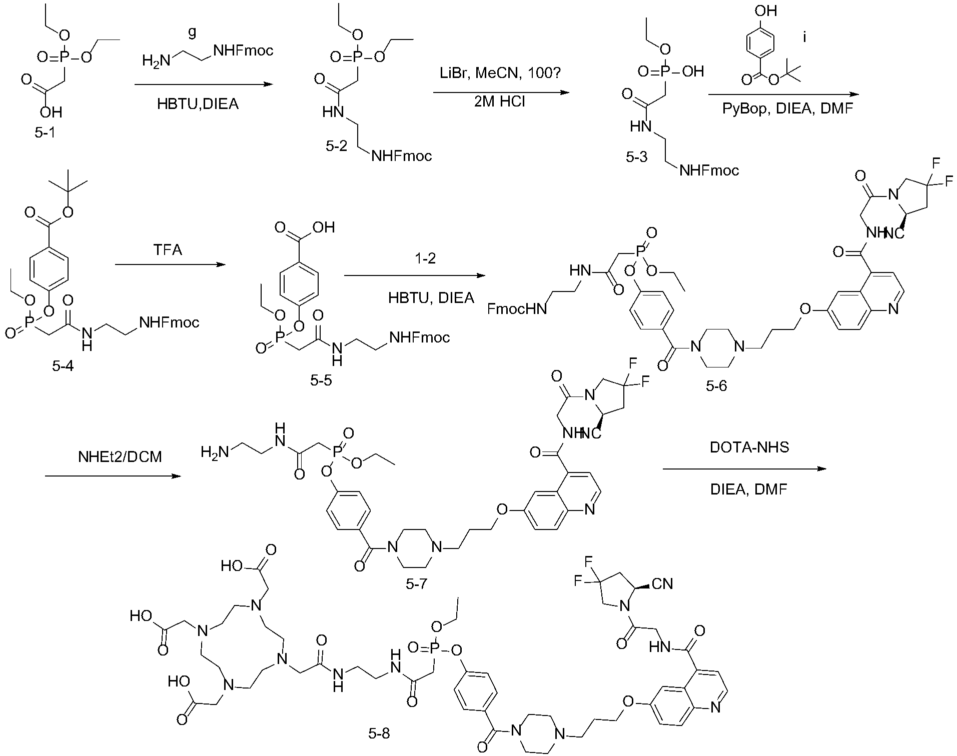

### Synthetic steps:

5-1 (50 mg, 1 equivalent) was dissolved in MeCN, and HBTU (134 mg, 1.2 equivalents), DIPEA (114 mg, 3 equivalents), and reactant g (100 mg, 1.2 equivalents) were added. After reaction at room temperature for 30 min, the mixture was separated on a silica gel column (DCM:MeOH = 10:1) and evaporated to dryness to give 5-2 in a yield of 81%.

5-2 (95 mg) was dissolved in MeCN, LiBr was added, and the mixture was heated at 100°C under nitrogen protection for 5 h. A white precipitate gradually formed during the reaction and was filtered to give product 5-3 in a yield of 40%.

5-3 (36 mg, 1 equivalent) was dissolved in DMF, and starting material i (19.4 mg, 1.2 equivalents), PyBop (52 mg, 1.2 equivalents), and DIPEA (32 mg, 3 equivalents) were added. After reaction at room temperature for 1 h, the mixture was separated on a silica gel column (DCM:MeOH = 20:1) and evaporated to dryness to give product 5-4 in a yield of 83%.

5-4 (43 mg) was dissolved in DCM, TFA was added for deprotection, and the mixture was evaporated to dryness to give product 5-5 in a yield of 95%.

5-5 (6.8 mg, 1.2 equivalents) was dissolved in MeCN, and 1-2 (5 mg, 1 equivalent), HBTU (4.3 mg, 1.2 equivalents), and DIPEA (4 mg, 3 equivalents) were added. After reaction at room temperature for 30 min, the mixture was separated by HPLC and lyophilized to give 5-6 in a yield of 62%. (Method: 0 min: 90% H₂O + 10% MeCN; 12 min: 50% H₂O + 50% MeCN; 15 min: 10% H₂O + 90% MeCN; 17 min: 90% H₂O + 10% MeCN.)

5-6 (6.5 mg, 1 equivalent) was dissolved in MeCN, and NHEt2 was added. After deprotection, the mixture was separated by HPLC and lyophilized to give 5-7 in a yield of 75%. (Method: 0 min: 90% H₂O + 10% MeCN; 12 min: 50% H₂O + 50% MeCN; 15 min: 10% H₂O + 90% MeCN; 17 min: 90% H₂O + 10% MeCN.)

5-7 (3.8 mg, 1 equivalent) was dissolved in DMF, and DOTA-NHS (2.9 mg, 1.2 equivalents) and DIPEA (3.1 mg, 5 equivalents) were added. After reaction at room temperature for 1 h, the mixture was separated by HPLC and lyophilized to give 5-8 (LDP-FAPI-DOTA). (Method: 0 min: 90% H₂O + 10% MeCN; 12 min: 50% H₂O + 50% MeCN; 15 min: 10% H₂O + 90% MeCN; 17 min: 90% H₂O + 10% MeCN; separation column: XBridge^{®} C18, 5µm, 4.6×150 mm.) The product was characterized by UPLC-MS, and the measured molecular weight was [C₅₃H₇₂F₂K₂N₁₂O₁₅P]²⁺ = 612.22, t_{R} = 1.56 min.

Experiments showed that, after binding to FAP protein, LDP-FAPI-DOTA can covalently label the FAP protein with DOTA, thereby achieving covalent delivery of a nuclide, increasing uptake of a small-molecule radiopharmaceutical at a tumor site, and prolonging the retention time of the retained nuclide at the tumor site.

### LDP-FAPI-DOTA can achieve covalent attachment to FAP protein

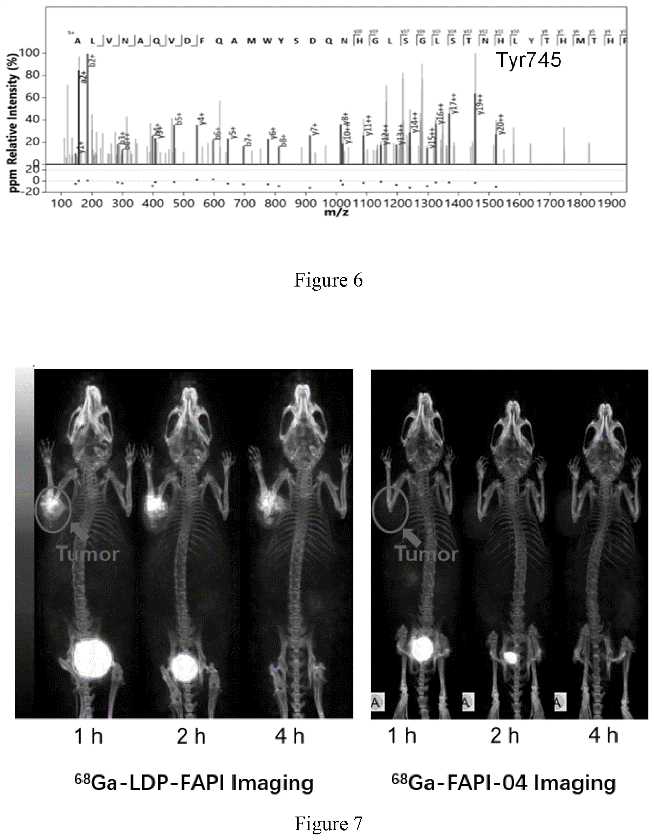

FAP protein and ¹⁷⁷Lu-labeled LDP-FAPI-DOTA were co-incubated at a ratio of 5:1 at 37°C and pH 7.4. Samples were taken at 1, 3, 10, 24, 32, and 53 h for SDS-PAGE. Autoradiography revealed clear radioactivity at the protein band (see FIG. 6), indicating that covalent attachment to FAP protein was achieved. The covalent site was identified by tandem mass spectrometry.

### LDP-FAPI-DOTA can increase tumor uptake

The same HT1080-FAP tumor-bearing mouse was administered the compounds intravenously via the tail vein. PET imaging with ⁶⁸Ga-FAPI-04 was first performed, and PET imaging with ⁶⁸Ga-LDP-FAPI-DOTA was performed 24 h later. As shown in FIG. 7, it can be seen that tumor uptake of LDP-FAPI-DOTA was markedly higher than that of FAPI-04.

### Example 3: LDP-FAP-H-FITC, LDP-B-FAP-H-MMAE, LDP-B-FAP-F-MMAE, LDP-B-FAP-F-NIR-DOTA, LDP-B-FAP-H-MMAE-DOTA and LDP-B-FAP-F-MMAE-DOTA

### LDP-FAP-H-FITC

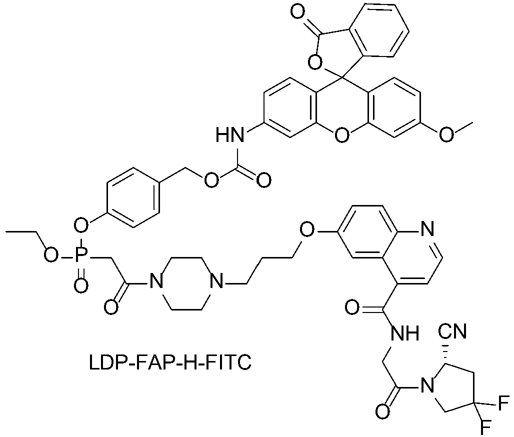

LDP-FAP-H-FITC was prepared according to the following reaction scheme.

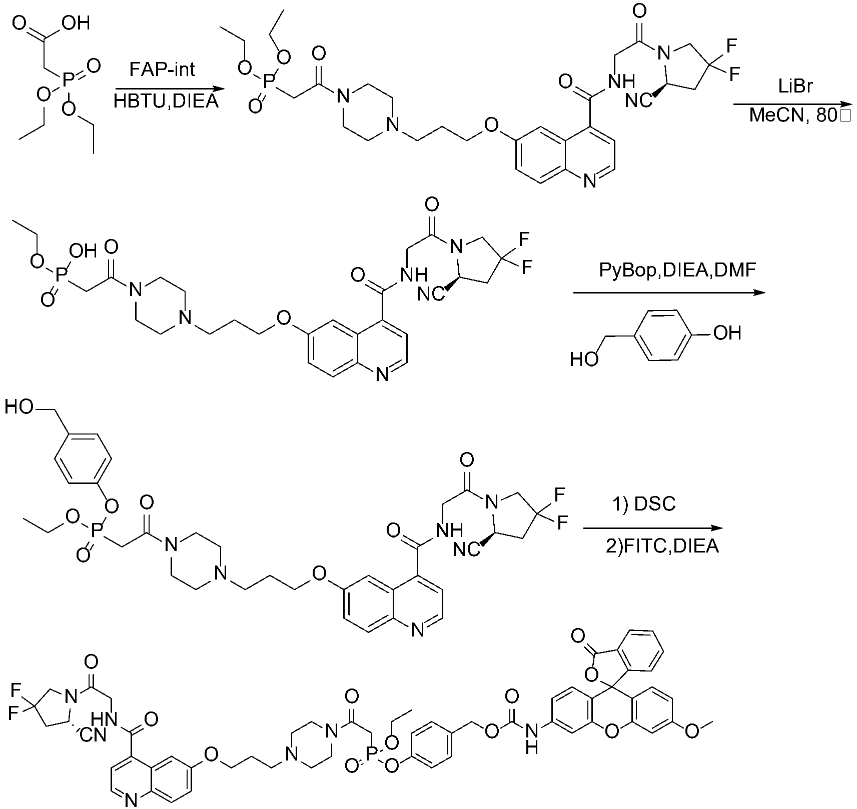

### Synthetic steps:

Diethyl phosphonoacetic acid (50 mg, 1 equivalent) and FAP int (124 mg, 1 equivalent) were dissolved in DMF, and DIEA (50 µL) and HBTU (116 mg, 1.2 equivalents) were added. The reaction was conducted at room temperature for 30 min, followed by purification by reverse-phase HPLC to give 120 mg of product in a yield of 74%.

The product from the preceding step was dissolved in MeCN, and LiBr (200 mg, greater than 10 equivalents) was added. The reaction was conducted overnight at 80°C, followed by purification by reverse-phase HPLC to give 93 mg of product in a yield of 80%.

The product from the preceding step and p-hydroxybenzyl alcohol were dissolved in DMF, and PyBop (1.2 equivalents) and DIEA (3 equivalents) were added. The reaction was conducted at room temperature for 30 min, followed by purification by reverse-phase HPLC to give the product in a yield of 65%.

The product from the preceding step was preactivated with DSC (1.2 equivalents) for 1 h, after which amino-FITC and DIEA were added, and the mixture was heated at 60°C. The product was obtained in a yield of 20% and purified by HPLC. The final product was characterized by UPLC-MS: [M+H]⁺ = 1114.35, purity 98%, t_{R} = 2.54 min.

### LDP-B-FAP-H-MMAE

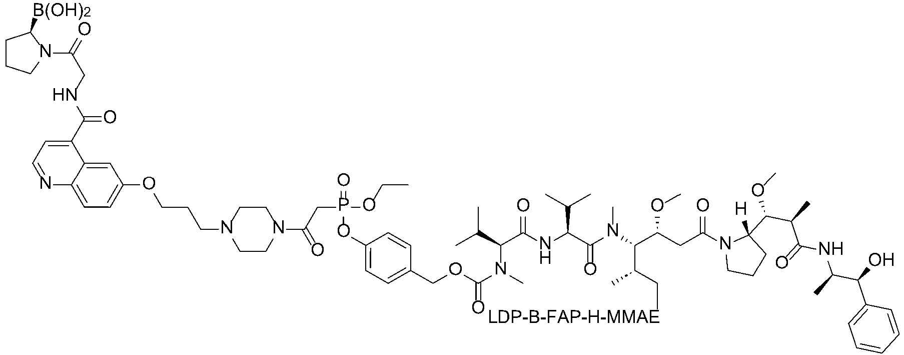

### Synthesis and characterization:

The synthetic route was the same as that for LDP-FAP-H-FITC, except that FITC in the synthetic route was replaced with MMAE. The final product was characterized by UPLC-MS: [M+H]⁺ = 1469.78, t_{R} = 2.43 min.

### LDP-B-FAP-F-MMAE

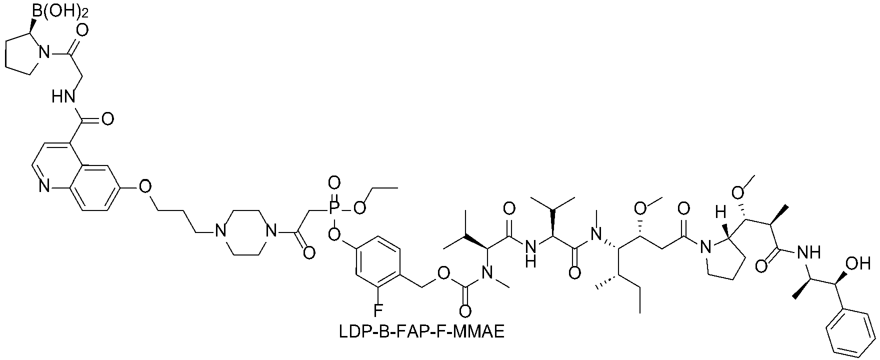

### Synthesis and characterization:

The synthetic route was the same as that for LDP-FAP-H-FITC, except that FITC in the synthetic route was replaced with MMAE and p-hydroxybenzyl alcohol was replaced with 3-fluoro-p-hydroxybenzyl alcohol. The final product was characterized by UPLC-MS: [M+H]⁺ = 1487.77, t_{R} = 2.43 min.

### LDP-B-FAP-F-NIR-DOTA

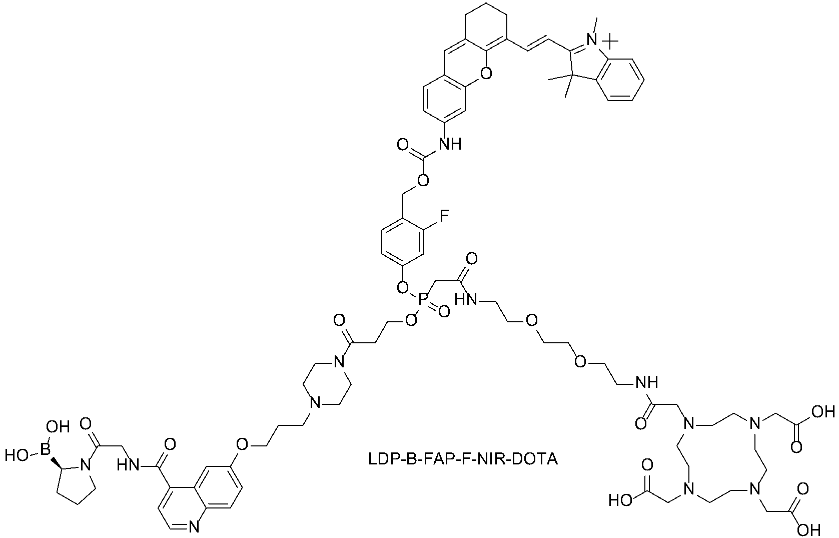

### Synthesis and characterization:

The synthetic route was the same as that for LDP-FAP-MMAE, except that MMAE was replaced with NIR, N-Fmoc-ethylenediamine was replaced with N-Fmoc-PEG2-ethylenediamine, and p-hydroxybenzyl alcohol was replaced with 3-F-p-hydroxybenzyl alcohol. The final product was characterized by UPLC-MS: [M]⁺ = 1712.76, t_{R} = 2.12 min.

### LDP-B-FAP-H-MMAE-DOTA

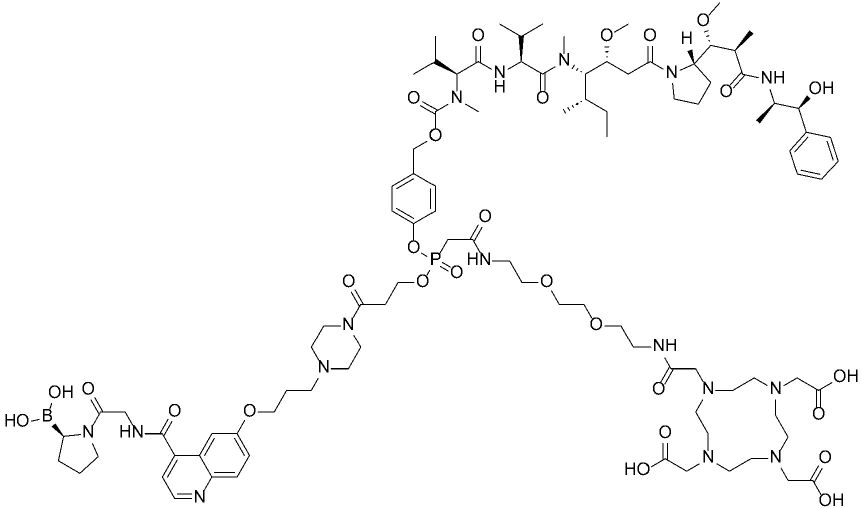

### Synthesis and characterization:

The synthetic route was the same as that for LDP-FAP-MMAE, except that NFmoc-ethylenediamine was replaced with NFmoc-PEG2-ethylenediamine. The final product was characterized by UPLC-MS: [M+H]⁺ = 2030.06, t_{R} = 2.03 min.

### LDP-B-FAP-F-MMAE-DOTA

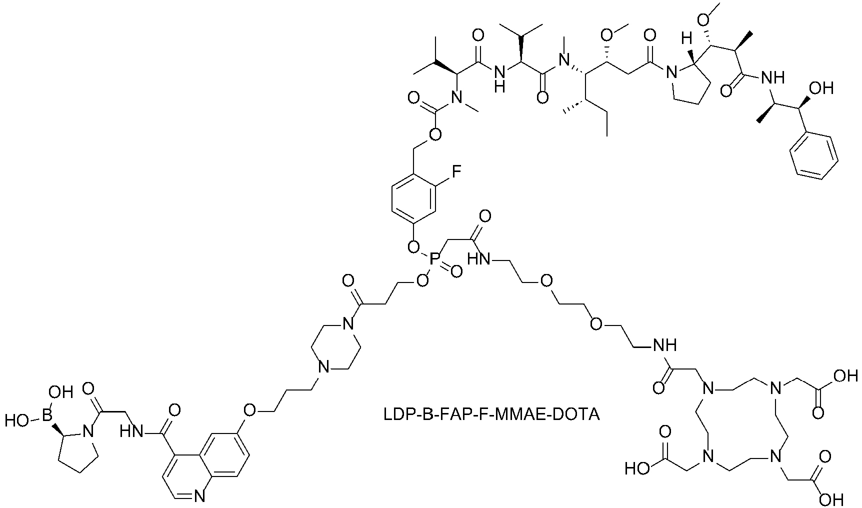

### Synthesis and characterization:

The synthetic route was the same as that for LDP-FAP-MMAE, except that NFmoc-ethylenediamine was replaced with NFmoc-PEG2-ethylenediamine and p-hydroxybenzyl alcohol was replaced with 3-F-p-hydroxybenzyl alcohol. The final product was characterized by UPLC-MS: [M+H]⁺ = 2048.05, t_{R} = 2.04 min.

### Biological test data

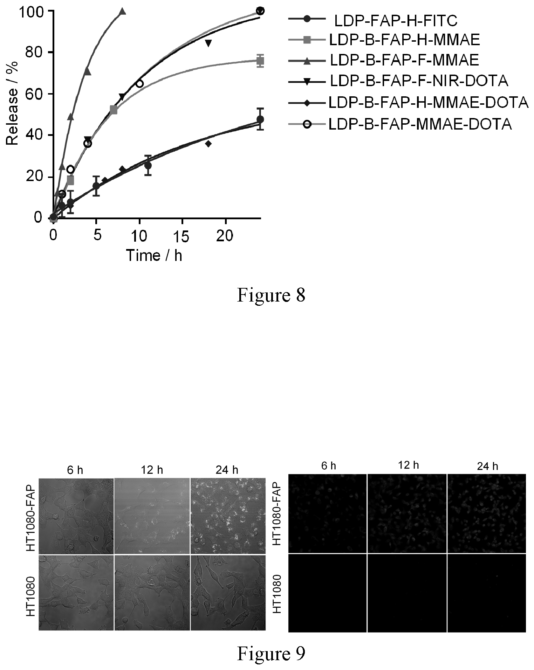

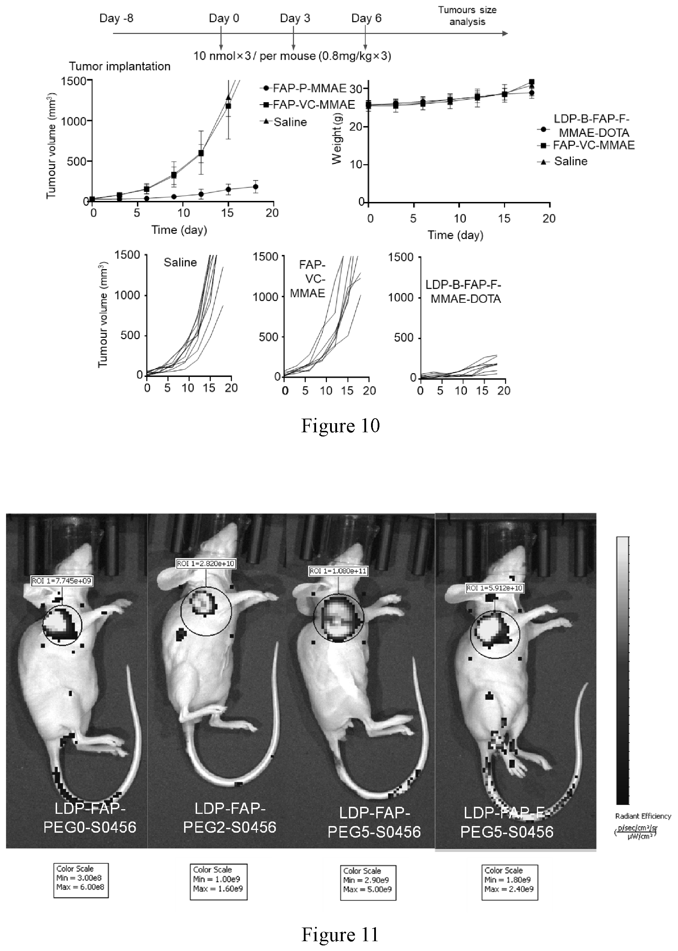

(1) FAP protein (5 µM) and each of the foregoing molecules (250 nM) were co-incubated in PBS at pH 7.4 and 37°C, and MMAE release was measured by UPLC-MS (SIR M/Z = 719) at different times. The test data showed that all of the foregoing molecules could release a functional molecule in the presence of FAP protein. The test results are summarized in FIG. 8.
(2) LDP-FAP-H-FITC and LDP-B-FAP-F-NIR-DOTA, each at 1 µM, were respectively co-incubated with HT1080 cells and HT080-FAP-expressing cells for 6, 12, and 24 h, and fluorescence confocal microscopy imaging was performed at the corresponding time points. The AF488 fluorescence channel was used for LDP-FAP-H-FITC, and the Cy5 fluorescence channel was used for LDP-B-FAP-F-NIR. Fluorescence signal generation was observed in FAP-expressing cells, whereas almost no fluorescence signal was observed in FAP-negative cells. The test results are summarized in FIG. 9, in which the left side shows green fluorescence and the right side shows red fluorescence.
(3) Mouse treatment test with LDP-B-FAP-F-MMAE-DOTA. A PDX tumor model was established in BNDG mice. When the tumor reached 50 mm³, each mouse was administered a single dose of 10 nmol, for a total of three doses at intervals of 3 days. A marked antitumor effect was observed without a marked decrease in body weight in the treatment group. The control groups were FAP-Val-Cit-PAB-MMAE and normal saline. The dosing regimen, changes in tumor volume, changes in body weight, and tumor-change curves for each mouse are summarized in FIG. 10.

### Example 4: LDP-FAP-PEG0-S0456, LDP-FAP-PEG2-S0456, LDP-FAP-PEG5-S0456, LDP-FAP-F-PEG5-S0456

### LDP-FAP-PEG0-S0456

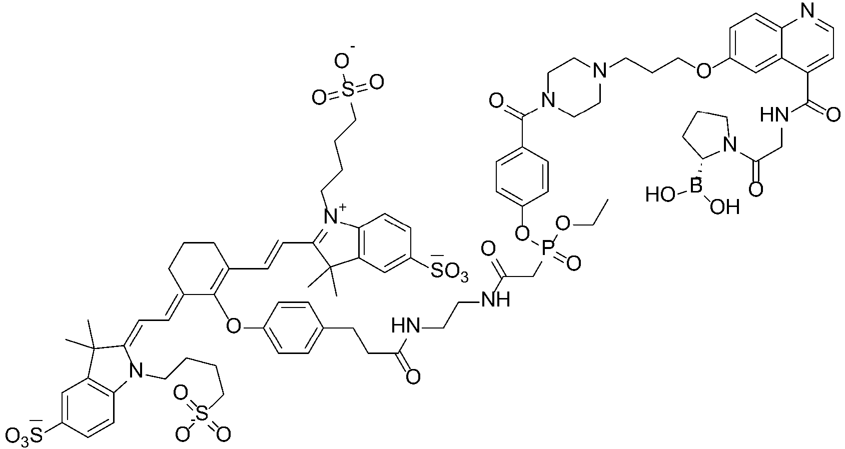

### Synthesis and characterization:

The synthetic route was the same as that for LDP-FAPI-DOTA, except that DOTA was replaced with S0456. For all example molecules containing an S0456 fluorescent molecule module, H₂O (0.1%) used in HPLC and UPLC-MS for synthetic separation and analytical characterization was replaced with H₂O (10 mM ammonium acetate). The final product was characterized by UPLC-MS: M⁺ = 1780.59, t_{R} = 1.66 min.

### LDP-FAP-PEG2-S0456

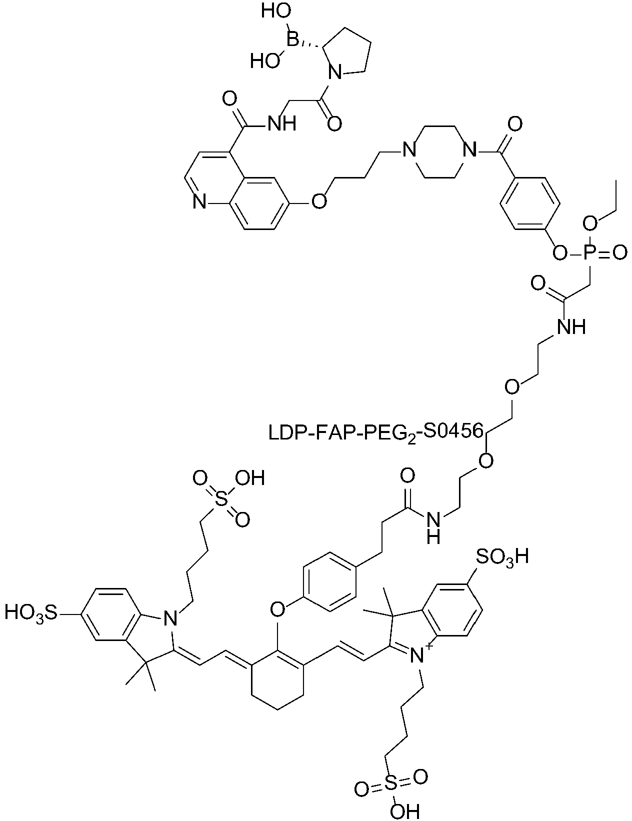

### Synthesis and characterization:

The synthetic route was the same as that for LDP-FAPI-DOTA, except that NFmoc-ethylenediamine was replaced with NFmoc-PEG2-ethylenediamine and DOTA was replaced with S0456. The final product was characterized by UPLC-MS: [M]⁺ = 1868.64, t_{R} = 1.65 min.

### LDP-FAP-PEG5-S0456

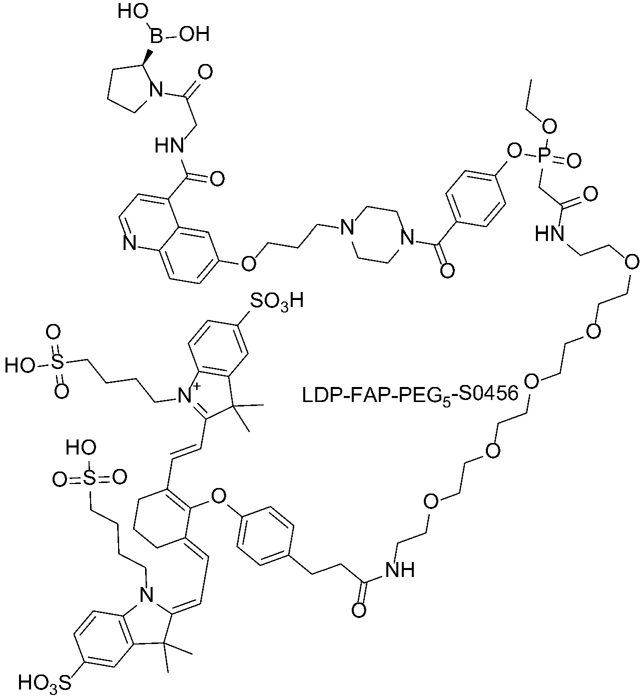

### Synthesis and characterization:

The synthetic route was the same as that for LDP-FAPI-DOTA, except that NFmoc-ethylenediamine was replaced with NFmoc-PEG5-ethylenediamine and DOTA was replaced with S0456. The final product was characterized by UPLC-MS: [M]⁺ = 2000.72, t_{R} = 1.66 min.

### LDP-FAP-F-PEG5-S0456

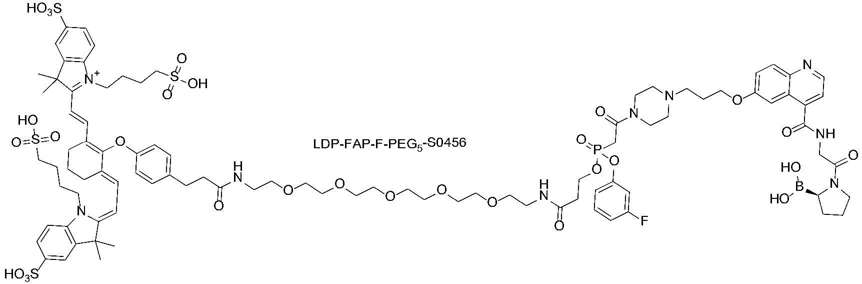

### Synthesis and characterization:

The synthetic route was the same as that for LDP-FAP-MMAE, except that DOTA was replaced with S0456, NFmoc-PEG2-ethylenediamine was replaced with NFmoc-PEG5-ethylenediamine, and p-hydroxybenzyl alcohol was replaced with 3-fluorophenol. The final product was characterized by UPLC-MS: [M]⁺ = 2018.71, t_{R} = 1.67 min.

### Biological test data:

The foregoing molecules were subjected to in vivo near-infrared fluorescence imaging in HT1080-FAP tumor-bearing mice. Each mouse was intravenously injected via the tail vein with 10 nmol of a molecule (four mice for each molecule, n = 4), and imaging was performed 6 h after administration. The imaging parameters were Ex = 720 nm and Em = 790 nm. The test results are summarized in FIG. 11.

### Example 5: LDP-FAP-S0456-DOTA

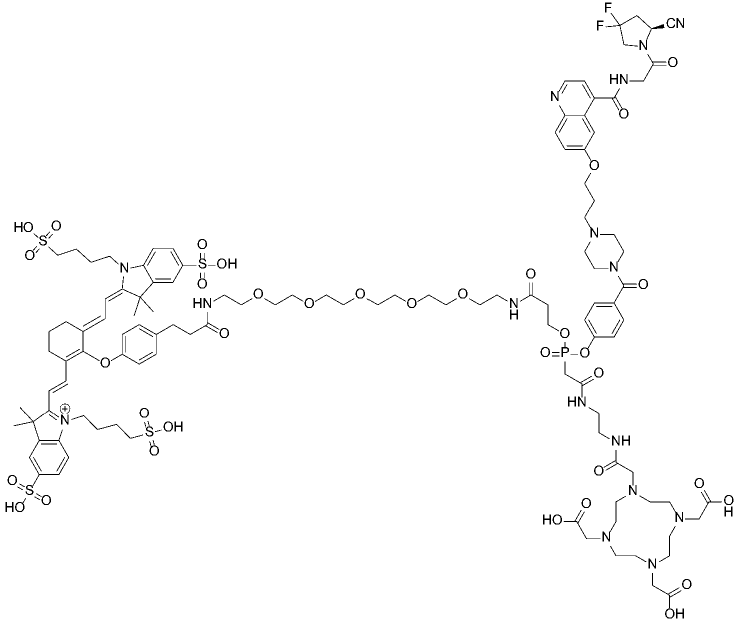

### Synthesis and characterization:

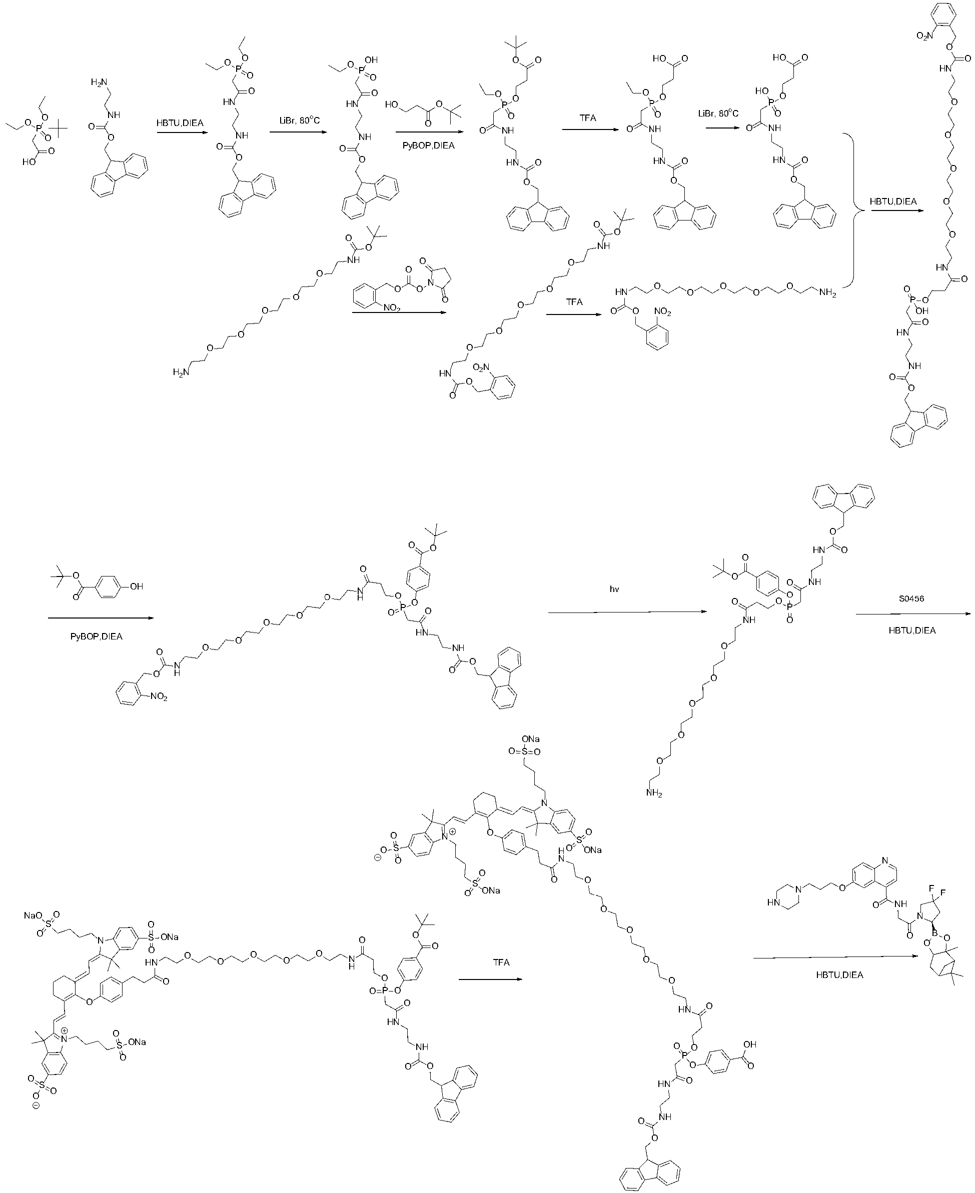

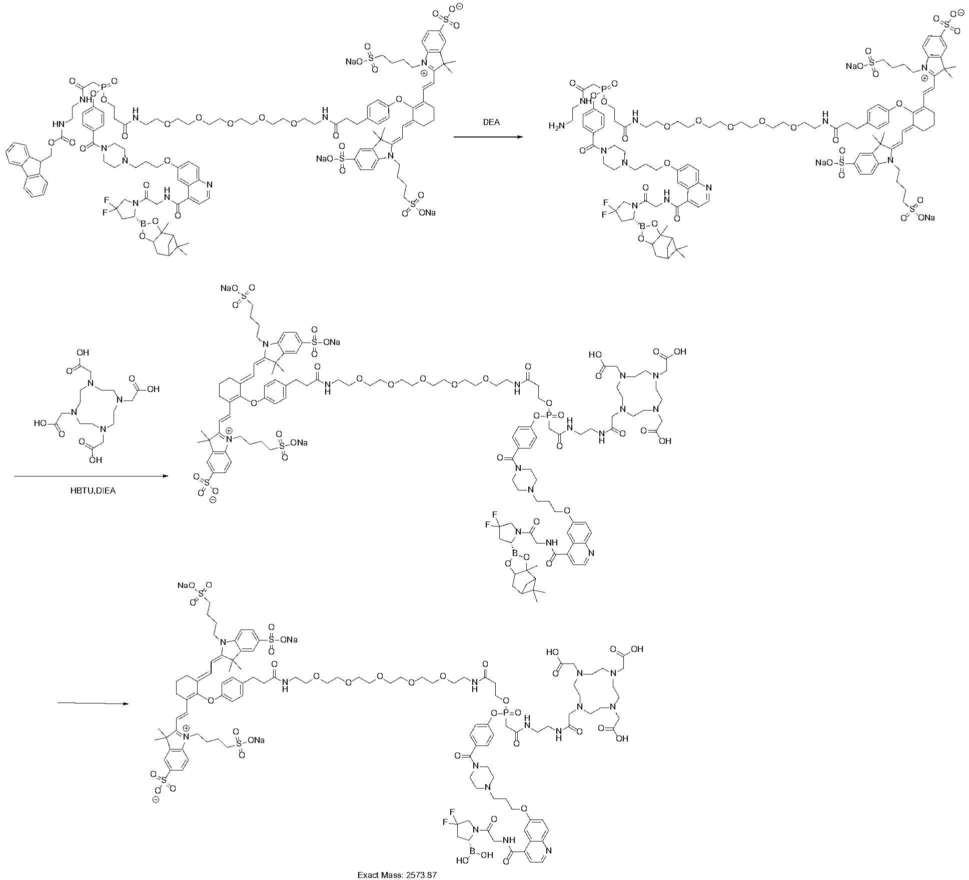

Diethyl phosphonoacetic acid (5 g, 1 equivalent) and N-Fmoc-ethylenediamine (8.6 g, 1.2 equivalents) were dissolved in DMF, and DIEA (4.7 mL) and HBTU (14.5 mg, 1.5 equivalents) were added. The reaction was conducted at room temperature for 30 min, followed by purification on a reverse-phase column to give 8 g of product in a yield of 69%.

The product from the preceding step was dissolved in MeCN, and LiBr (9 g, excess) was added. The reaction was conducted overnight at 80°C, followed by purification on a reverse-phase column to give 6 g of product in a yield of 80%.

The product from the preceding step and tert-butyl 3-hydroxypropionate (1.2 equivalents) were dissolved in MeCN, and PyBop (1.5 equivalents) and DIEA (2 equivalents) were added. The reaction was conducted at room temperature for 30 min, followed by purification on a reverse-phase column to give 5 g of product in a yield of 65%.

The product from the preceding step was dissolved in MeCN, and TFA was added to a concentration of 50%. The reaction was conducted at room temperature for 20 min, followed by purification on a reverse-phase column to give 4.28 g of product in a yield of 95%.

The product from the preceding step was dissolved in MeCN, and LiBr (9 g, excess) was added. The reaction was conducted overnight at 80°C, followed by purification on a reverse-phase column to give 3.64 g of product in a yield of 90%. This product was designated P1.

The compound shown below (5 g, 1 equivalent) and the compound shown below (4.2 g, 1.1 equivalents) were dissolved in MeCN, and a small amount of DIEA was added. The reaction was conducted at room temperature for 40 min to give 6.61 g of product in a yield of 90%.

The product from the preceding step was added to a solution of TFA in MeCN, and the reaction was conducted at room temperature for 20 min to give 5.15 g of product in a yield of 95%. This product was designated P2.

P1 and P2 were mixed and dissolved in MeCN, and DIEA (2.8 mL) and HBTU (4.4 g, 1.5 equivalents) were added. The reaction was conducted at room temperature for 50 min, followed by purification on a reverse-phase column to give 5.0 g of product in a yield of 72%.

The product from the preceding step and tert-butyl p-hydroxybenzoate (1.2 equivalents) were mixed and dissolved in MeCN, and DIEA (2 mL) and HBTU (3.0 g, 1.5 equivalents) were added. The reaction was conducted at room temperature for 50 min, followed by purification on a reverse-phase column to give 3.87 g of product in a yield of 65%.

The product from the preceding step was dissolved in MeCN and irradiated overnight with a 365 nm ultraviolet lamp to give 1.91 g of product in a yield of 59%.

A portion (100 mg) of the product from the preceding step and S0456 (1 equivalent) were dissolved in MeCN, and DIEA (50 microliters) and HBTU (1.5 equivalents) were added. The reaction was conducted at room temperature for 50 min, followed by purification on a reverse-phase column to give 167 mg of product in a yield of 80%.

The product from the preceding step was added to a solution of TFA in MeCN, and the reaction was conducted at room temperature for 20 min to give 146 mg of product in a yield of 90%.

The product from the preceding step and FAP int (50 mg, 1 equivalent) were dissolved in DMF, and DIEA (30 µL) and HBTU (57.5 mg, 2 equivalents) were added. The reaction was conducted at room temperature for 30 min, followed by purification by reverse-phase HPLC to give 150 mg of product in a yield of 78%.

The product from the preceding step was added to a solution of TEA in MeCN, and the reaction was conducted at room temperature for 20 min to give 123 mg of product in a yield of 90%.

DOTA (excess) was dissolved in DMF, and DIEA (30 µL) and HBTU (40 mg, 2 equivalents) were added. The reaction was conducted at room temperature for 30 min, after which the product from the preceding step was added. The reaction was conducted at room temperature for 30 min, followed by purification by reverse-phase HPLC to give 70 mg of product in a yield of 50%.

The product from the preceding step was added to 4 M HCl, and the reaction was conducted at room temperature for 20 min to give 40 mg of the final product in a yield of 60%. The final product was characterized by UPLC-MS: purity greater than 95%, [M+H]²⁺ = 1253.46, t_{R} = 1.68 min.

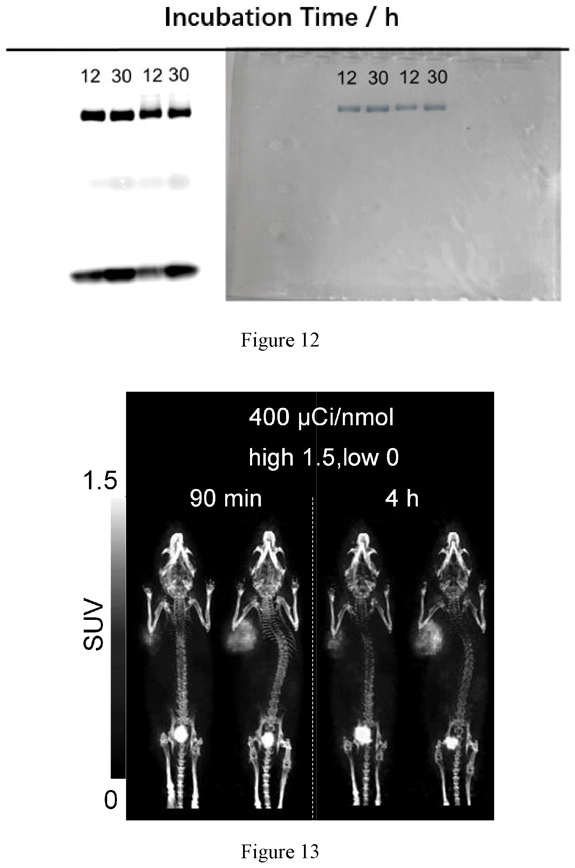

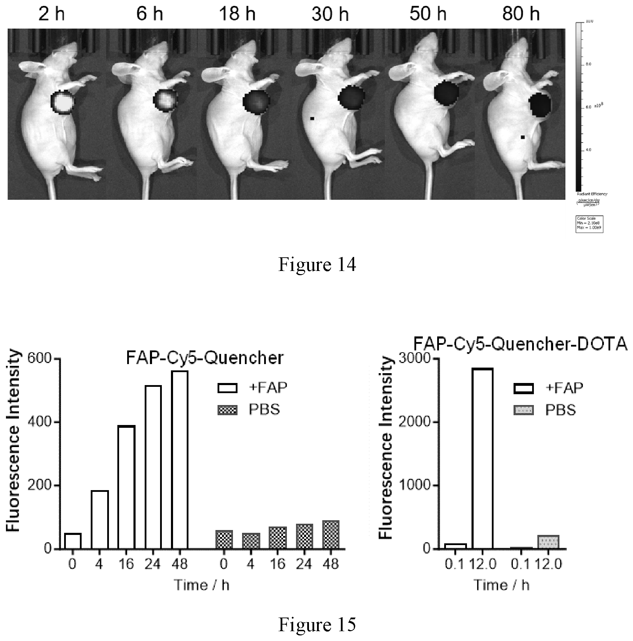

Biological test data:
(1) The foregoing molecule was subjected to a covalent SDS-PAGE experiment. FAP protein and the ¹⁷⁷Lu-labeled molecule were co-incubated at a ratio of 5:1 at 37°C and pH 7.4. Samples were taken at 12 and 30 h (n = 2) for SDS-PAGE experiments. Autoradiography revealed clear radioactivity at the protein band, indicating that covalent attachment to FAP protein was achieved. The left panel of FIG. 12 shows the autoradiography result. The upper band corresponds to the Coomassie brilliant blue-stained protein band in the right panel of FIG. 12, and the lower band represents the non-covalently bound small molecule.
(2) The foregoing molecule was radiolabeled with ⁶⁸Ga. 10 nmol of the molecule and 10 mCi of ⁶⁸GaCl₃ were combined in a sodium acetate buffer at pH = 4, 0.5 mg of gentisic acid was added, and the mixture was heated at 90°C for 10 min and then purified through a C18 cartridge. The labeled product was intravenously injected via the tail vein into HT1080-FAP tumor-bearing mice for PET-CT imaging. The molar activity of the molecule was 400 µCi/nmol. The test results are summarized in FIG. 13 and show that the molecule specifically accumulated at the tumor site.
(2) Near-infrared fluorescence imaging was performed in HT1080-FAP tumor-bearing mice. Each mouse was intravenously administered 1 nmol via the tail vein (n = 4), and imaging was performed at different time points. The imaging parameters were Ex = 720 nm and Em = 790 nm. The test results are summarized in FIG. 14. The molecule accumulated at the tumor site and exhibited high tumor retention.

### Example 6: FAP-Cy5-Quencher and FAP-Cy5-Quencher-DOTA

### FAP-Cy5-Quencher

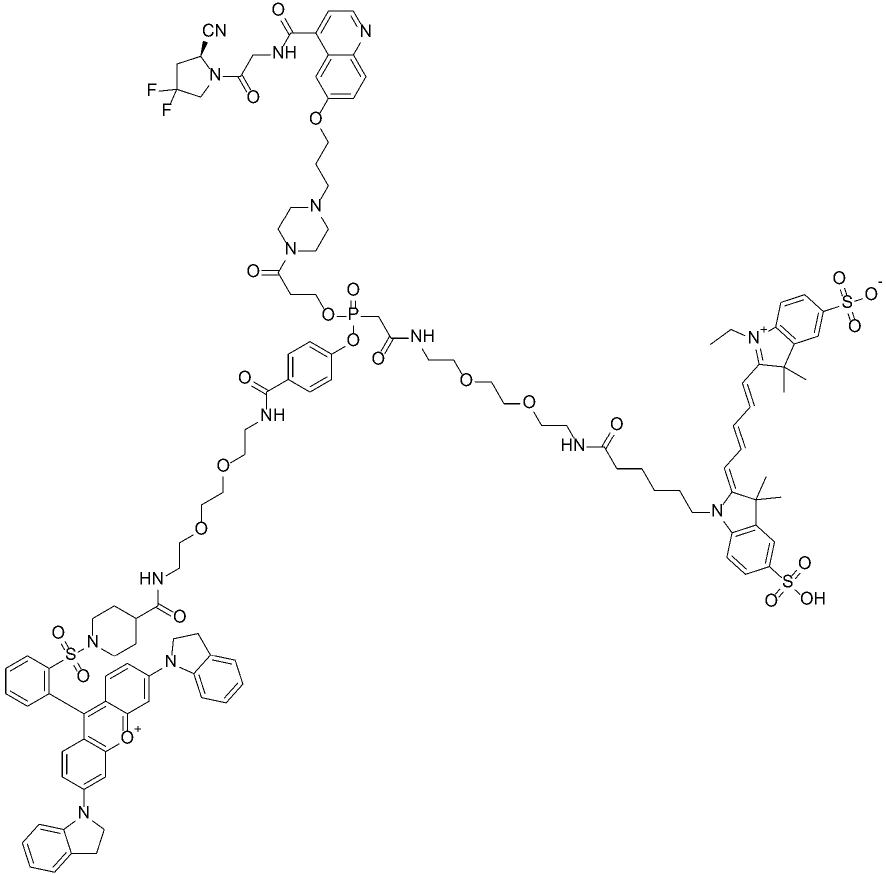

Synthesis and characterization: The synthetic route was the same as that for LDP-FAP-S0456-DOTA, except that the FAP-targeting head was replaced with NFmoc-PEG2-ethylenediamine-QSY-21, DOTA was replaced with NFmoc-PEG2-ethylenediamine-Cy5, and NFmoc-ethylenediamine was replaced with NFmoc-PEG2-ethylenediamine.

The final product was characterized by UPLC-MS: [M+H]²⁺ = 1182.45, t_{R} = 2.35 min.

### FAP-Cy5-Quencher-DOTA

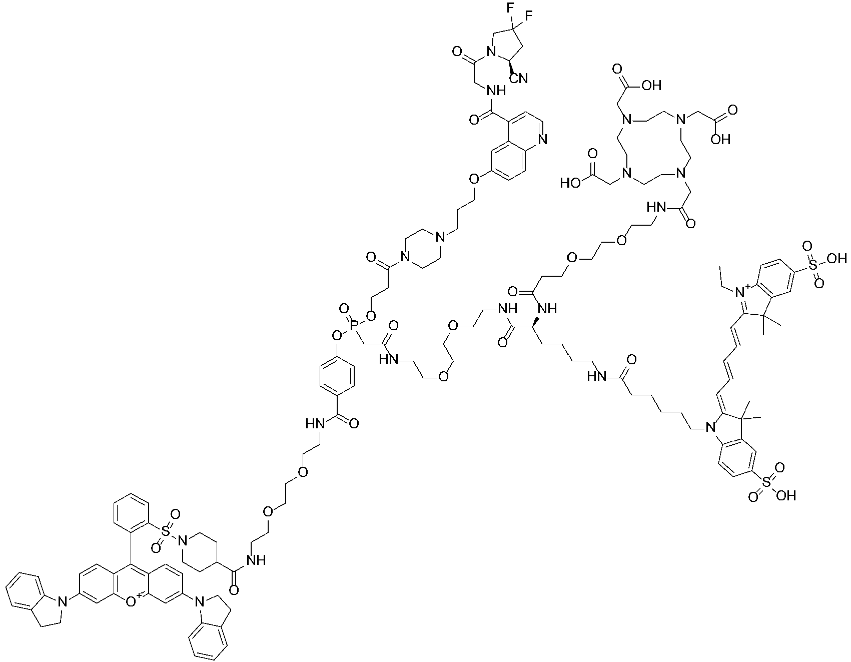

Synthesis and characterization: The synthetic route was the same as that for FAP-Cy5-Quencher, except that NFmoc-PEG2-ethylenediamine-Cy5 was replaced with Lys-DOTA-Cy5.

The final product was characterized by UPLC-MS: [M]²⁺ = 1518.64, t_{R} = 2.13 min.

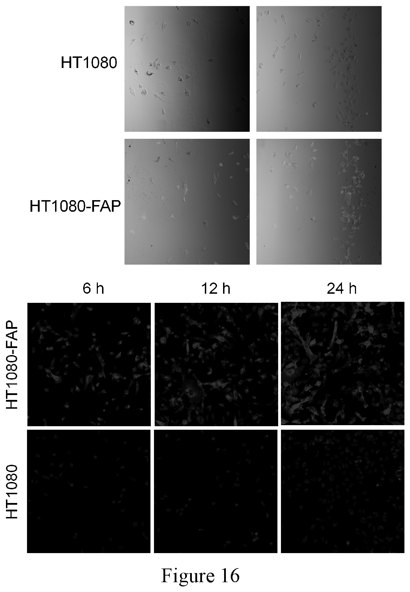

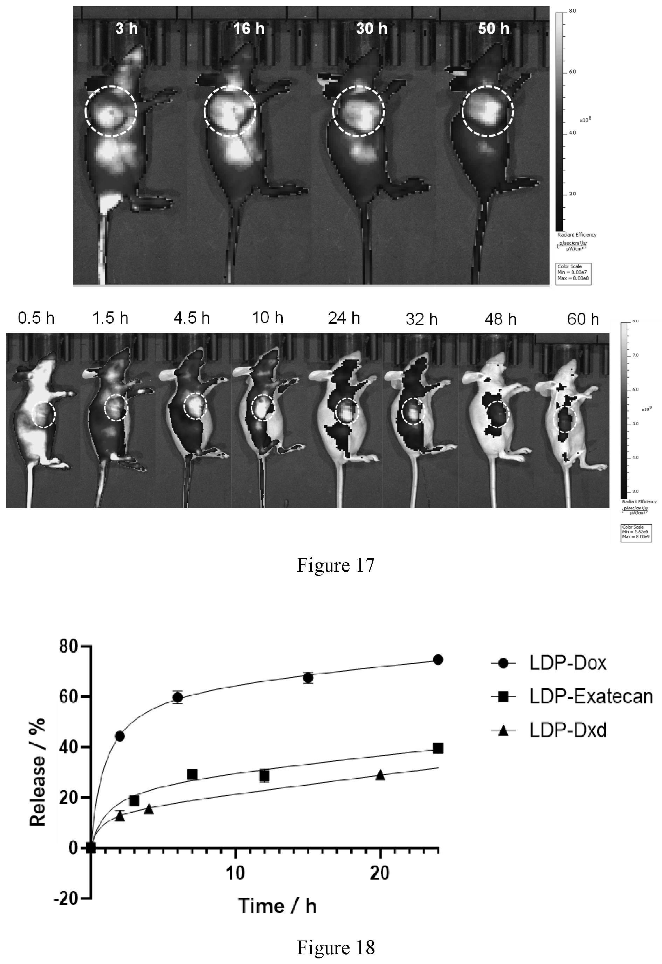

Biological test data:
(1) Each of the foregoing molecules (500 nmol) and protein (5 µM) were co-incubated in PBS at pH 7.4 and 37°C. Fluorescence intensity was measured at the corresponding time points using Ex = 620 nm and Em = 670 nm, with a PBS group without FAP protein serving as a control. The results showed that the fluorescence signal increased markedly after addition of FAP protein. FIG. 15 respectively shows the experimental results for FAP-Cy5-Quencher and FAP-Cy5-Quencher-DOTA.
(2) Each of the foregoing molecules (1 µM/1 µM) was respectively co-incubated with HT1080 cells and HT080-FAP-expressing cells, and fluorescence confocal microscopy imaging was performed at the corresponding time points using the Cy5 fluorescence channel. The test results are summarized in FIG. 16. FIG. 16 shows generation of a fluorescence signal in FAP-expressing cells, whereas almost no fluorescence signal was observed in FAP-negative cells. The lower panels respectively show the experimental results after co-incubation with FAP-Cy5-Quencher for 12 h and with FAP-Cy5-Quencher-DOTA for 6-24 h.
(3) Near-infrared fluorescence imaging was performed in HT1080-FAP tumor-bearing mice. Each mouse was intravenously administered 10 nmol via the tail vein and imaged. The molecules were found to specifically accumulate at the tumor site and exhibit prolonged tumor retention. The test results are summarized in FIG. 17. The upper and lower panels of FIG. 17 respectively show imaging results at 3-50 h after administration of FAP-Cy5-Quencher and at 0.5-60 h after administration of FAP-Cy5-Quencher-DOTA.

### Example 7: LDP-Dox, LDP-Exatecan, LDP-Dxd

### LDP-Dox

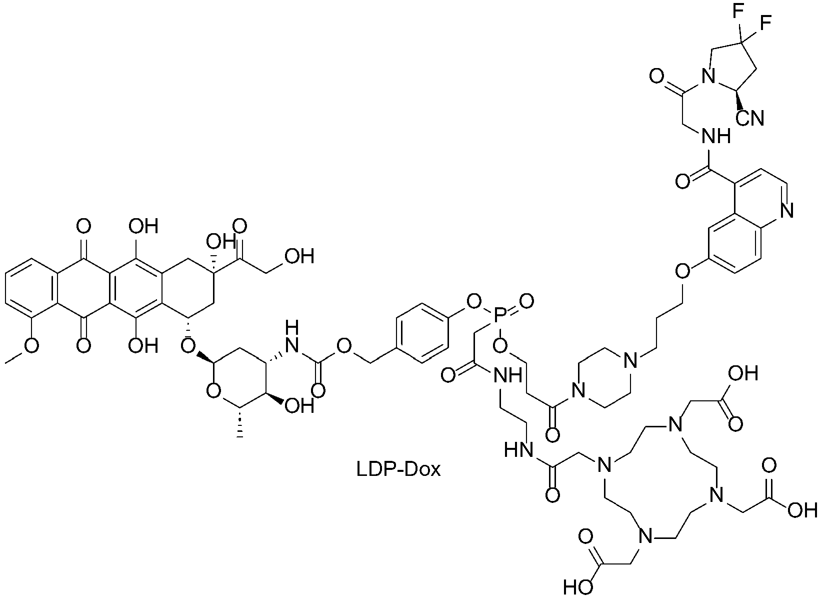

### Synthesis and characterization:

The synthetic route was the same as that for LDP-FAP-MMAE, except that MMAE was replaced with Dox. The final product was characterized by UPLC-MS: [M+H]⁺ = 1784.65, t_{R} = 2.23 min.

### LDP-Exatecan

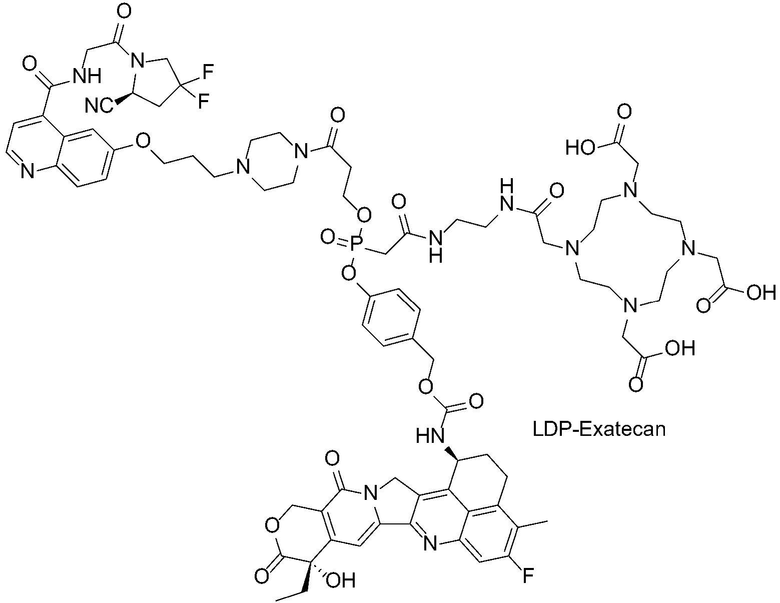

### Synthesis and characterization:

The synthetic route was the same as that for LDP-FAP-MMAE, except that MMAE was replaced with Exatecan. The final product was characterized by UPLC-MS: [M+H]⁺ = 1676.64, t_{R} = 2.12 min.

### LDP-Dxd

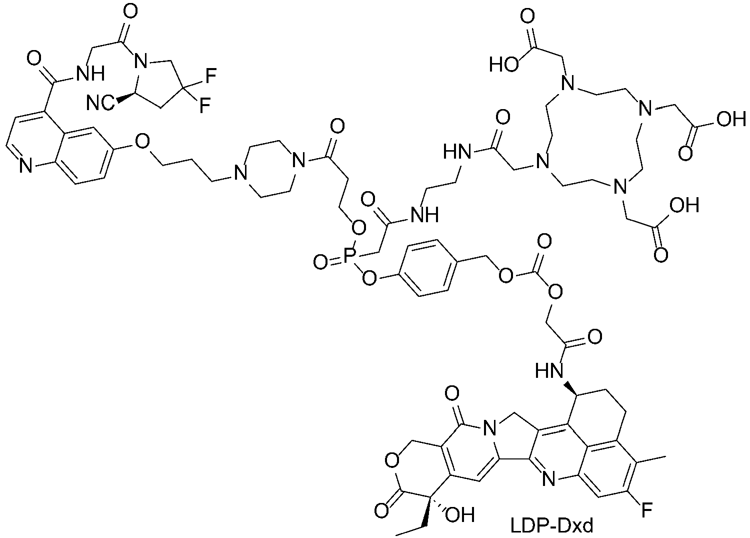

### Synthesis and characterization:

The synthetic route was the same as that for LDP-FAP-MMAE, except that MMAE was replaced with Dxd. The final product was characterized by UPLC-MS: [M+H]⁺ = 1734.64, t_{R} = 2.15 min.

Biological test data
(1) FAP protein (5 µM) and each of the foregoing molecules (250 nM) were co-incubated in PBS at pH 7.4 and 37°C, and release of the drug molecule was measured by UPLC-MS at different time points. The test results are summarized in FIG. 18.

### Example 8: CA-P-1, CA-P-2

### CA-P-1

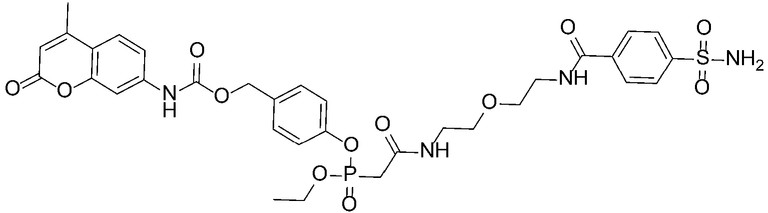

### Synthesis and characterization:

The synthetic route was the same as that for LDP-FAP-H-FITC, except that FITC was replaced with coumarin and the FAP-targeting head was replaced with a CA-targeting head (benzenesulfonamide). The final product was characterized by UPLC-MS: [M+H]⁺ = 745.19, t_{R} = 2.23 min.

### CA-P-2

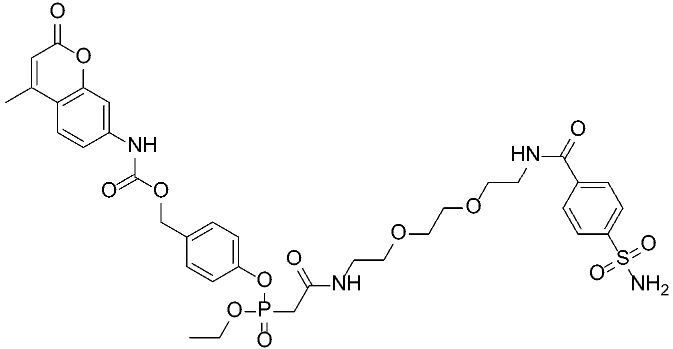

### Synthesis and characterization:

The synthetic route was the same as that for LDP-FAP-H-FITC, except that FITC was replaced with coumarin, the FAP-targeting head was replaced with a CA-targeting head (benzenesulfonamide), and NFmoc-ethylenediamine was replaced with NFmoc-PEG2-ethylenediamine. The final product was characterized by UPLC-MS: [M+H]⁺ = 789.21, t_{R} = 2.22 min.

### Biological test data:

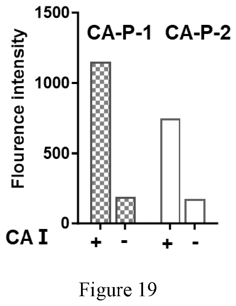

Carbonic anhydrase 1 protein (5 µM) and each of the foregoing molecules (250 nM) were co-incubated in PBS at pH 7.4 and 37°C. After incubation for 24 h, the increase in fluorescence signal was measured using a fluorometer, with a group without protein serving as a control. The test results are summarized in FIG. 19.

The foregoing descriptions are merely exemplary embodiments of the present invention and are not intended to limit the scope of protection of the present invention, which is defined by the appended claims.

## Claims

1. A compound of formula (I), or a pharmaceutically acceptable salt, stereoisomer or solvate thereof, 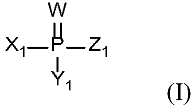 wherein
W is oxygen or sulfur, preferably oxygen;
X₁ is -L₁-R₁;
Y₁ is -L₁-R₂;
Z₁ is -L₂-R₃-L₁-R₄;
L₁, at each occurrence, is independently a bond or a linking group;
L₂ is O, S, or NH, preferably oxygen;
R₃ is an optionally substituted arylene, heteroarylene, cycloalkylene or heterocycloalkylene group; the substituent is selected from F, Br, Cl, methoxy, trifluoromethyl, and cyano;
one of R₁, R₂ and R₄ is a targeting group, and the remaining two are independently a targeting group, a drug molecule group, an isotope chelator or labeling precursor group, a fluorescent or molecular labeling group, or a capping group.

2. The compound of formula (I) according to claim 1, or a pharmaceutically acceptable salt, stereoisomer or solvate thereof, wherein R₃ is selected from
optionally substituted C₅₋₁₄ arylene;
optionally substituted C₅₋₁₄ heteroarylene containing 1 to 3 heteroatoms independently selected from N, O, or S;
optionally substituted divalent saturated or partially unsaturated fused, bridged or spiro C₅₋₁₄ cycloalkylene containing a single ring or multiple rings; or
optionally substituted divalent saturated or partially unsaturated fused, bridged or spiro C₅₋₁₄ heterocycloalkylene containing a single ring or multiple rings, which contains 1 to 3 heteroatoms independently selected from N, O, or S;
the substituent is selected from F, Br, Cl, methoxy, trifluoromethyl, and cyano.

3. The compound of formula (I) according to claim 1, or a pharmaceutically acceptable salt, stereoisomer or solvate thereof, wherein R₃ is selected from optionally substituted 1,2-phenylene, 1,3-phenylene, 1,4-phenylene, 1,2-naphthylene, 1,5-naphthylene, 1,6-naphthylene, 2,6-naphthylene, furanylene, thienylene, pyrrolylene, imidazolylene, pyrazolylene, triazolylene, pyridylene, pyrazinylene, pyrimidinylene, pyridazinylene, cyclopentylene, cyclohexylene, tetrahydrofuranylene, tetrahydropyrrolylene, tetrahydrothienylene, tetrahydropyranylene, piperidinylene, tetrahydrothiopyranylene, dioxanylene, piperazinylene, 1,4-piperazinylene, pyrazinylene, morpholinylene, and dioxanylene; the substituent is selected from F, Br, Cl, methoxy, trifluoromethyl, and cyano; or
R₃ is preferably 3-X-1,4-phenylene, 2-X-1,4-phenylene, 2-X-1,5-phenylene, 3-X-1,5-phenylene, 3-X-1-phenylene, 2-X-1-phenylene, or 4-X-1-phenylene, X is selected from F, Br, Cl, methoxy, trifluoromethyl, and cyano.

4. The compound of formula (I) according to claim 1, or a pharmaceutically acceptable salt, stereoisomer or solvate thereof, wherein
R₄ is a targeting group or a drug molecule group, R₁ and R₂ are each independently a targeting group, an isotope chelator or labeling precursor group, a fluorescent or molecular labeling group, or a capping group.

5. The compound of formula (I) according to claim 1, or a pharmaceutically acceptable salt, stereoisomer or solvate thereof, wherein
R₄ is a targeting group, R₁ and R₂ are each independently a drug molecule group, an isotope chelator or labeling precursor group, a fluorescent or molecular labeling group, or a capping group.

6. The compound of formula (I) according to claim 1, or a pharmaceutically acceptable salt, stereoisomer or solvate thereof, wherein
R₄ is a targeting group, one of R₁ and R₂ is a drug molecule group, an isotope chelator or labeling precursor group, or a fluorescent or molecular labeling group, and the other is a capping group.

7. The compound of formula (I) according to any one of claims 1-6, or a pharmaceutically acceptable salt, stereoisomer or solvate thereof, satisfying one or more of the following conditions:
(a) L₁, at each occurrence, is independently a bond, or -Lₐ-(L_{b})ₘ-L_{c}-(L_{d})ₙ-Lₑ-L_{f}-, wherein Lₐ, L_{b}, L_{c}, L_{d}, Lₑ, L_{f}, at each occurrence, are each independently selected from a bond, -O-, -S-, -SS-, -S(=O)₂-, -NH-, -N(CH₃)-, -C(R^{a})(R^{b})-, -C(=O)-, -C(=O)O-, -OC(=O)-, -C(=O)NH-, - NHC(=O)-, -C(=O)-N(CH₃)-, -N(CH₃)-C(=O)-, -CH₂CH₂-, -CH₂CH₂CH₂-, -CH₂CH₂CH₂CH₂-, -CH₂NH-, -NHCH₂-, -CH₂O-, -OCH₂-, -CH=N-O-, -C(CH₃)=N-NH-, -C(CH₃)=N-NH-C(=O)-, -NH-CH₂-C(=CH-COOH)-, a divalent peptide group containing 1, 2, 3 or 4 amino acids, cyclohexylene, phenylene, heterocycloalkylene (for example, piperazine-1,4-diyl), or 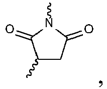 R^{a} and R^{b}, at each occurrence, are each independently selected from H, methyl, ethyl, propyl, isopropyl, phenyl, -OH, -SH, -NH₂, -NH(CH₃), or -NH(CH₃)₂, the subscripts m and n are each independently 0, 1, 2, 3, or 4;
(b) the capping group is selected from -L_{g}-(Lₕ)ₙ-Lᵢ-R^{c}, wherein L_{g}, Lₕ, and Lᵢ, at each occurrence, are each independently selected from a bond, -O-, -S-, -C(R^{a})(R^{b})-, -NH-, -N(CH₃)-, -C(=O)-, -CH₂O-, or -OCH₂-, the subscript n is an integer of 0-4, R^{a} and R^{b}, at each occurrence, are independently selected from H, methyl, ethyl, propyl, isopropyl, phenyl, -OH, -SH, -NH₂, - NH(CH₃), or -NH(CH₃)₂, R^{c} is selected from methyl, ethyl, hydroxyl, or thiol;
(c) the drug molecule group is derived from a cytotoxic drug, an immunomodulator, an antibiotic, a protein degrader or a molecular glue;
(d) the isotope chelator or labeling precursor group is selected from 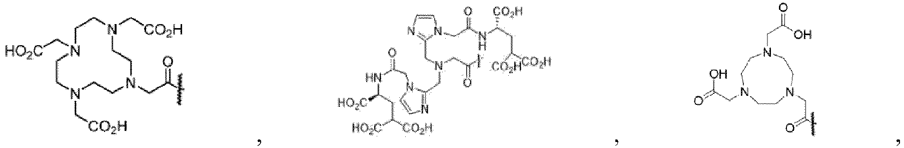 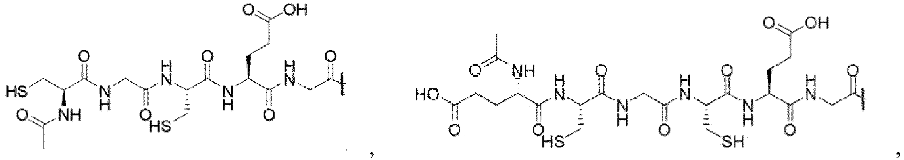 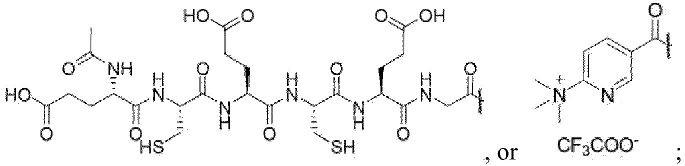
(e) the fluorescent or molecular labeling group is selected from a fluorescent labeling molecule group, an affinity purification labeling group, or a click-chemistry labeling group;
(f) the targeting group is selected from ligand groups having high affinity for FAP, FOLR1, integrin, carbonic anhydrase, or Nectin4.

8. The compound of formula (I) according to any one of claims 1-7, or a pharmaceutically acceptable salt, stereoisomer or solvate thereof, wherein L₁ is selected from -NH-, -CH₂-, - N(CH₃)-, -C(=O)-, 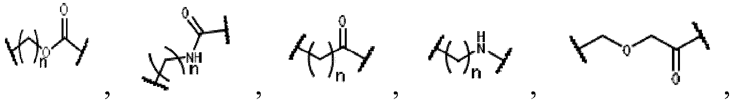 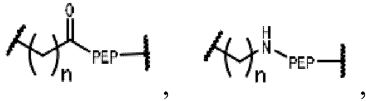 -C(=O)-PEP-, -NH-PEP-, 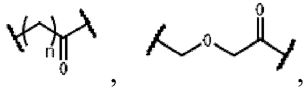 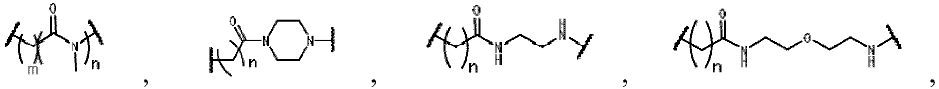 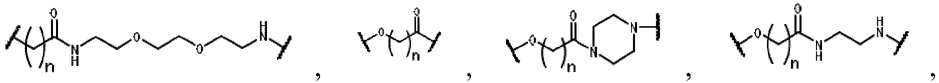 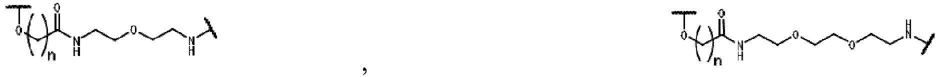 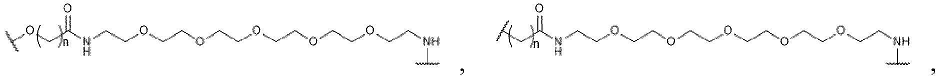 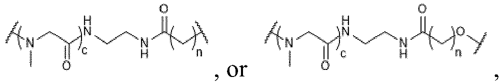 wherein the subscripts m and n are each 0, 1, 2, 3 or 4, PEP represents a divalent peptide group containing 1, 2, 3 or 4 amino acids, and the subscript c is 1, 2, 3, 4, 5, 6, 7, 8, 9 or 10.

9. The compound of formula (I) according to any one of claims 1-8, or a pharmaceutically acceptable salt, stereoisomer or solvate thereof, wherein the capping group is selected from methyl, methoxy, 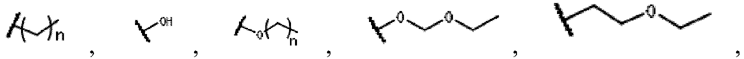 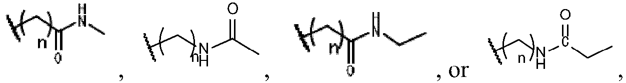 the subscript n is 1, 2, 3 or 4.

10. The compound of formula (I) according to any one of claims 1-9, or a pharmaceutically acceptable salt, stereoisomer or solvate thereof, wherein L₁ in Z₁ is selected from -NH-, -CH₂-, -N(CH₃)-, -C(=O)-, 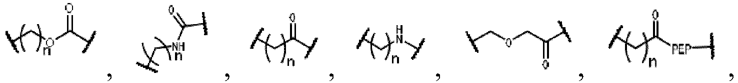 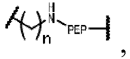 -C(=O)-PEP-, -NH-PEP-, wherein the subscript n is an integer of 1-4, and PEP represents a divalent peptide group containing 1, 2, 3 or 4 amino acids.

11. The compound of formula (I) according to any one of claims 1-10, or a pharmaceutically acceptable salt, stereoisomer or solvate thereof, wherein L₁ in X₁ or Y₁ is selected from 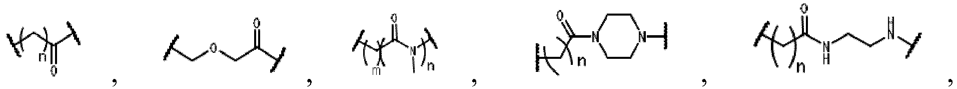 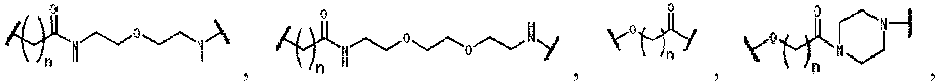 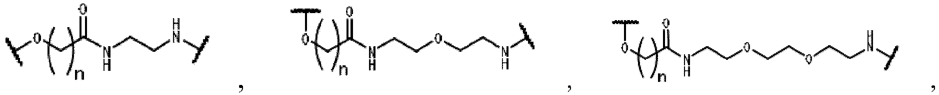 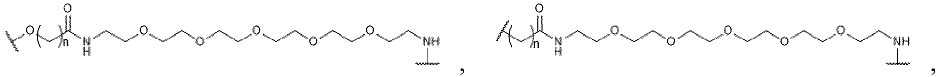 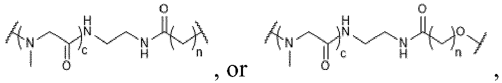 wherein the subscripts m and n are each 0, 1, 2, 3 or 4, the subscript c is 1, 2, 3, 4, 5, 6, 7, 8, 9 or 10.

12. The compound of formula (I) according to any one of claims 1-11, or a pharmaceutically acceptable salt, stereoisomer or solvate thereof, wherein the drug molecule group is selected from:

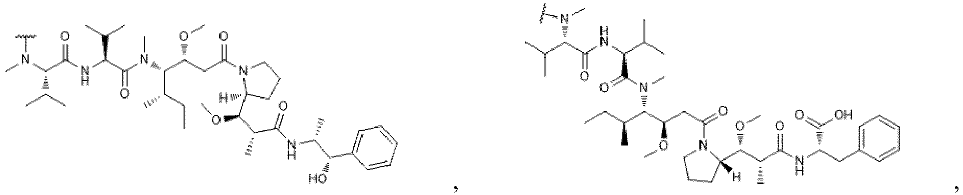

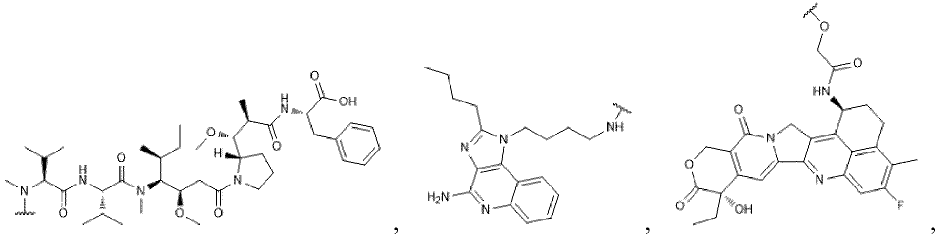

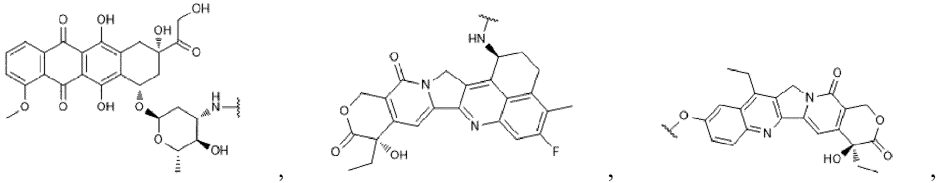

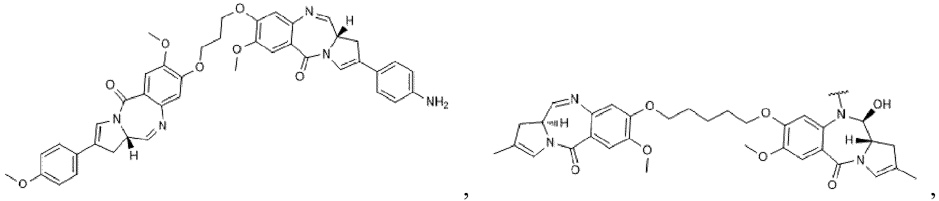

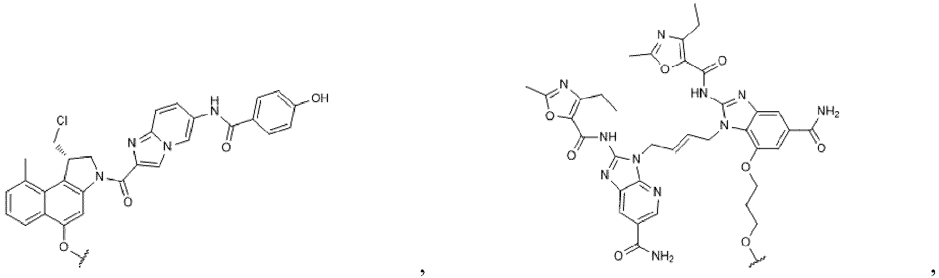

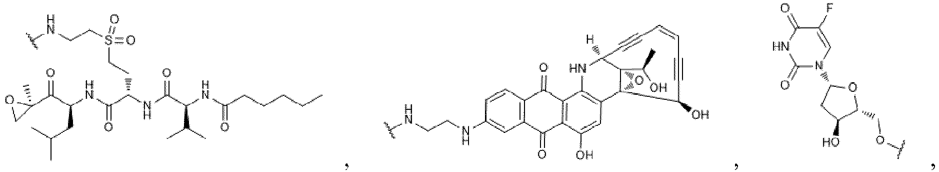

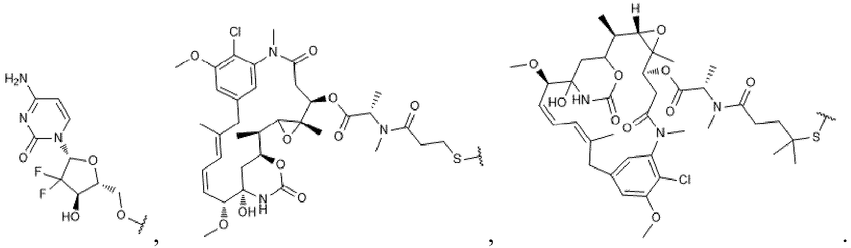

13. The compound of formula (I) according to any one of claims 1-12, or a pharmaceutically acceptable salt, stereoisomer or solvate thereof, wherein the isotope chelator or labeling precursor group and the fluorescent or molecular labeling group are selected from:

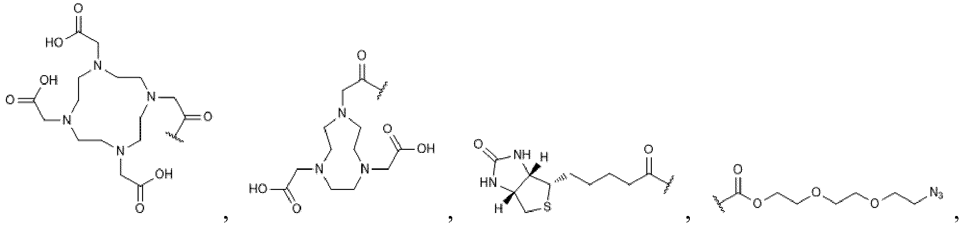

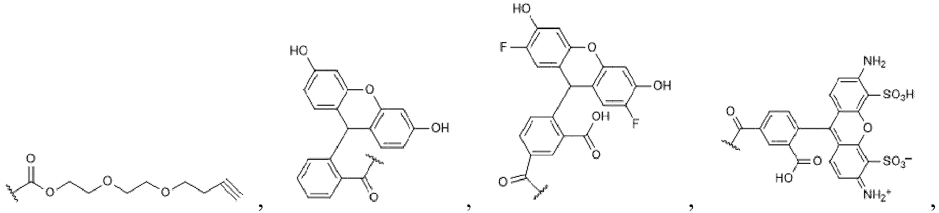

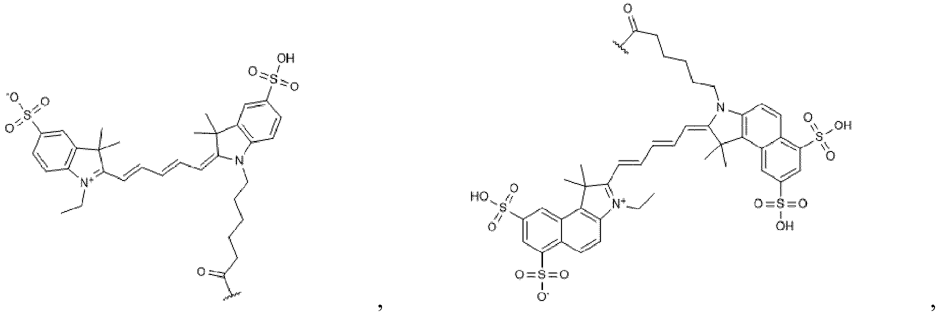

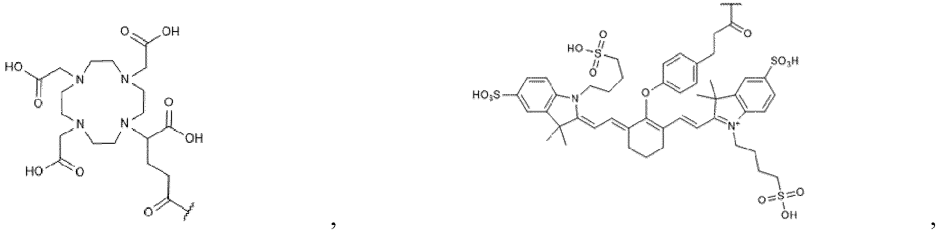

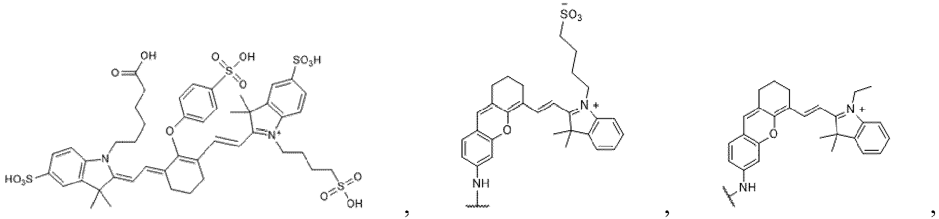

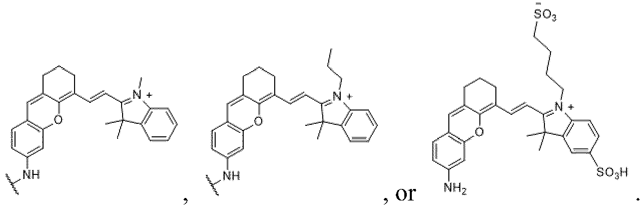

14. The compound of formula (I) according to any one of claims 1-13, or a pharmaceutically acceptable salt, stereoisomer or solvate thereof, wherein the targeting group is selected from 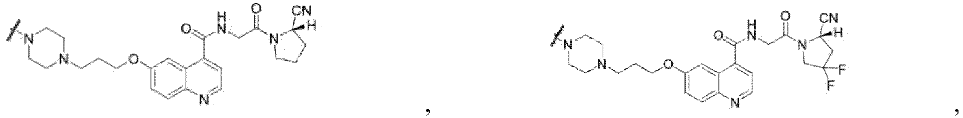 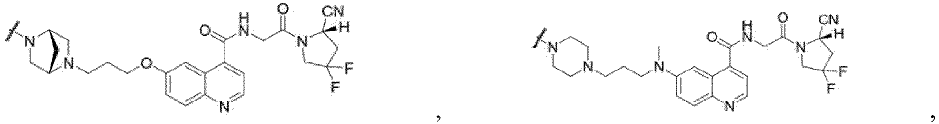 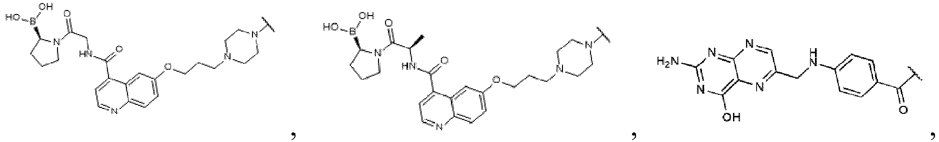 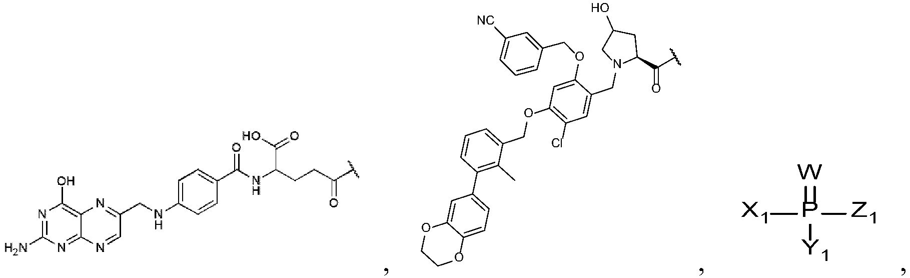 or

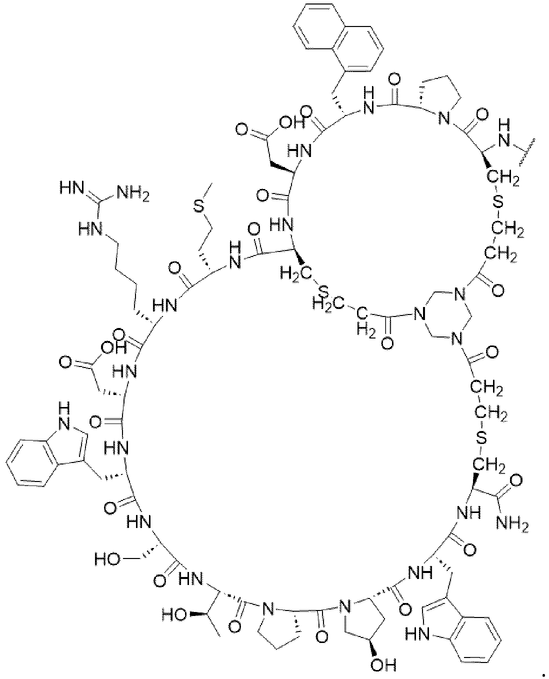

15. The compound of formula (I) according to claim 14, or a pharmaceutically acceptable salt, stereoisomer or solvate thereof, wherein the targeting group is selected from

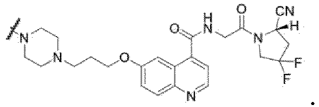

16. The compound of formula (I) according to any one of claims 1-15, or a pharmaceutically acceptable salt, stereoisomer or solvate thereof, wherein one of R₁, R₂, and R₄ is an isotope chelator or labeling precursor group.

17. The compound of formula (I) according to claim 1, or a pharmaceutically acceptable salt, stereoisomer or solvate thereof, wherein the compound of formula (I) is selected from 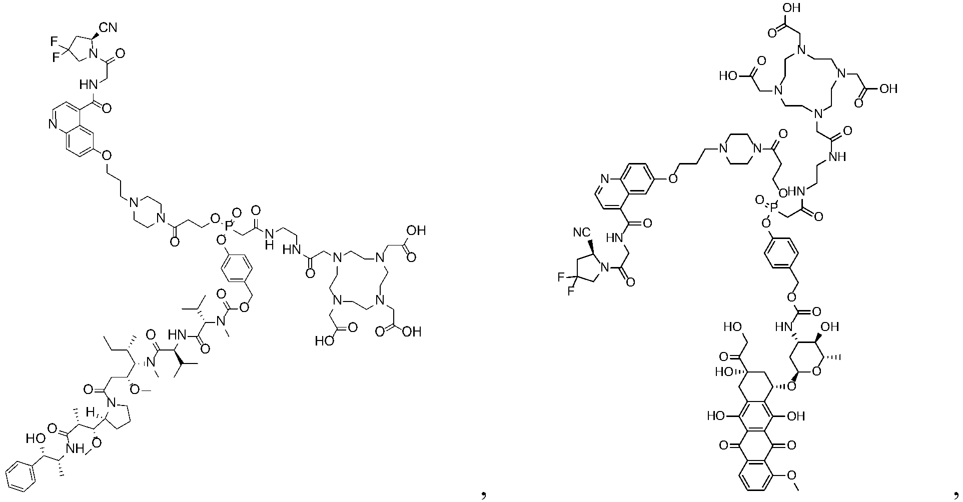 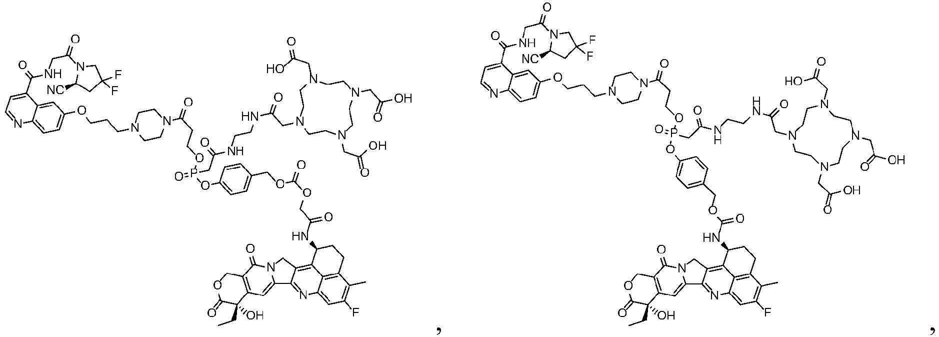 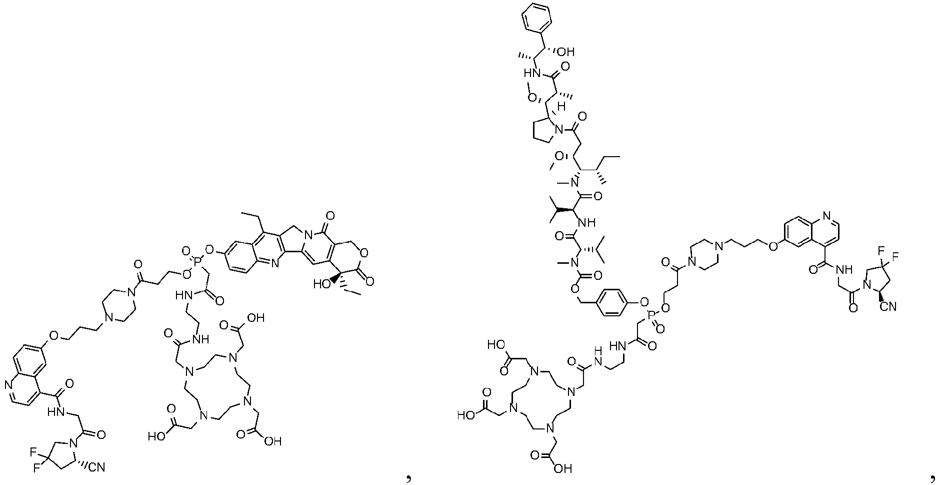 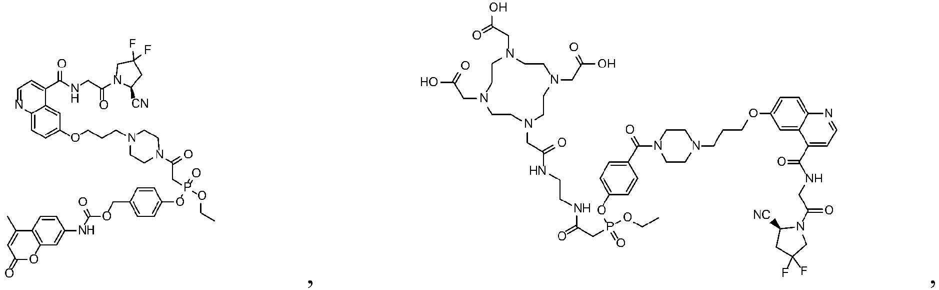 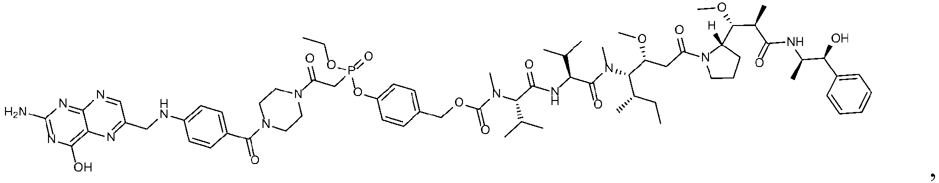 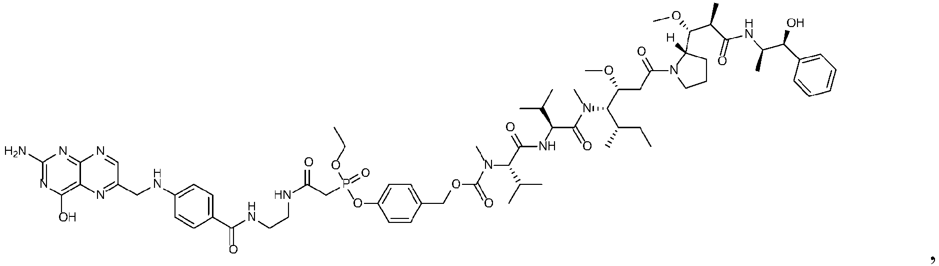 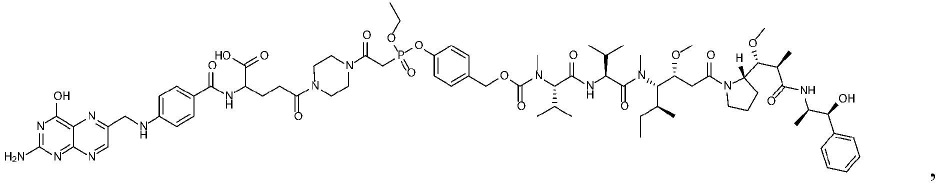 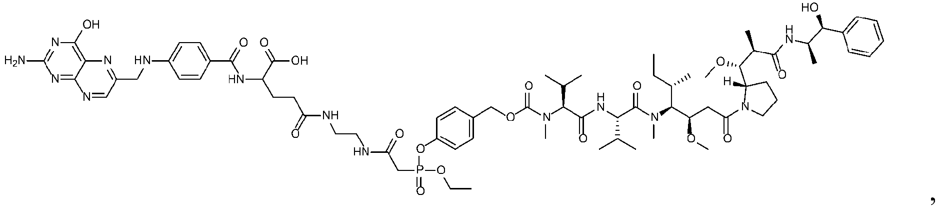 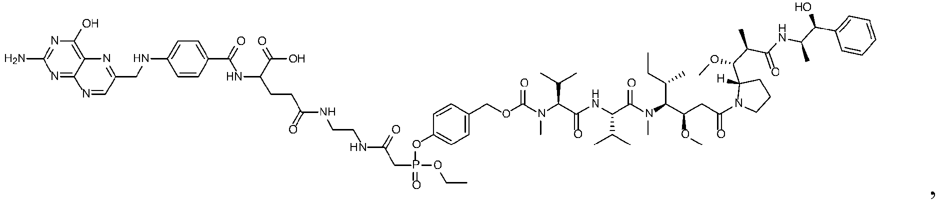 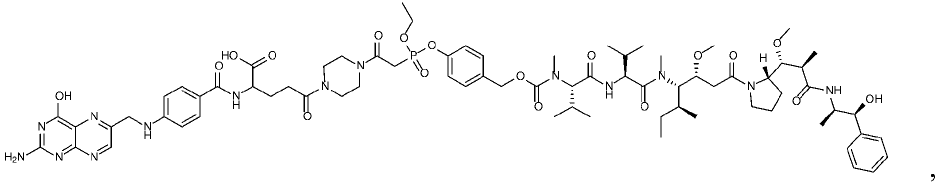 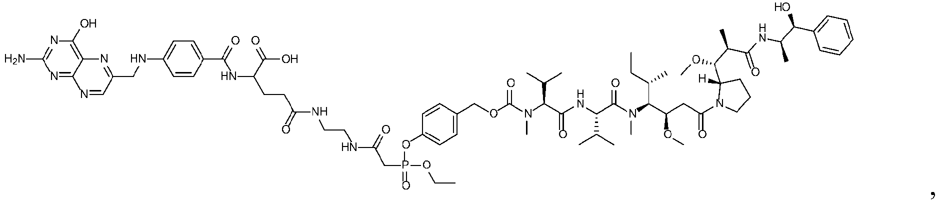 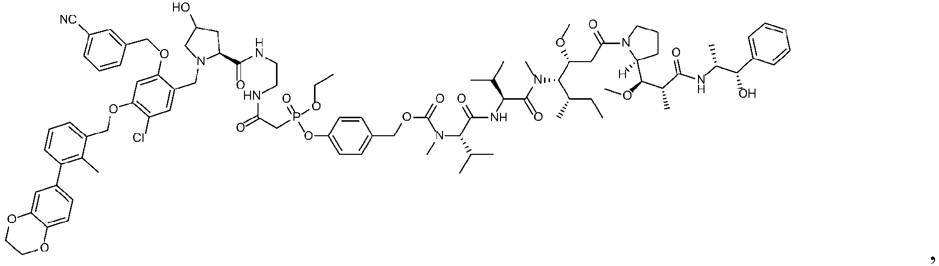 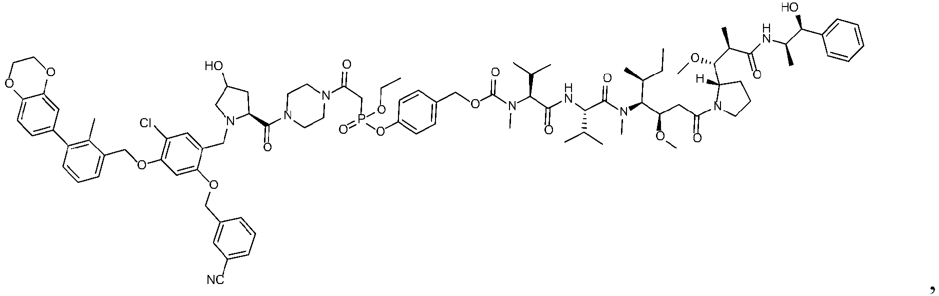 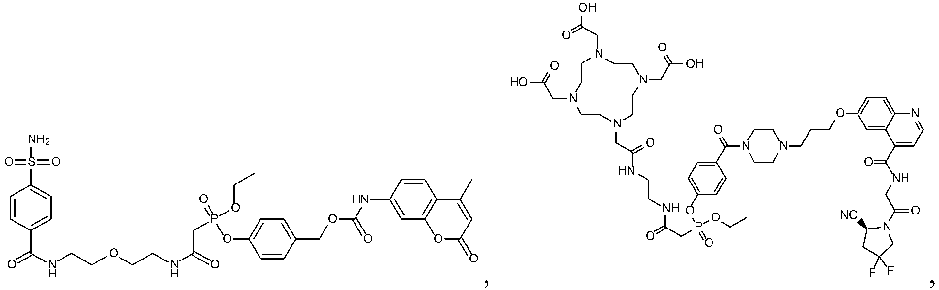 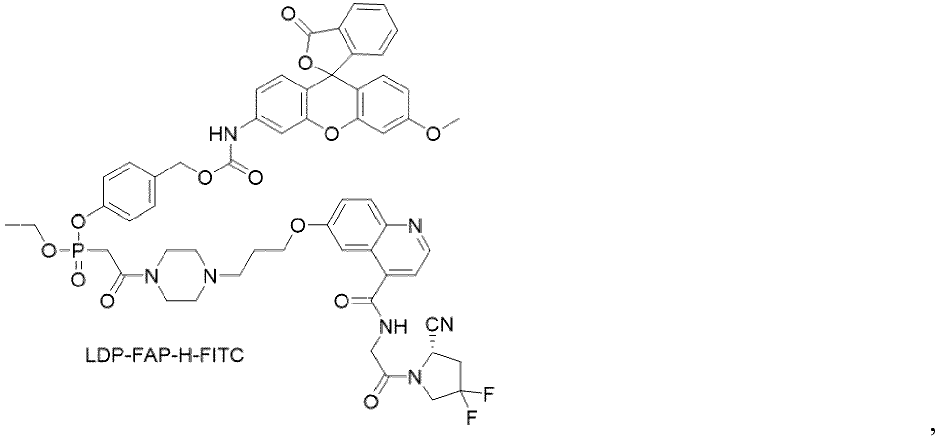 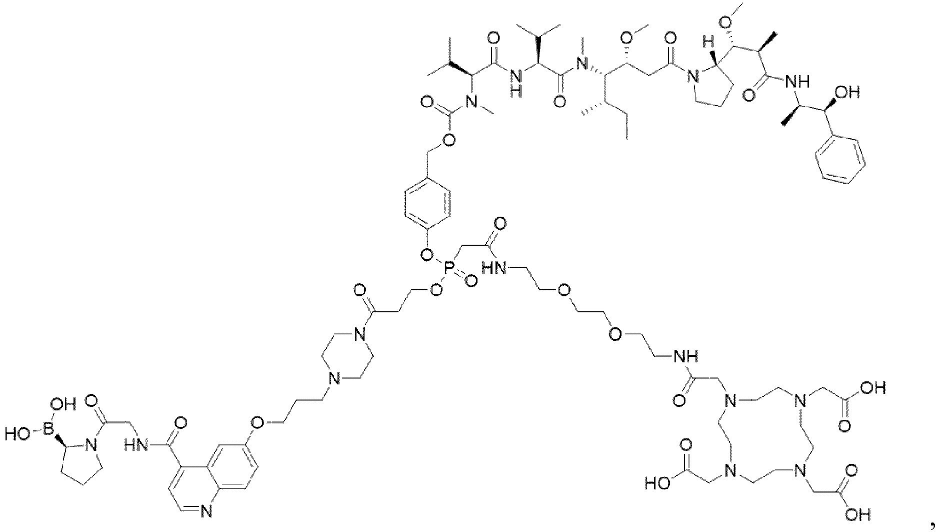 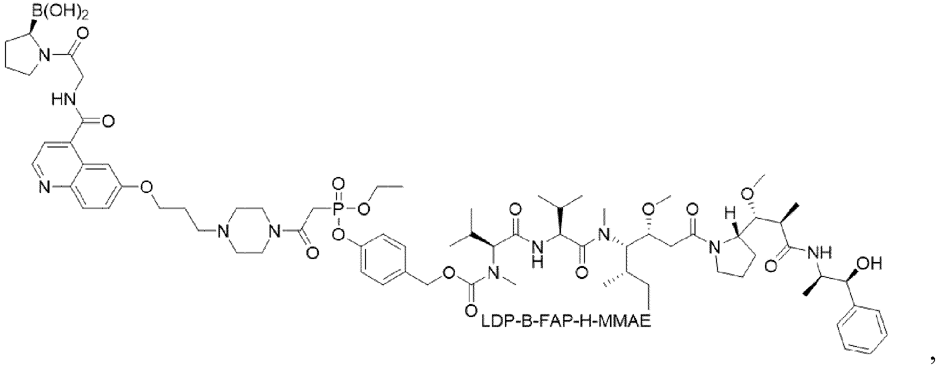 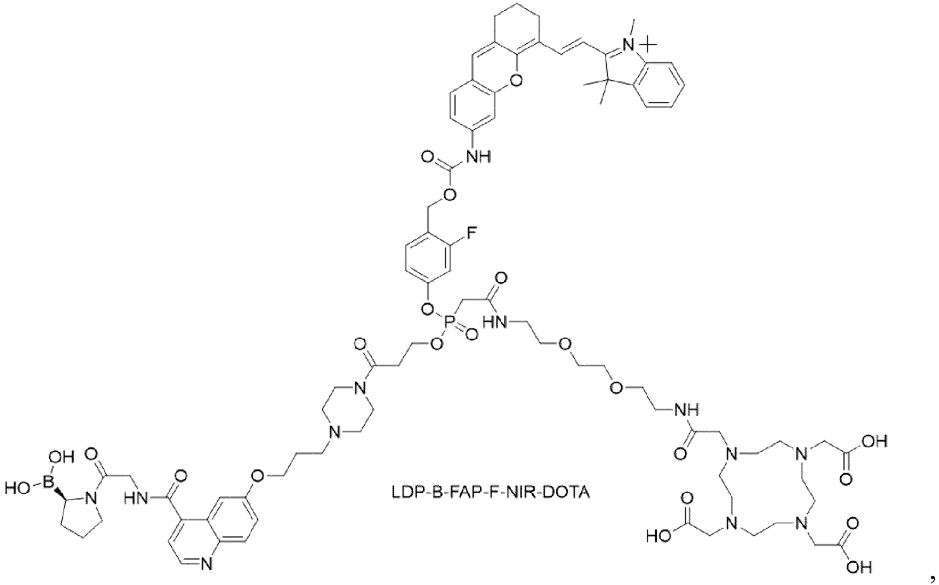 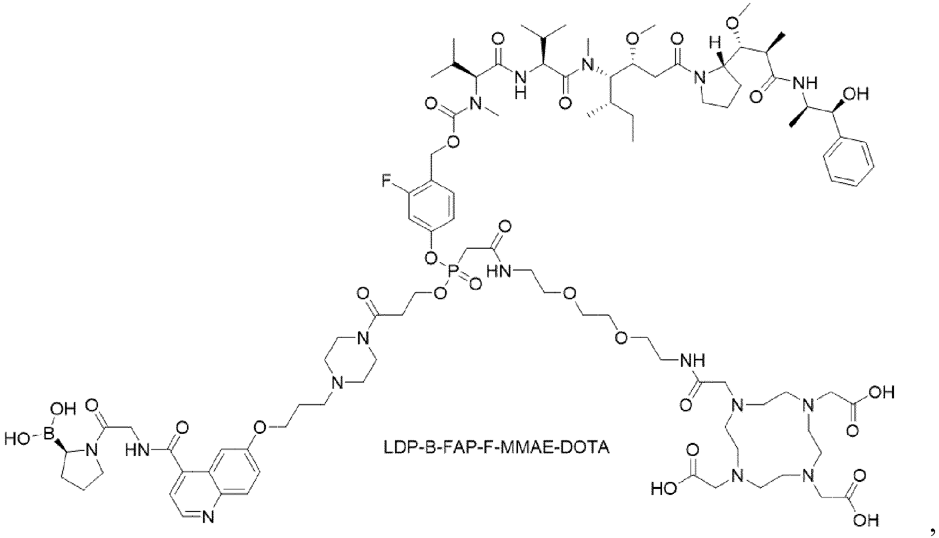 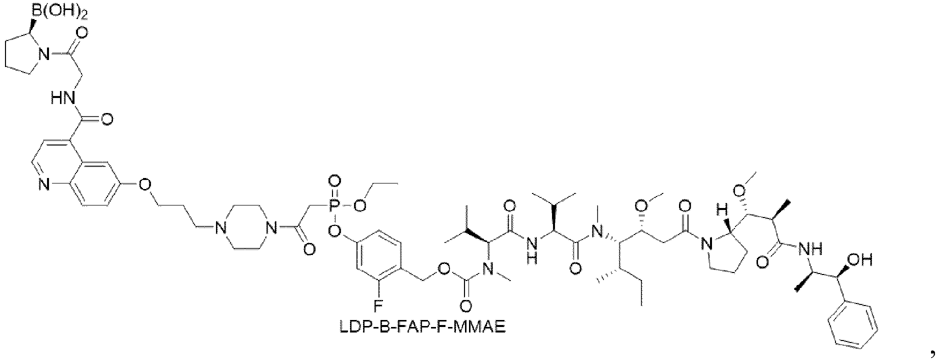 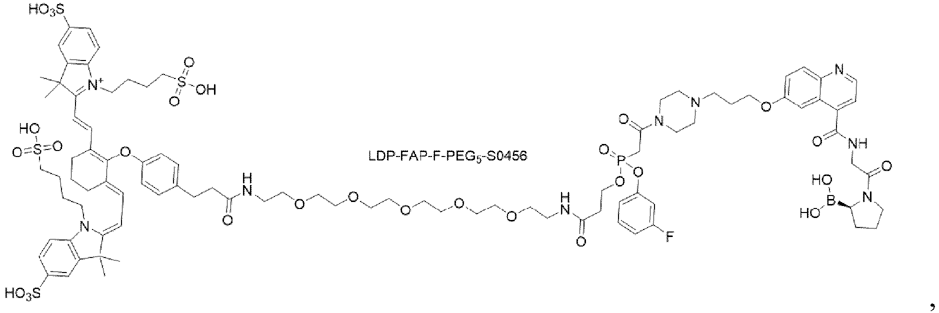 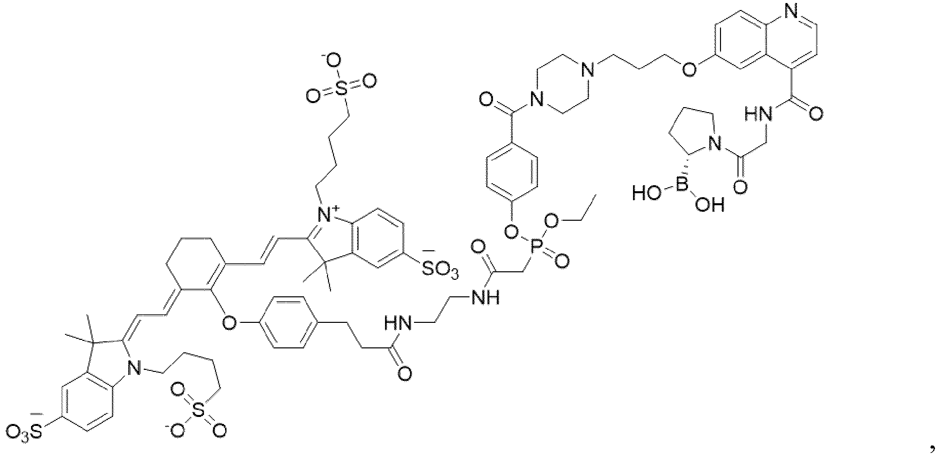 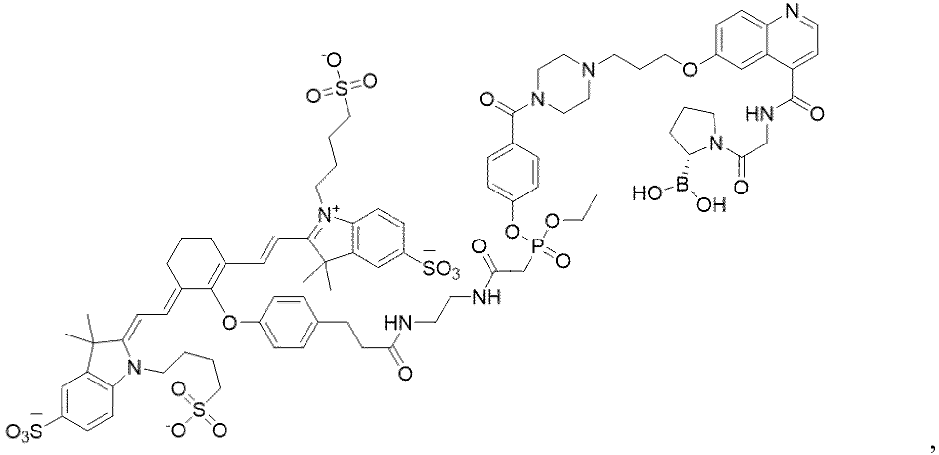 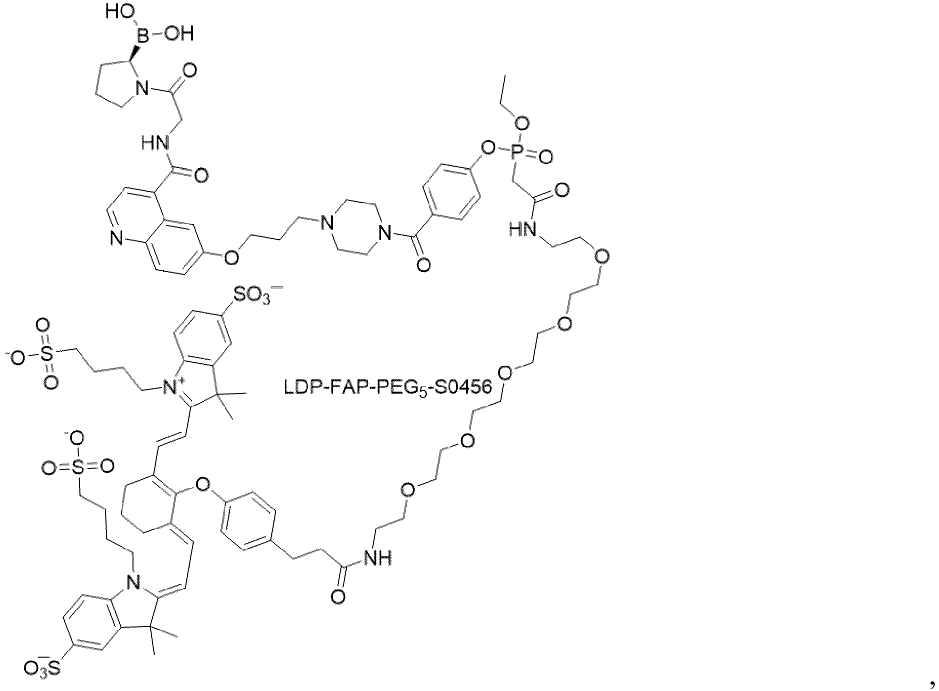 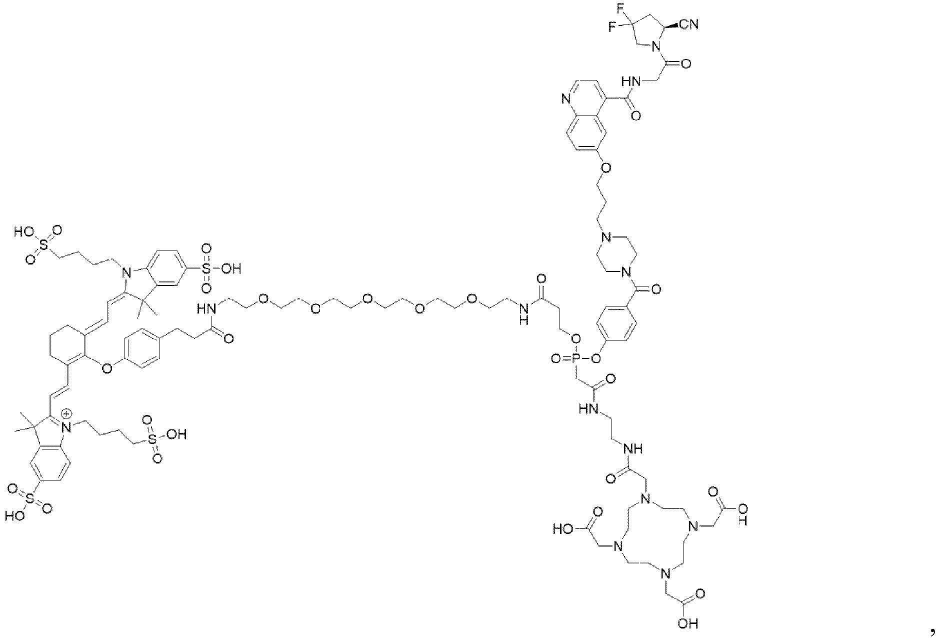 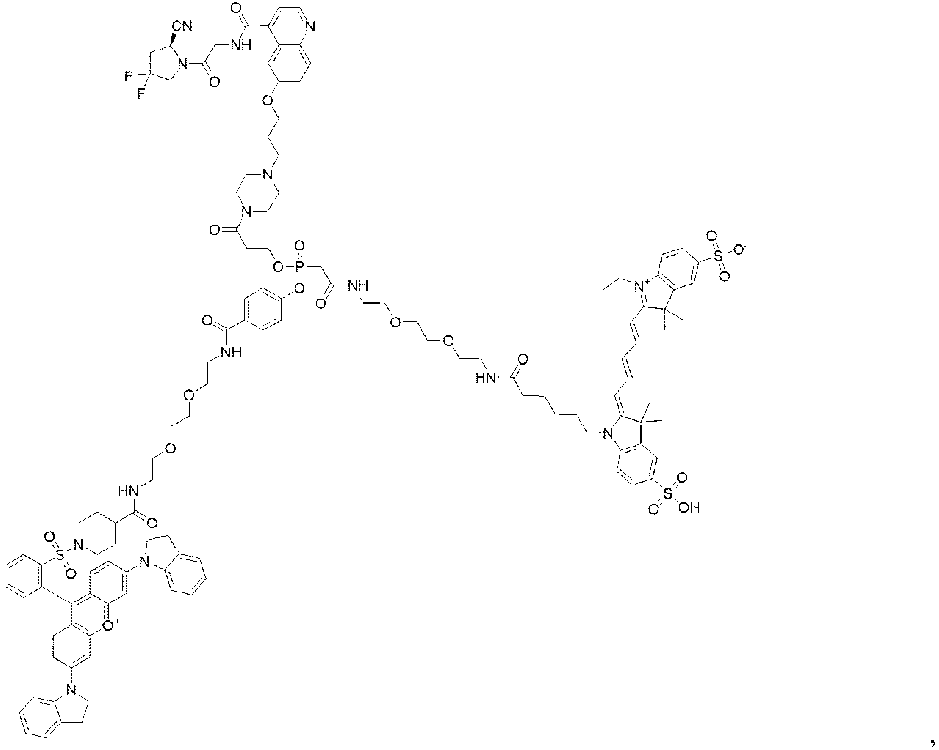 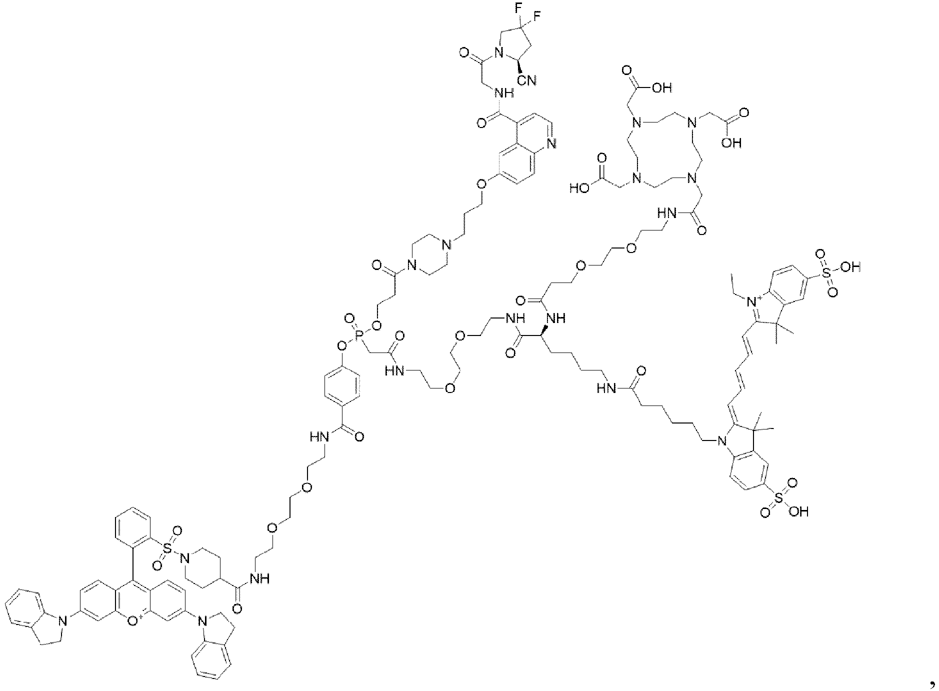 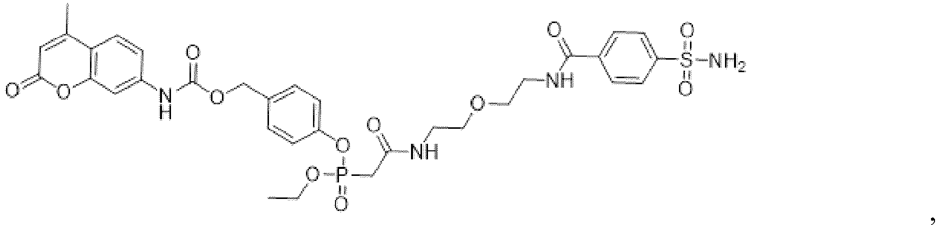 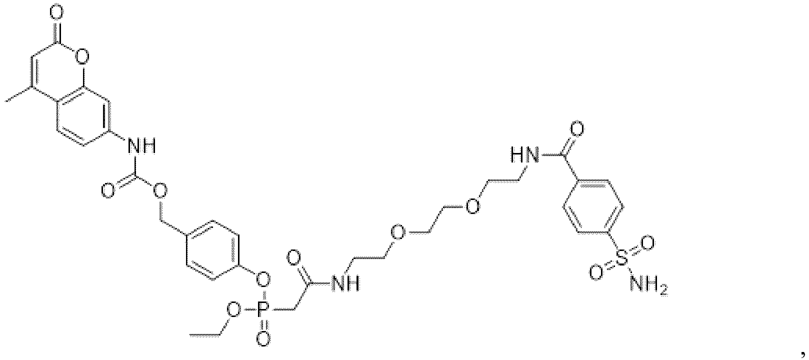 or

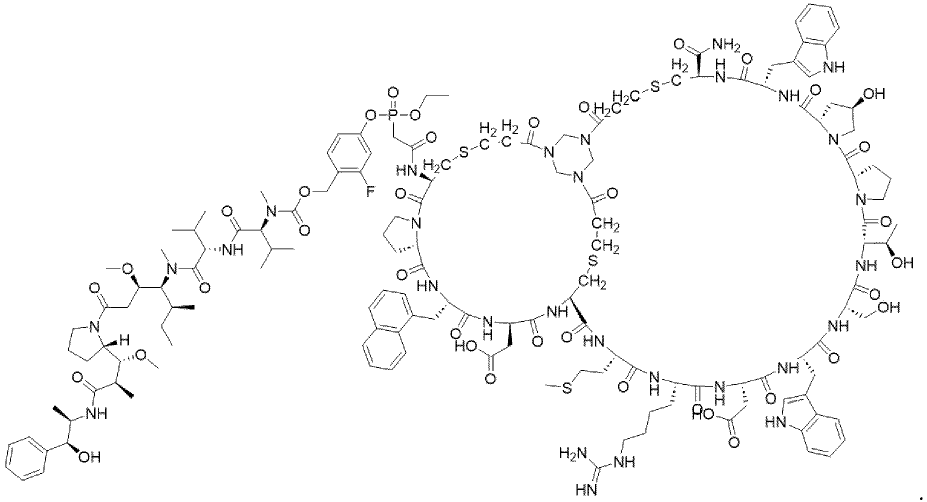

18. A chelate or nuclide label, comprising
(i) the compound of formula (I) according to any one of claims 1-17, or a pharmaceutically acceptable salt, stereoisomer or solvate thereof; and
(ii) a radionuclide.

19. The chelate or nuclide label according to claim 18, wherein the radionuclide is selected from: ¹⁸F, ⁵¹Cr, ⁶⁷Ga, ⁶⁸Ga, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, 86y, ⁸⁸Y, ⁹⁰Y, ¹⁴⁹Pm, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg, ²¹¹At, ¹⁵¹Eu, ¹⁵³Eu, ¹⁶⁹Eu, ²⁰¹Tl, ²⁰³Pb, ²¹²Pb, ⁶⁴Cu, ⁶⁷Cu, ¹⁸⁸Re, ¹⁸⁶Re, ¹⁹⁸Au, ²²⁵Ac, ²²⁷Th, and ¹⁹⁹Ag.

20. The chelate or nuclide label according to claim 18, wherein the radionuclide is ⁶⁸Ga or ⁸⁶Y.

21. A pharmaceutical composition, comprising or consisting of:
the compound of formula (I) according to any one of claims 1-17, or a pharmaceutically acceptable salt, stereoisomer or solvate thereof; or the chelate or nuclide label according to any one of claims 18-20,
optionally, and a pharmaceutically acceptable excipient.

22. A kit, comprising or consisting of:
(i) the compound of formula (I) according to any one of claims 1-17, or a pharmaceutically acceptable salt, stereoisomer or solvate thereof; or the chelate or nuclide label according to any one of claims 18-20; or the pharmaceutical composition according to claim 21, and
(ii) instructions for diagnosing or treating a disease.

23. Use of the compound of formula (I) according to any one of claims 1-17, or a pharmaceutically acceptable salt, stereoisomer or solvate thereof, or the chelate or nuclide label according to any one of claims 18-20, or the pharmaceutical composition according to claim 21, in the manufacture of a medicament for implementing targeted therapy or diagnosis.

24. The use according to claim 23, wherein the disease targeted by the targeted therapy is a malignant proliferative disease, an immune disease or an infectious disease.

25. The use according to claim 24, wherein the malignant proliferative disease is selected from: breast cancer, pancreatic cancer, small intestine cancer, colon cancer, rectal cancer, lung cancer, head and neck cancer, ovarian cancer, hepatocellular carcinoma, esophageal cancer, hypopharyngeal cancer, nasopharyngeal cancer, laryngeal cancer, myeloma cells, bladder cancer, cholangiocarcinoma, clear cell renal cell carcinoma, neuroendocrine tumor, oncogenic osteomalacia, sarcoma, cancer of unknown primary, thymic carcinoma, glioma, neuroglioma, astrocytoma, cervical cancer, prostate cancer, thyroid cancer, gastric cancer, bone cancer, lung cancer, lymphoma, corpus uteri cancer, gallbladder cancer, oral cancer and testicular cancer.

26. The use according to claim 24, wherein the immune disease is selected from: rheumatoid arthritis (RA), ankylosing spondylitis (AS), juvenile idiopathic arthritis (JIA), non-radiographic axial spondyloarthritis (nr-AxSpA), psoriasis, psoriatic arthritis (PsA), Crohn's disease (CD), ulcerative colitis (UC), systemic lupus erythematosus (SLE), lupus nephritis (LN), multiple sclerosis (MS), bronchial asthma, and the like.

27. The compound of formula (I) according to any one of claims 1-17, or a pharmaceutically acceptable salt, stereoisomer or solvate thereof, or the chelate or nuclide label according to any one of claims 18-20, or the pharmaceutical composition according to claim 21, for use in implementing targeted therapy or diagnosis.

28. A method for implementing targeted therapy or diagnosis, comprising using the compound of formula (I) according to any one of claims 1-17, or a pharmaceutically acceptable salt, stereoisomer or solvate thereof, or the chelate or nuclide label according to any one of claims 18-20, or the pharmaceutical composition according to claim 21.

29. A method for inhibiting overexpression of fibroblast activation protein in a subject in need thereof, the method comprising administering to the subject in need thereof an effective amount of the compound of formula (I) according to any one of claims 1-17, or a pharmaceutically acceptable salt, stereoisomer or solvate thereof, or the chelate or nuclide label according to any one of claims 18-20, or the pharmaceutical composition according to claim 21.

30. A method for diagnosing or treating a disease **characterized by** overexpression of fibroblast activation protein, the method comprising administering to a subject in need thereof an effective amount of the compound of formula (I) according to any one of claims 1-17, or a pharmaceutically acceptable salt, stereoisomer or solvate thereof, or the chelate or nuclide label according to any one of claims 18-20, or the pharmaceutical composition according to claim 21.

31. The method according to claim 29 or 30, wherein the disease **characterized by** overexpression of fibroblast activation protein is selected from cancer, chronic inflammation, atherosclerosis, fibrosis, tissue remodeling and scarring disease; preferably, the cancer is selected from breast cancer, pancreatic cancer, small intestine cancer, colon cancer, rectal cancer, lung cancer, head and neck cancer, ovarian cancer, hepatocellular carcinoma, esophageal cancer, hypopharyngeal cancer, nasopharyngeal cancer, laryngeal cancer, myeloma cells, bladder cancer, cholangiocarcinoma, clear cell renal cell carcinoma, neuroendocrine tumor, oncogenic osteomalacia, sarcoma, cancer of unknown primary, thymic carcinoma, glioma, neuroglioma, astrocytoma, cervical cancer and prostate cancer.

32. Use of the compound of formula (I) according to any one of claims 1-17, or a pharmaceutically acceptable salt, stereoisomer or solvate thereof, or the chelate or nuclide label according to any one of claims 18-20, or the pharmaceutical composition according to claim 21, in the manufacture of a medicament for inhibiting overexpression of fibroblast activation protein in a subject in need thereof.

33. Use of the compound of formula (I) according to any one of claims 1-17, or a pharmaceutically acceptable salt, stereoisomer or solvate thereof, or the chelate or nuclide label according to any one of claims 18-20, or the pharmaceutical composition according to claim 21, in the manufacture of a medicament for diagnosing or treating a disease **characterized by** overexpression of fibroblast activation protein.

34. The use according to claim 32 or 33, wherein the disease **characterized by** overexpression of fibroblast activation protein is selected from cancer, chronic inflammation, atherosclerosis, fibrosis, tissue remodeling and scarring disease; preferably, the cancer is selected from breast cancer, pancreatic cancer, small intestine cancer, colon cancer, rectal cancer, lung cancer, head and neck cancer, ovarian cancer, hepatocellular carcinoma, esophageal cancer, hypopharyngeal cancer, nasopharyngeal cancer, laryngeal cancer, myeloma cells, bladder cancer, cholangiocarcinoma, clear cell renal cell carcinoma, neuroendocrine tumor, oncogenic osteomalacia, sarcoma, cancer of unknown primary, thymic carcinoma, glioma, neuroglioma, astrocytoma, cervical cancer and prostate cancer.

35. A compound of formula (II), 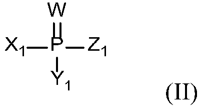 wherein
W is oxygen or sulfur, preferably oxygen;
X₁ is -L₁-R₁;
Y₁ is -L₁-R₂;
Z₁ is -L₂-R₃-L₁-R₄;
L₁, at each occurrence, is independently a bond, a linking group or absent;
L₂ is O, S, or NH, preferably oxygen;
R₃ is an optionally substituted arylene, heteroarylene, cycloalkylene or heterocycloalkylene, the substituent is selected from F, Br, Cl, methoxy, trifluoromethyl, and cyano;
R₁, R₂, R₄ are capping groups;
the capping group is selected from -L_{g}-(Lₕ)ₙ-Lᵢ-R^{c}, wherein L_{g}, Lₕ, Lᵢ, at each occurrence, are each independently selected from a bond, -O-, -S-, -C(R^{a})(R^{b})-, -NH-, -N(CH₃)-, -C(=O)-, - CH₂O-, or -OCH₂-, the subscript n is an integer of 0-4, R^{a} and R^{b}, at each occurrence, are independently selected from H, methyl, ethyl, propyl, isopropyl, phenyl, -OH, -SH, -NH₂, - NH(CH₃), or -NH(CH₃)₂, R^{c} is selected from methyl, ethyl, hydroxyl, or thiol;
or a pharmaceutically acceptable salt, stereoisomer or solvate thereof.

36. The compound of formula (II) according to claim 35, or a pharmaceutically acceptable salt, stereoisomer or solvate thereof, wherein R₃ is selected from
optionally substituted C₅₋₁₄ arylene;
optionally substituted C₅₋₁₄ heteroarylene containing 1 to 3 heteroatoms independently selected from N, O, or S;
optionally substituted divalent saturated or partially unsaturated fused, bridged or spiro C₅₋₁₄ cycloalkylene containing a single ring or multiple rings; or
optionally substituted divalent saturated or partially unsaturated fused, bridged or spiro C₅₋₁₄ heterocycloalkylene containing a single ring or multiple rings, which contains 1 to 3 heteroatoms independently selected from N, O, or S;
the substituent is selected from F, Br, Cl, methoxy, trifluoromethyl, and cyano.

37. The compound of formula (II) according to claim 35, or a pharmaceutically acceptable salt, stereoisomer or solvate thereof, wherein R₃ is selected from optionally substituted 1,2-phenylene, 1,3-phenylene, 1,4-phenylene, 1,2-naphthylene, 1,5-naphthylene, 1,6-naphthylene, 2,6-naphthylene, furanylene, thienylene, pyrrolylene, imidazolylene, pyrazolylene, triazolylene, pyridylene, pyrazinylene, pyrimidinylene, pyridazinylene, cyclopentylene, cyclohexylene, tetrahydrofuranylene, tetrahydropyrrolylene, tetrahydrothienylene, tetrahydropyranylene, piperidinylene, tetrahydrothiopyranylene, dioxanylene, piperazinylene, 1,4-piperazinylene, pyrazinylene, morpholinylene, and dioxanylene; the substituent is selected from F, Br, Cl, methoxy, trifluoromethyl, and cyano; or
R₃ is preferably 3-X-1,4-phenylene, 2-X-1,4-phenylene, 2-X-1,5-phenylene, 3-X-1,5-phenylene, 3-X-1-phenylene, 2-X-1-phenylene, 4-X-1-phenylene; X is selected from F, Br, Cl, methoxy, trifluoromethyl, and cyano.

38. Use of the compound according to claim 36 or 37 in preparing the compound according to claim 1.

39. A method for preparing the compound according to claim 1, comprising using the compound according to claim 36 or 37 as an intermediate.

40. Use of the compound according to claim 36 or 37 in the manufacture of a medicament for implementing targeted therapy or diagnosis.
